(19) Europäisches Patentamt / European Patent Office / Office européen des brevets

(11) **EP 4 647 125 A2**

(12) **EUROPEAN PATENT APPLICATION**

(43) Date of publication:
**12.11.2025 Bulletin 2025/46**

(21) Application number: **25189523.1**

(22) Date of filing: **16.05.2022**

(51) International Patent Classification (IPC):
***A61P 27/02*** *(2006.01)*

(52) Cooperative Patent Classification (CPC):
**A61K 38/179; A61P 27/02**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.05.2021 US 202163189541 P**
**20.08.2021 US 202163235398 P**
**07.01.2022 US 202263297420 P**
**03.02.2022 US 202263306315 P**

(62) Document number(s) of the earlier application(s) in accordance with Art. 76 EPC:
**22728724.0 / 4 185 318**

(71) Applicants:
• **Regeneron Pharmaceuticals, Inc.**
**Tarrytown, NY 10591 (US)**
• **Bayer Healthcare LLC**
**Whippany, NJ 07981 (US)**

(72) Inventors:
• **VITTI, Robert L.**
**Tarrytown, 10591 (US)**
• **BERLINER, Alyson J.**
**Tarrytown, 10591 (US)**
• **CHU, Karen**
**Tarrytown, 10591 (US)**
• **ASMUS, Friedrich**
**14129 Berlin (DE)**
• **DA SILVA LEAL, Sergio Casimiro**
**4153 Reinach (CH)**
• **EISSING, Thomas**
**40764 Langenfeld (DE)**
• **RITTENHOUSE, Kay D.**
**Randolph, NJ 07869 (US)**

(74) Representative: **J A Kemp LLP**
**80 Turnmill Street**
**London EC1M 5QU (GB)**

Remarks:
•The complete document including Reference Table(s) and the Sequence Listing(s) can be downloaded from the EPO website
•This application was filed on 15-07-2025 as a divisional application to the application mentioned under INID code 62.
•Claims filed after the date of receipt of the application (Rule 68(4) EPC).

(54) **EXTENDED, HIGH DOSE VEGF ANTAGONIST REGIMENS FOR TREATMENT OF ANGIOGENIC EYE DISORDERS**

(57) The present invention relates to a method for treating or preventing an angiogenic eye disorder, in a subject in need thereof, comprising administering to an eye of the subject, a single initial dose of about 8 mg or more of a VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose.

EP 4 647 125 A2

**Description**

This application claims the benefit of U.S. Provisional Patent Application No. 63/189,541, filed May 17, 2021; U.S. Provisional Patent Application No. 63/235,398, filed August 20, 2021; U.S. Provisional Patent Application No. 63/297,420, filed January 7, 2022; and U.S. Provisional Patent Application No. 63/306,315, filed February 3, 2022; each of which is herein incorporated by reference for all purposes.

**REFERENCE TO A SEQUENCE LISTING**

The sequence listing of the present application is submitted electronically as an ASCII formatted sequence listing with a file name "10861WO01-Sequence", creation date of May 16, 2022, and a size of 6,006 bytes. This sequence listing submitted is part of the specification and is herein incorporated by reference in its entirety.

**FIELD OF THE INVENTION**

The field of the present invention relates to methods for treating or preventing angiogenic eye disorders by administering a VEGF antagonist.

**BACKGROUND OF THE INVENTION**

Neovascular (wet) AMD (nAMD) is a major health issue in aging populations globally. Vision loss in nAMD results from the abnormal growth and leakage of blood vessels in the macula. In elderly patients affected by nAMD, vision loss frequently has an even greater impact, as it substantially reduces the visual compensation of functional impairment by other age-related comorbidities, such as arthritis and osteoporosis.

Intravitreally (IVT) administered anti-vascular endothelial growth factor (VEGF) therapies like EYLEA® inhibit neovascular vessel growth and leakage in the retina, and they are currently the standard-of-care for patients with nAMD. They not only maintain visual function but also provide clinically meaningful visual gains. Treatment of nAMD is chronic and life-long in most patients to suppress retinal edema and recurrences of choroidal neovascularization (CNV). Although the currently approved IVT anti-VEGF therapies are efficacious and well-tolerated, the need for IVT injections every 4 to 8 weeks, specifically in the initial phase and during maintenance of treatment, represents a significant burden to physicians, patients, and caregivers. While the procedure is straightforward and relatively easy to perform, capacity issues for ensuring an appropriate injection frequency in order to achieve patient outcomes similar to those seen in the pivotal studies represent an increasing challenge to individual practices and the healthcare system, overall. Moreover, high frequency dosing leads to increased burdens on patients, e.g., to find transportation and miss work. A secondary effect of this burden is a lower probability of non-compliance with the prescribed treatment regimen.

While the efficacy and safety of currently approved VEGF antagonist therapies have been established for the treatment of nAMD, there remains an unmet medical need for the development of therapies with the potential to reduce treatment burden while providing at least similar or even improved visual outcomes over currently available standard-of-care.

EYLEA (2 mg dose, administered at a concentration of 40 mg/mL, also called intravitreal aflibercept injection [IAI]) is currently approved in the United States (US) for the treatment of nAMD, and is also approved for the treatment of macular edema following retinal vein occlusion (RVO), diabetic macular edema (DME), and diabetic retinopathy (DR).

**SUMMARY OF THE INVENTION**

The present invention provides a method for treating or preventing an angiogenic eye disorder (*e.g.*, neovascular age-related macular edema (nAMD), diabetic macular edema (DME), diabetic retinopathy (DR), macular edema (ME) secondary to retinal vein occlusion (RVO) (ME-RVO), in a subject in need thereof, comprising administering to an eye of the subject (*e.g.*, by intravitreal injection), *e.g.*, in about 70 μl, a single initial dose of about 8 mg or more of a VEGF antagonist (*e.g.*, aflibercept) to an eye of the subject, followed by one or more secondary doses of the VEGF antagonist, followed by one or more tertiary doses of the VEGF antagonist; wherein each secondary dose is administered about 2 to 4 or 3 to 5 weeks after the immediately preceding dose; and wherein each tertiary dose is administered about 4, 8 or 12 weeks after the immediately preceding dose.

In an embodiment of the invention, wherein, *e.g.*, while receiving such a regimen: (i) with respect to visual acuity or best corrected visual acuity (BCVA), the subject achieves: no loss in visual acuity or BCVA; a gain in visual acuity or BCVA; no loss of visual acuity or BCVA by about week 4, 8, 9, 12, 16, 20, 24, 28, 32, 36, 40 or 44, following the initial dose wherein visual acuity or BCVA is according to ETDRS or the Snellen equivalent; no loss of visual acuity or BCVA of about 5 or more, about 10 or more, or about 15 or more letters by about week 4, 8, 9, 12, 16, 20, 24, 28, 32, 36, 40 or 44, following the initial dose wherein visual acuity or BCVA is according to ETDRS or the Snellen equivalent; a gain in visual acuity or BCVA, of about 5 or more, about 10 or more or about 15 or more letters, by about week 4, 8, 9, 12, 16, 20, 24, 28, 32, 36, 40 or 44 following the initial dose, wherein visual acuity or BCVA is according to ETDRS or the Snellen equivalent; and/or a gain in visual acuity or BCVA of about 6 or 7 or 8 letters by about week 8 and maintaining a gain of about 6 or 7 or 8 letters until at least about week 44 wherein visual acuity or BCVA is according to ETDRS or the Snellen equivalent; (ii) with respect to central retinal thickness (CRT), the subject achieves: a decrease in central retinal thickness; a decrease in central retinal thickness by at least about 123, 125, 131, 142, 147, 149, 150, 151, 156, 157, 158, 159, 161, 162, 166, 167, 168, 172, 173, 175, 177, 178 or 183 micrometers ($\mu$m) by about week 4, 8, 9, 12, 16, 20, 24, 28, 32, 36, 40 or 44 following the initial dose; a decrease in central retinal thickness of about 47 micrometers from about week 12 to about week 20 following the initial dose; a decrease in central retinal thickness of about 17 micrometers from about week 24 to about week 32 following the initial dose; a decrease in central retinal thickness of about 18 micrometers from about week 36 to about week 44 following the initial dose; a decrease in central retinal thickness of about 123, 131 161 micrometers by about week 4, 8, 12, 16 or 20 following the initial dose and maintaining the decrease until at least about week 44 following the initial dose; and/or a reduction in CRT of about 159, 160, 161 or 162 micrometers by about week 4 or 8 or 12 and maintaining a reduction of about 159, 160, 161 or 162 micrometers until at least about week 44; (iii) with respect to retinal fluid, the subject achieves: a dry retina having no intraretinal fluid and no subretinal fluid; or no intraretinal fluid; or no subretinal fluid; in the center subfield or macula as measured by spectral domain optical coherence tomography; a dry retina having no intraretinal fluid and no subretinal fluid; or no intraretinal fluid; or no subretinal fluid; in the center subfield as measured by spectral domain optical coherence tomography by about week 4, 8, 9, 12, 16, 20, 24, 28, 32, 36, 40 or 44, following the initial dose; no SRF and IRF in the macula as measured by SD-OCT by week 16 or week 44 following the initial dose; no sub-retinal pigment epithelium (RPE) fluid until at least about week 44 following the initial dose as measured by spectral domain optical coherence tomography; and/or maintenance of a dry retina once achieved until at least about week 44 following the initial dose as measured by spectral domain optical coherence tomography; and/or (iv) the subject achieves: a reduction in total choroidal neovascularization (CNV) lesion size by at least about 3.2 or 3.3 $\mu$m by about week 4, 8, 9, 12, 16, 20, 24, 28, 32, 36, 40 or 44 following the initial dose; no significant increase in intraocular pressure from baseline by about week 4, 8, 9, 12, 16, 20, 24, 28, 32, 36, 40 or 44 or later following the initial dose; and/or no significant increase in systolic (S) and/or diastolic (D) blood pressure from baseline by about week 4, 8, 9, 12, 16, 20, 24, 28, 32, 36, 40 or 44 or later following the initial dose. The scope of the present invention also encompasses methods for achieving any of such achievements in a subject in need thereof who is suffering from an angiogenic eye disorder, by administering the dosing regimen set forth above. For example, in an embodiment of the invention, the method for treating or preventing an angiogenic eye disorder (*e.g.*, neovascular age-related macular edema, diabetic retinopathy, diabetic macular edema or macular edema following retinal vein occlusion (RVO) ) includes administering a single initial dose of VEGF antagonist (*e.g.*, aflibercept), followed by one or more secondary doses of the VEGF antagonist, followed by one or more tertiary doses of the VEGF antagonist; wherein each secondary dose is administered about 4 weeks after the immediately preceding dose; and wherein each tertiary dose is administered about 4, 8 or 12 weeks after the immediately preceding dose. In an embodiment of the invention, relative to a subject receiving such a dosing regimen, except that only 2 mg of VEGF antagonist is administered, the subject receiving 8 mg of VEGF antagonist exhibits a greater decrease in central retinal thickness after 4, 8, 12, 16 or more weeks; a greater improvement in best corrected visual acuity after 4, 8, 12, 16 or more weeks; and/or a greater likelihood of having a dry retina (*e.g.*, lacks intraretinal fluid and/or subretinal fluid) after 4, 8, 12, 16 or more weeks. In an embodiment of the invention, 2-4 weeks is 2, 3 or 4 weeks. The present invention further provides a method for improving best corrected visual acuity, decreasing central retinal thickness and/or achieving a dry retina, in the eye of a subject (*e.g.*, suffering from neovascular age-related macular edema, diabetic retinopathy, diabetic macular edema or macular edema following retinal vein occlusion (RVO)) in need thereof, comprising administering to the eye of the subject, a single initial dose of about 8 mg or more of a VEGF antagonist (*e.g.*, aflibercept) to an eye of the subject, followed by one or more secondary doses of the VEGF antagonist, followed by one or more tertiary doses of the VEGF antagonist; wherein each secondary dose is administered about 2 to 4 weeks after the immediately preceding dose; and wherein each tertiary dose is administered about 4, 8 or 12 weeks after the immediately preceding dose. Also provided by the present invention is a method for promoting retinal drying, in the eye of a subject suffering from an angiogenic eye disorder (*e.g.*, neovascular age-related macular edema, diabetic retinopathy, diabetic macular edema or macular edema following retinal vein occlusion (RVO)), comprising administering to the eye of the subject, a single initial dose of about 8 mg or more of a VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose; for example, wherein retinal drying is characterized by no intraretinal fluid (IRF) and/or no subretinal fluid (SRF) in the eye of the subject, *e.g.*, after the subject has received the three monthly doses of the VEGF antagonist. In an embodiment of the invention, the VEGF antagonist is:

(i) a VEGF receptor fusion protein, for example, comprising two polypeptides that comprise (1) a VEGFR1 component comprising amino acids 27 to 129 of SEQ ID NO: 2; (2) a VEGFR2 component comprising amino acids 130-231 of SEQ ID NO: 2; and (3) a multimerization component comprising amino acids 232-457 of SEQ ID NO: 2;
(ii) a VEGF receptor fusion protein comprising two polypeptides that comprise immunoglobin-like (Ig) domain 2 of a VEGFR1 and Ig domain 3 of VEGFR2, and a multimerizing component;
(iii) a VEGF receptor fusion protein comprising two polypeptides that comprise an immunoglobin-like (Ig) domain 2 of VEGFR1, an Ig domain 3 of VEGFR2, an Ig domain 4 of said VEGFR2; and a multimerizing component;
(iv) a VEGF receptor fusion protein comprising two VEGFR1R2-FcΔC1(a) polypeptides encoded by the nucleic acid sequence of SEQ ID NO: 1; or
(v) selected from the group consisting of: aflibercept, conbercept, bevacizumab, ranibizumab, pegaptanib, brolucizumab, an anti-VEGF DARPin, abicipar pegol, faricimab, an anti-VEGF antibody or antigen-binding fragment thereof or biopolymer conjugate thereof (*e.g.*, KSI-301), bevacizumab, ranibizumab and a bispecific anti-VEGF/ANG2 antibody.

In an embodiment of the invention, the VEGF antagonist is administered to the eye of the subject in a pharmaceutical formulation, for example, which is selected from the group consisting of A-KKKK as so designated herein. In an embodiment of the invention, the VEGF antagonist is administered to the eye from a syringe, e.g., a pre-filled syringe, (*e.g.*, which is glass, plastic and/or sterile). In an embodiment of the invention, the syringe is characterized by the ornamental design as set forth in International Design Registration No. DM/212 509; which is herein incorporated by reference.

In an embodiment of the invention, a dosage of about 8 mg or more is a dose of about 9, 9.3, 9.33, 9.7, 9.8, 9.9, 9.7-9.9 mg or more and such a dosage amount may vary within a given range, *e.g.*, ± about 0.5, or ± about 0.51 mg. The volume in which a dose is delivered can be, for example, about 70, 81, 82, 81.7, 85, 86, 87, 85-87 microliters and the volume may vary within a given range, *e.g.*, ± about 4, 4.45, 4.5, or 5 microliters. Doses may be delivered with a dose delivery device (DDD) which is a syringe.

Highly precise doses of VEGF antagonist (*e.g.*, aflibercept) may be delivered, for example, in a volume that is device-determined. In an embodiment of the invention, a dose is delivered with a syringe by a method that includes the steps: (a) withdrawing a plunger rod of the syringe to fill the syringe with the formulation; (b) priming the syringe, thereby removing air from the syringe and, thus avoiding injection of air into the eye, by advancing the plunger rod by a predetermined distance into the syringe body until advancement of the plunger rod is resisted by a stop; (c) rotating the plunger rod about a longitudinal axis; and (d) actuating the plunger rod to dispense a predetermined (device-determined) volume (*e.g.*, about 70, 81, 82, 81.7, 85, 86, 87, 85-87 microliters, ± about 4, 4.45, 4.5, or 5 microliters) of the formulation.

**BRIEF DESCRIPTION OF THE FIGURES**

**Figure 1:** Summary of CANDELA Clinical Trial Plan and Dosing Schedule.
**Figure 2:** Baseline Demographics of Patients in CANDELA trial (IAI dosing group, HD dosing group and All patients (all patients with data reported as of data cut-off (IAI n=27; HD n=28))) at week 16.
**Figure 3:** Continued Baseline Demographics of Patients in CANDELA Trial at (all patients with data reported as of data cut-off (IAI n=27; HD n=28))) at week 16.
**Figure 4:** Baseline Blood Pressures of IAI and HD Dosing Groups in CANDELA trial (all patients with data reported as of data cut-off (IAI n=27; HD n=28))) at week 16.
**Figure 5:** Mean Change in Central Retinal Thickness (CRT) from Baseline (BL) in IAI and HD CANDELA Dosing Groups in Patients Completing Week 16. Completers are patients with OCT values at both weeks 12 and 16 (IAI n=22; HD n=27).
**Figure 6:** Mean change in Best Corrected Visual Acuity (BCVA) (ETDRS letters) from Baseline (BL) in IAI and HD CANDELA Dosing Groups in Patients Completing to Week 16 (IAI n=22; HD n=27).
**Figure 7:** Proportion of HD and IAI patients in CANDELA Completing to Week 16 with a Dry Retina (no intraretinal or subretinal fluid) at Baseline and Weeks 4, 8, 12 and 16.
**Figure 8:** Proportion of HD and IAI patients in CANDELA Completing to week 16 with a Retina that is Not Dry (intraretinal and/or subretinal fluid) at Baseline and Weeks 4, 8, 12 and 16.
**Figure 9:** Proportion of HD and IAI Patients in CANDELA Completing to Week 16 with Intraretinal Fluid (IRF) at Baseline and Weeks 4, 8, 12 and 16.
**Figure 10:** Proportion of HD and IAI Patients in CANDELA Completing to Week 16 with Subretinal fluid (SRF) at Baseline and Weeks 4, 8, 12 and 16.
**Figure 11:** Retinal Fluid status of IAI patients (n=22) in CANDELA Completing to Week 16 at Baseline and Weeks 4, 8, 12 and 16.

**Figure 12:** Retinal Fluid Status of HD Patients in CANDELA Completing to Week 16 at Baseline and Weeks 4, 8, 12 and 16. N=27 at Baseline, Week 8, 12 and 16; N=26 at Week 4.

**Figure 13:** Summary of Treatment Exposure of IAI and HD Patients in CANDELA Trial. IAI n=27; HD n=28.

**Figure 14:** Summary of Treatment Emergent Adverse Events (TEAE) of IAI and HD Patient Study Eye in CANDELA Trial. IAI n=27; HD n=27.

**Figure 15:** Continued Summary of Treatment Emergent Adverse Events (TEAE) of IAI and HD Patients in CANDELA Trial. IAI n=27; HD n=27.

**Figure 16:** Intraocular Pressure (IOP) of HD and IAI Patients in CANDELA trial. IAI n=27; HD n=28.

**Figure 17:** Summary of Non-Ocular Treatment Emergent Adverse Events (TEAE) of IAI and HD Patients in CANDELA trial. IAI n=27; HD n=28.

**Figure 18:** Continued Summary of Non-Ocular Treatment Emergent Adverse Events (TEAE) of IAI and HD Patients in CANDELA Trial. IAI n=27; HD n=28.

**Figure 19:** Summary of Non-Ocular Serious Adverse Events (SAES) of IAI and HD Patients in CANDELA Trial. TIA-transient ischemic attack. IAI n=27; HD n=28.

**Figure 20:** Summary of Hypertension Adverse Events of IAI and HD patients in CANDELA Trial. IAI n=27; HD n=28.

**Figure 21:** Mean Systolic Blood Pressure of IAI and HD Patients in CANDELA trial.

**Figure 22:** Mean Diastolic Blood Pressure of IAI and HD Patients in CANDELA Trial.

**Figure 23:** Mean Intraocular Pressure of IAI and HD Patients in CANDELA Trial.

**Figure 24:** Change in Intraocular Pressure from Baseline of IAI and HD Patients in CANDELA Trial.

**Figures 25A, 25B** and **25C:** Precision Dose Delivery Device Analyses. (Fig. 25A) Delivered dose summary for REGN3-PFS-0.5mL; (Fig. 25B) Delivered dose summary for 1mL BD Luer Lok syringe; (Fig. 25C) Individual value plot of doses delivered with REGN3-PFS-0.5mL and 1mL BD Luer Lok syringe.

**Figure 26:** Disposition and Exposure through week 16 in Patients treated with 8 mg Aflibercept High-Dose Regimen (HD) or 2 mg Aflibercept Regimen (IAI) or All Patients (Total) (analysis of additional patients reaching week 16: HD n=53; IAI n=53; All n=106).

**Figure 27:** Baseline Demographics of in Patients treated with HD, IAI or All Patients (Total) (analysis of additional patients reaching week 16). HD n=53; IAI n=53; All n=106.

**Figure 28:** Baseline Characteristics in Patients treated with HD, IAI or All Patients (analysis of additional patients reaching week 16). HD n=53; IAI n=53; All n=106.

**Figure 29:** Baseline Blood Pressure and Medical History of Hypertension in All Patients treated with HD or IAI (analysis of additional patients reaching week 16; HD n=53; IAI n=53).

**Figure 30:** Primary Efficacy Endpoint - Proportion of Patients Treated with HD or IAI Regimen with Dry Retina at Week 16 (analysis of additional patients reaching week 16; number of patients analyzed is shown).

**Figure 31:** Proportion of Patients Treated with HD or IAI Regimen without Intraretinal Fluid (IRF) and without Subretinal Fluid (SRF) at Week 16 (analysis of additional patients reaching week 16; number of patients analyzed is shown).

**Figure 32:** Median Change from Baseline in Central Retinal Thickness of Patients Treated with HD or IAI Regimen through Week 16 (analysis of additional patients reaching week 16; number of patients analyzed is shown).

**Figure 33:** Mean Change from Baseline in Central Retinal Thickness of Patients Treated with HD or IAI Regimen through Week 16 (analysis of additional patients reaching week 16; number of patients analyzed is shown).

**Figure 34:** Mean Change from Baseline to Week 12 in Choroidal Neovascular Size and Lesion Size of Patients Treated with HD or IAI Regimen. IAI n=44; HD n=46.

**Figure 35:** Mean Change from Baseline in Best Corrected Visual Acuity (BCVA) through Week 16 in Patients Treated with HD or IAI Regimen (analysis of additional patients reaching week 16; number of patients analyzed is shown).

**Figure 36:** Changes in Vision at Week 16 in Patients Treated with HD or IAI Regimen (analysis of additional patients reaching week 16; number of patients analyzed is shown).

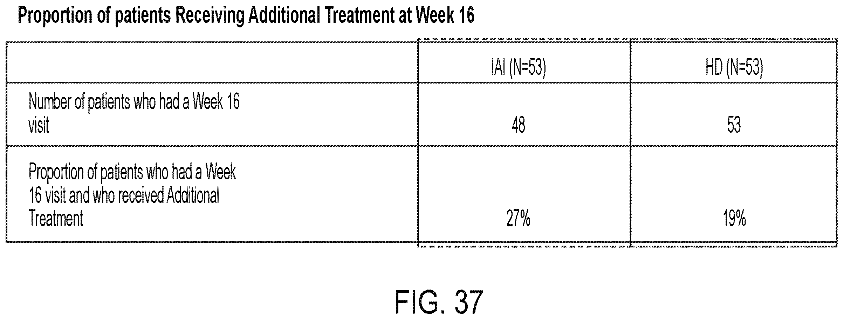

**Figure 37:** Proportion of Patients Treated with HD or IAI Regimen Receiving Additional Treatment at Week 16 (analysis of additional patients reaching week 16; HD n=53; IAI n=53).

**Figures 38A, 38B** and **38C:** Summary of Treatment Emergent Adverse Events (TEAEs) in Patients Treated with HD or IAI Regimen, (Fig. 38A) Ocular TEAEs in Study Eye Occurring in ≥2% of Patients through Week 16, (Fig. 38B) Ocular Serious TEAEs in the Study Eye through Week 16, (Fig. 38C) Intraocular Inflammation TEAEs in the Study Eye through Week 16 (analysis of additional patients reaching week 16; HD n=53; IAI n=53).

**Figure 39:** Mean Intraocular Pressure Change from Baseline in Patients Treated with HD or IAI Regimen through Week 16 (analysis of additional patients reaching week 16; number of patients analyzed is shown).

**Figure 40:** Summary of Intraocular Pressure Events in Patients Treated with HD or IAI Regimen through Week 16 (analysis of additional patients reaching week 16; HD n=53; IAI n=53).

**Figure 41:** Anti-Platelet Trialists' Collaboration (APTC) Events or Deaths in Patients Treated with HD or IAI Regimen through Week 16 (analysis of additional patients reaching week 16; HD n=53; IAI n=53).

**Figure 42:** Hypertension Adverse Events in Patients Treated with HD or IAI Regimen through Week 16 (analysis of additional patients reaching week 16; HD n=53; IAI n=53).

**Figures 43A** and **43B:** Mean Change from Baseline in Blood Pressure (Fig. 43A: Systolic; Fig. 43B: Diastolic) in Patients Treated with HD or IAI Regimen through Week 16-All Patients (analysis of additional patients reaching week 16; HD n=53; IAI n=53).

**Figures 44A and 44B:** Mean Change from Baseline in Blood Pressure (Fig. 44A: Systolic; Fig. 44B: Diastolic) in Patients Treated with HD or IAI Regimen through Week 16-Dense Pharmacokinetic (PK) Sub-study Patients (analysis of additional patients reaching week 16; HD n=15; IAI n=15).

**Figure 45:** Patient Disposition as of study start, week 16 and week 44 of HD and IAI patient study groups and combined groups (All).

**Figure 46:** Baseline Demographics (sex, ethnicity, race, age) of HD and IAI patient study groups and combined groups (All).

**Figure 47:** Baseline Characteristics of the Study Eye of HD and IAI patient study groups and combined groups (All). 58 ETDRS letters is about equivalent to 20/60-20/70 Snellen visual acuity. SD=standard deviation; FA=fluorescein angiography.

**Figure 48:** Treatment Exposure summary through Week 44 of HD and IAI patient study groups.

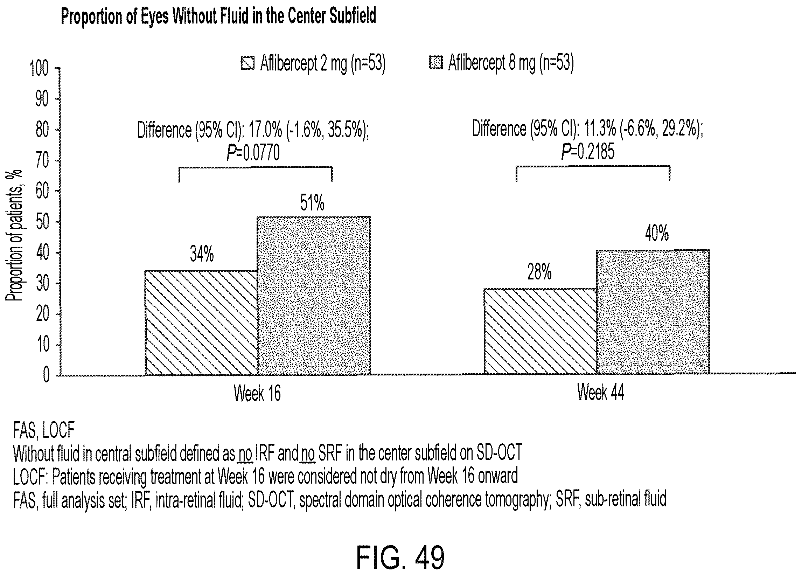

**Figure 49:** Proportion of Eyes without Fluid in the Center Subfield (no intraretinal fluid (IRF) and no subretinal fluid (SRF) in the center subfield by SD-OCT (Spectral Domain Optical Coherence Tomography)) at Weeks 16 & 44. Percentages and number of patients out of 53 (n/53) are shown. LOCF=last observation carried forward. FAS=full analysis set. At baseline, 9/53 (17.0%) and 10/53 (18.9%) of eyes in the aflibercept 2 mg and 8 mg groups, respectively, had no IRF or SRF in the center subfield. These eyes met criteria for inclusion due to presence of pigment epithelial detachment (PED) due to nAMD

**Figure 50:** Proportion of Eyes without Fluid in the Macula (no IRF and no SRF in the macula on SD-OCT) at week 16 (difference of 17 (95% confidence interval: -0.9, 34.8)) and week 44 (difference of 17 (95% confidence interval: 1.1, 32.8)). Percentages and number of patients out of 53 (n/53) are shown.

**Figure 51:** Proportion of Eyes without Fluid in the Center Subfield (without fluid in the center subfield refers to no intraretinal fluid and no subretinal fluid in the center subfield on SD-OCT (Spectral Domain Optical Coherence Tomography)) at weeks 16, 20, 24 28, 32, 36, 40 and 44. Percentages and number of patients out of 53 (n/53) are shown. Syringes on the X-axis indicate scheduled dose visits.

**Figure 52:** Proportion of Eyes without Intraretinal Fluid (without intraretinal fluid is dry or with subretinal fluid only) in the Center Subfield at Weeks 16 and 44. Percentages and number of patients out of 53 (n/53) are shown.

**Figure 53:** Proportion of Eyes without Subretinal Fluid (without subretinal fluid is dry or with intraretinal fluid only) in the Center Subfield at Weeks 16 and 44. The week 16 and 44 treatment effect was 18.9%. Percentages and number of patients out of 53 (n/53) are shown.

**Figure 54:** Mean Change from Baseline (BL) in Central Retinal Thickness (CRT; micrometers) over time through Week 44. Syringes on the X-axis indicate scheduled dose visits. Week 16 data for patients receiving treatment at week 16 are carried forward. LOCF: Patients receiving treatment at week 16 were considered not dry from week 16 onward. Inset box shows changes in CRT between weeks 12 and 20; 24 and 32; or 36 and 44 in the HD and IAI groups.

**Figure 55:** Median Change from Baseline (BL) in Central Retinal Thickness (CRT; micrometers) over time through Week 44. Syringes on the X-axis indicate scheduled dose visit. LOCF: Patients receiving treatment at week 16 were considered not dry from week 16 onward.

**Figure 56:** Mean Change from Baseline (BL) in Best Corrected Visual Acuity (BCVA; ETDRS letters) over time through Week 44. Syringes on the X-axis indicate scheduled dose visit. LOCF: Patients receiving treatment at week 16 were considered not dry from week 16 onward.

**Figure 57:** Proportion of Patients Losing and Gaining Vision (ETDRS letters) from Baseline (BL) at Week 44. Percentages and number of patients out of 53 (n/53) are shown.

**Figure 58:** Ocular Treatment Emergent Adverse Events (TEAEs) in the Study Eye Occurring in > 2% of Patients (Pts). Percentages and number of patients out of 53 (n/53) in HD and IAI groups are shown.

**Figure 59:** Ocular Serious Treatment Emergent Adverse Events (TEAEs) in the Study Eye. Percentages and number of patients out of 53 (n/53) in HD and IAI groups are shown.

**Figure 60:** Intraocular Inflammation Treatment Emergent Adverse Events (TEAEs) in the Study Eye. Percentages and number of patients out of 53 (n/53) in HD and IAI groups are shown.

**Figure 61:** Summary of Intraocular Pressure Events in the HD and IAI Study Groups. Safety analysis set. Percentages are calculated based on N. Percentages and number of patients out of 53 (n/53) in HD and IAI groups are shown.

**Figure 62:** Mean Pre-Dose Intraocular Pressure, Change from Baseline in the HD and IAI study groups over time (mmHg). Safety analysis set.

**Figure 63:** Non-Ocular Treatment Emergent Adverse Events (TEAEs) Occurring in $\geq$ 2% of Patients. Percentages and number of patients out of 53 (n/53) in HD and IAI groups are shown.

**Figure 64:** Non-Ocular Serious Treatment Emergent Adverse Events (TEAEs) in the HD and IAI study groups. Percentages and number of patients out of 53 (n/53) are shown.

**Figure 65:** Hypertension Treatment Emergent Adverse Events (TEAEs) in the HD and IAI study groups. Percentages and number of patients out of 53 (n/53) in HD and IAI groups are shown.

**Figure 66:** Adjudicated Anti-Platelet Trialists' Collaboration (APTC) Events and Deaths in the HD and IAI study groups.

## DETAILED DESCRIPTION OF THE INVENTION

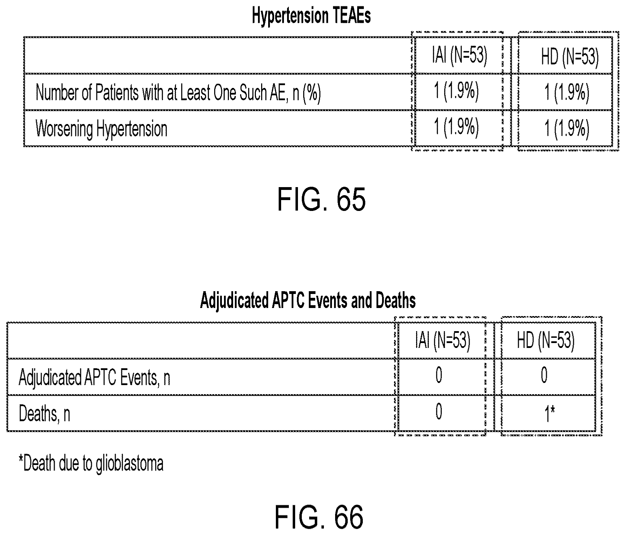

Increasing the molar fraction of VEGF antagonist therapeutic protein in the dosing formulation is a potential way to bring further benefits to patients with chorioretinal vascular diseases, including nAMD. A higher dose of aflibercept administered IVT has the potential to prolong the drug's therapeutic effects and for improvement in pharmacodynamics such as better drying. The resulting extension of treatment intervals early after the initiation of treatment to every 12 weeks would reduce the number of injections in the first treatment year. A potential decrease in injection-related treatment burden and safety events with fewer injections could be a significant contribution to patient care and healthcare services. The present invention provides, in part, a safe and effective method for treating angiogenic eye disorders with an 8 mg dose of aflibercept in a regimen calling for monthly loading doses before quarterly maintenance doses. First, patients receiving an 8 mg dose, which is four times the dose approved for Eylea, (2 mg), were not observed in the CANDELA clinical trial (discussed herein) to develop any more hypertension treatment-emergent adverse events than patients receiving 2 mg (Figure 65). Systemic exposure to VEGF inhibitor is known to be associated with the risk of systemic blood pressure (BP) increases. Moreover, intravitreal injection of VEGF inhibitors has been associated with blood pressure effects. See, for example, Hanna et al., (2019) "Three patients with injection of intravitreal vascular endothelial growth factor inhibitors and subsequent exacerbation of chronic proteinuria and hypertension", Clinical Kidney Journal, 2019, vol. 12, no. 1, 92-100 and Raiser et al., (2009) "The effect of intravitreal bevacizumab (Avastin) administration on systemic hypertension", Eye 23, 1714-1718. In addition, in the CANDELA clinical trial, subjects receiving the 8 mg dose (HD) achieved numerically superior anatomical improvements in the eye as well as numerically superior improvements in vision relative to subjects receiving a 2 mg dose (IAI). A higher proportion of eyes treated with aflibercept 8 mg (HD) were dry (without intraretinal or subretinal fluid on OCT) in the center subfield versus aflibercept 2 mg (IAI). Treatment groups followed identical dosing regimens with the 8 mg group receiving slightly fewer PRN doses. A change from baseline in central retinal thickness (CRT) suggested better anatomic outcomes with aflibercept 8 mg versus aflibercept 2 mg; and a change from baseline in BCVA favored aflibercept 8 mg (+7.9 vs +5.1 letters).

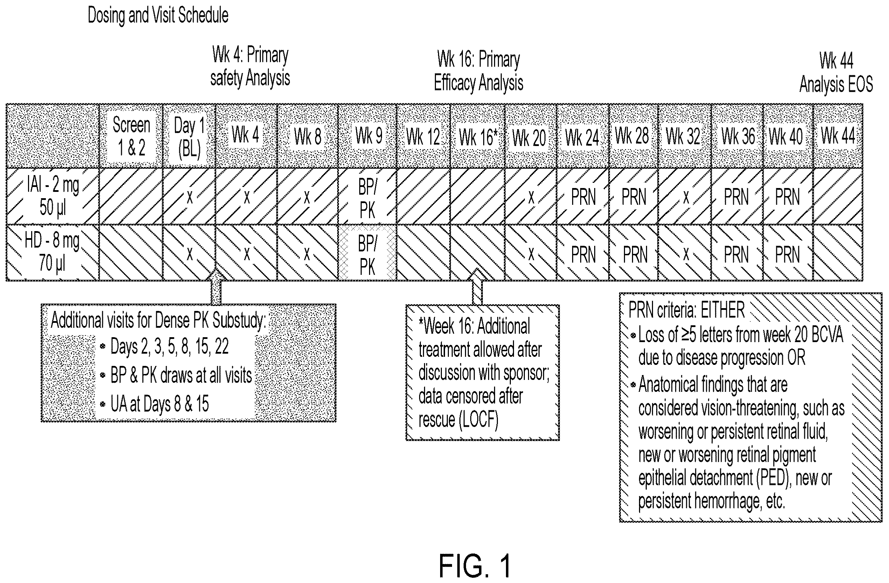

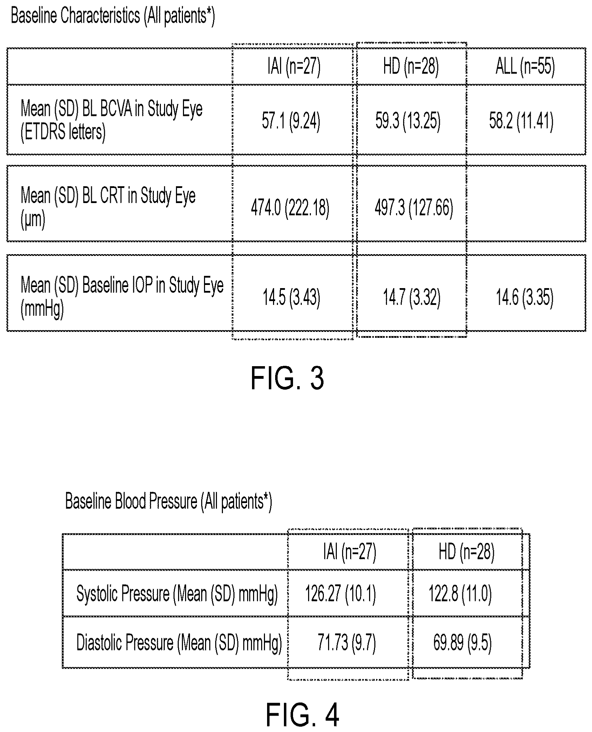

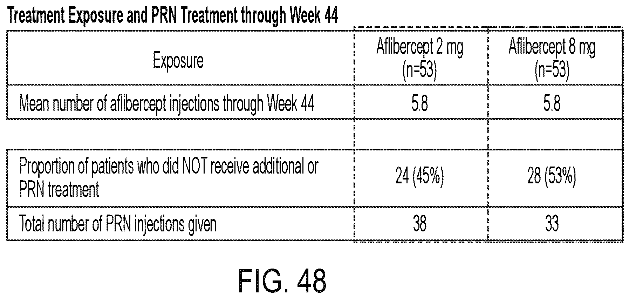

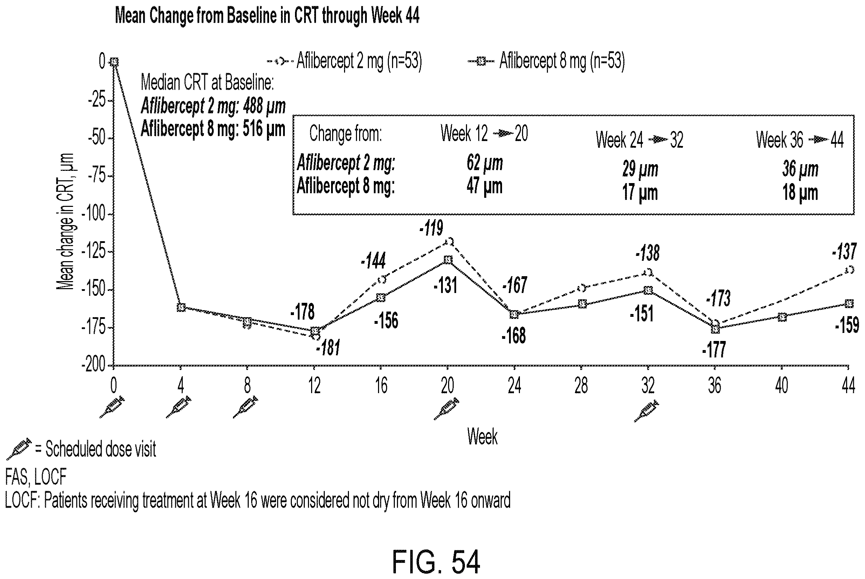

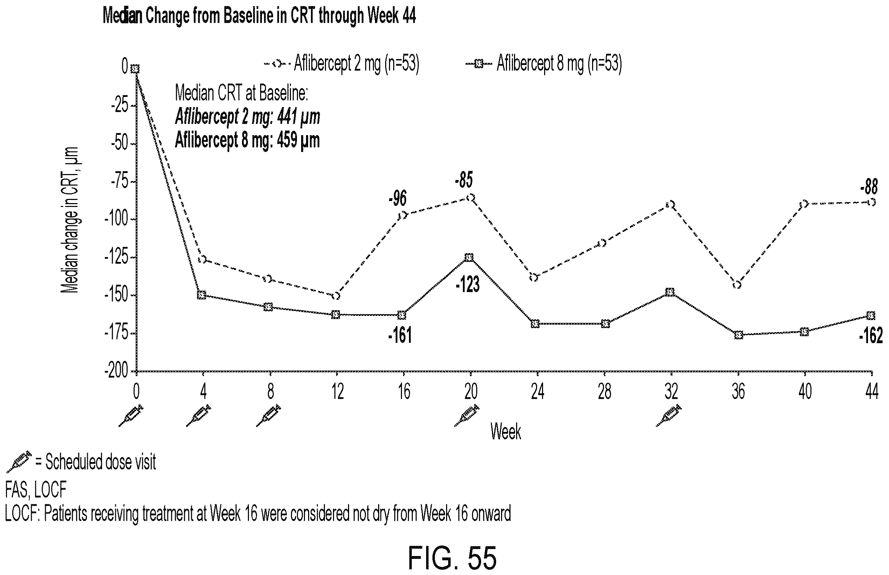

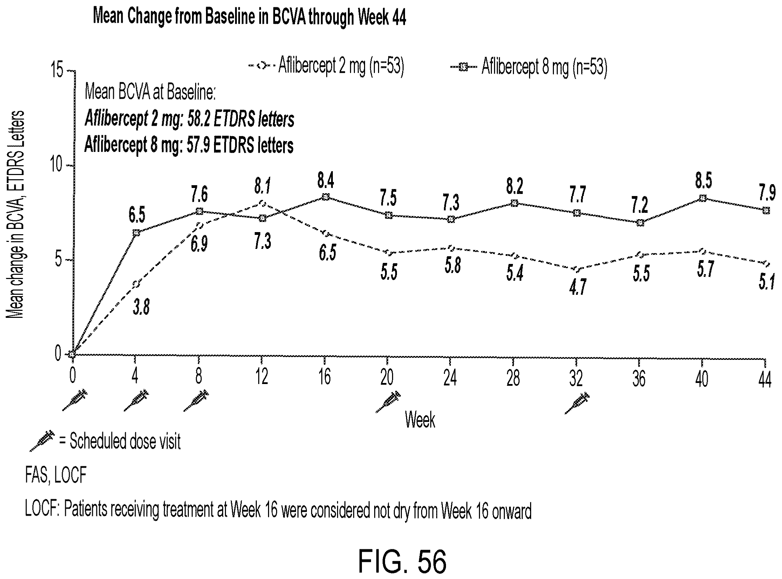

The anatomical and visual improvements for the HD patients that was observed was also comparable to those of subjects in the VIEW1 and VIEW2 trials (VIEW1/2) that received a 2 mg dose every 8 weeks (following three monthly loading doses (2q8)). See *e.g.*, Heier et al., Intravitreal Aflibercept (VEGF Trap-Eye) in Wet Age-related Macular Degeneration, Ophthalmology 2012;119:2537-2548. The VIEW1/2 2q8 subjects achieved 8.1 letters of BCVA mean improvement at 44 weeks following the initial dose (Heier *et al.* (2012), Figure 3); whereas the CANDELA HD subjects herein were observed to achieve a mean improvement of 7.9 letters at 44 weeks (Figure 56). Moreover, the improvements in the central retinal thickness (CRT) that were observed to be achieved by CANDELA HD subjects were comparable to that observed in the VIEW1/2 2q8 subjects. The VIEW1 2q8 subjects achieved a mean reduction of about 125 micrometers in CRT and VIEW2 2q8 subjects achieved about 150 micrometers mean reduction in CRT, at 44 weeks (Heier *et al.* (2012), Figure 4). The CANDELA HD subjects were observed to achieve a mean reduction of 159 micrometers and a median reduction of 162 micrometers at 44 weeks (Figure 54 & Figure 55; and Heier *et al.* (2012), Figure 4). While the 2q8 VIEW1/2 subjects and the CANDELA HD subjects were not evaluated side-by-side in the same clinical trial and the VIEW trials had a greater number of participants, these data suggest that subjects can be administered 8 mg doses of aflibercept as infrequently as every 12 weeks yet achieve anatomic and visual outcomes comparable to that of patients dosed every 8 weeks (following three monthly loading doses) with 2 mg. While the VIEW1/2 subjects received only the scheduled doses within the first year, some of the CANDELA subjects received additional doses *pro re nata* after week 16 (Figure 1). Twenty eight out of 53 of the CANDELA HD subjects did not receive any additional doses (Figure 48). Moreover, time-domain optical coherence tomography was performed in VIEW1/2 to evaluated CRT whereas, a more sensitive spectral domain optical coherence tomography (SD-OCT) was used in CANDELA.

"Isolated" VEGF antagonists and VEGF receptor fusion proteins (*e.g.*, aflibercept), polypeptides, polynucleotides and vectors, are at least partially free of other biological molecules from the cells or cell culture from which they are produced. Such biological molecules include nucleic acids, proteins, other VEGF antagonists and VEGF receptor fusion proteins, lipids, carbohydrates, or other material such as cellular debris and growth medium. An isolated VEGF antagonist or VEGF receptor fusion protein may further be at least partially free of expression system components such as biological molecules from a host cell or of the growth medium thereof. Generally, the term "isolated" is not intended to refer to a complete absence of such biological molecules (*e.g.*, minor or insignificant amounts of impurity may remain) or to an absence of water, buffers, or salts or to components of a pharmaceutical formulation that includes the VEGF antagonists or VEGF receptor fusion proteins.

A "subject" or "patient" is a mammal, for example a human, mouse, rabbit, monkey or non-human primate. A subject or patient may be said to be "suffering from" an angiogenic eye disorder such as nAMD, DR or DME. Such a subject has the disorder in one or both eyes. In an embodiment of the invention, a subject or patient has one or more of the following characteristics (at or before the start of treatment):

1. ≥50 years of age
2. Subfoveal CNV secondary to nAMD, *e.g.*, including juxtafoveal lesions that affect the fovea in an eye.
3. Best Corrected Visual Acuity (BCVA) Early Treatment Diabetic Retinopathy Study (ETDRS) letter score of 78 to 24 (Snellen equivalent of 20/32 to 20/320) in an eye; or about 58±10, 58 ±14 or 58±12 ETDRS letters (or Snellen equivalent).
4. A central retinal thickness in an eye greater than normal, *e.g.*, greater than approximately 130, 140, 150, 160, 170 or 180 micrometers, *e.g.,* as determined manually or by optical coherence tomography (OCT) mapping software, for example with a central retinal thickness of about 300, 400, 500 or 600 micrometers or more; or about 488.1±204.9, 516.2±175.64 or 502.1±190.6 micrometers.
5. Intraocular pressure of about 14.8±3.4 or 14.9±3.4 mmHg.
6. nAMD lesion size of about 7.9±6.21, 7.7±6.84 or 7.8±6.50 mm$^2$.
7. Choroidal neovascularization lesion size of about 7.9±6.20, 7.5±6.86 or 7.7±6.51 mm$^2$.
8. As per fluorescein angiography (FA), has occult choroidal neovascularization, minimally classic choroidal neovascularization or predominantly classic choroidal neovascularization. and/or, has or lacks any one or more of the following characteristics:

1. CNV (choroidal neovascularization) due to any cause other than nAMD in either eye.
2. Subretinal hemorrhage in an eye that is ≥50% of the total lesion area.
3. Intraocular pressure ≥25 mm Hg in an eye.
4. Evidence of infectious blepharitis, keratitis, scleritis, or conjunctivitis in an eye.
5. Any intraocular inflammation and/or ocular infection in an eye.
6. Any history of macular hole of stage 2 and above in an eye.
7. Iris neovascularization, vitreous hemorrhage, or tractional retinal detachment visible in an eye.
8. Uncontrolled blood-pressure (BP) (defined as systolic >140 mm Hg or diastolic >90 mm Hg).
9. Variation by more than 10% in the 3 pre-randomization BP measurements.
10. History of cerebrovascular accident/ transient ischemic attack or myocardial infarction/ acute coronary syndrome.
11. Renal failure, dialysis, or history of renal transplant.
12. Known sensitivity to aflibercept.
13. Any other intraocular surgery within 12 weeks (84 days).

**VEGF Antagonists**

The present invention includes methods for using a VEGF antagonist for treating or preventing angiogenic eye disorders. VEGF antagonists include molecules which interfere with the interaction between VEGF and a natural VEGF receptor, *e.g.*, molecules which bind to VEGF or a VEGF receptor and prevent or otherwise hinder the interaction between VEGF and a VEGF receptor. Specific, exemplary VEGF antagonists include anti-VEGF antibodies, anti-VEGF receptor antibodies, and VEGF receptor fusion proteins.

For purposes herein, a "VEGF receptor fusion protein" refers to a molecule that comprises one or more VEGF receptors or domains thereof, fused to another polypeptide, which interferes with the interaction between VEGF and a natural VEGF receptor, *e.g.*, wherein two of such fusion polypeptides are associated thereby forming a homodimer or other multimer. Such VEGF receptor fusion proteins may be referred to as a "VEGF-Trap" or "VEGF Trap". VEGF receptor fusion proteins within the context of the present disclosure that fall within this definition include chimeric polypeptides which comprise two or more immunoglobulin (Ig)-like domains of a VEGF receptor such as VEGFR1 (also known as Flt1) and/or VEGFR2 (also known as Flk1 or KDR), and may also contain a multimerizing domain (for example, an Fc domain).

An exemplary VEGF receptor fusion protein is a molecule referred to as VEGF1R2-FcΔC1(a) which is encoded by the nucleic acid sequence of SEQ ID NO:1 or nucleotides 79-1374 or 79-1371 thereof.

VEGF1R2-FcΔC1(a) comprises three components:

(1) a VEGFR1 component comprising amino acids 27 to 129 of SEQ ID NO:2;
(2) a VEGFR2 component comprising amino acids 130 to 231 of SEQ ID NO:2; and

(3) a multimerization component ("FcΔC1(a)") comprising amino acids 232 to 457 of SEQ ID NO:2 (the C-terminal amino acids of SEQ ID NO:2, *i.e.*, K458, may or may not be included in the VEGF receptor fusion proteins, see U.S. Patent No. 7,396,664 or 7,354,579, incorporated herein for all purposes). Note that amino acids 1 to 26 of SEQ ID NO:2 are the signal sequence.

If the multimerizing component (MC) of a VEGF receptor fusion protein is derived from an IgG (*e.g.,* IgG1) Fc domain, then the MC has no fewer amino acids than are in amino acids 232 to 457 of SEQ ID NO:2. Thus, the IgG of the MC cannot be truncated to be shorter than 226 amino acids.

In an embodiment of the invention, the VEGF receptor fusion protein comprises amino acids 27-458 or 27-457 of SEQ ID NO: 2.

```
atggtcagctactgggacaccggggtcctgctgtgcgcgctgctcagctgtctgcttctcacaggatctagttccgg

aagtgataccggtagacctttcgtagagatgtacagtgaaatccccgaaattatacatgactgaaggaagggagc

tcgtcattccctgccgggttacgtcacctaacatcactgttactttaaaaaagtttccacttgacactttgatccct

gatggaaaacgcataatctgggacagtagaaagggcttcatcatatcaaatgcaacgtacaaagaaatagggcttct

gacctgtgaagcaacagtcaatgggcatttgtataagacaaactatctcacacatcgacaaaccaatacaatcatag

atgtggttctgagtccgtctcatggaattgaactatctgttggagaaaagcttgtcttaaattgtacagcaagaact

gaactaaatgtggggattgacttcaactgggaataccttcttcgaagcatcagcataagaaacttgtaaaccgaga

cctaaaaacccagtctgggagtgagatgaagaaatttttgagcaccttaactatagatggtgtaacccggagtgacc

aaggattgtacctgtgcagcatccagtgggctgatgaccaagaagaacagcacatttgtcagggtccatgaaaag

gacaaaactcacacatgcccaccgtgcccagcacctgaactcctggggggaccgtcagtcttcctcttccccccaaa

acccaaggacaccctcatgatctcccggacccctgaggtcacatgcgtggtggtggacgtgagccacgaagaccctg

aggtcaagttcaactggtacgtggacggcgtggaggtgcataatgccaagacaaagccgcgggaggagcagtacaac

agcacgtaccgtgtggtcagcgtcctcaccgtcctgcaccaggactggctgaatggcaaggagtacaagtgcaaggt

ctccaacaaagccctcccagcccccatcgagaaaaccatctccaaagccaaagggcagccccgagaaccacaggtgt

acaccctgcccccatcccgggatgagctgaccaagaaccaggtcagcctgacctgcctggtcaaaggcttctatccc

agcgacatcgccgtggagtgggagagcaatgggcagccggagaacaactacaagaccacgcctcccgtgctggactc

cgacggctccttcttcctctacagcaagctcaccgtggacaagagcaggtggcagcagggggaacgtcttctcatgct

ccgtgatgcatgaggctctgcacaaccactacacgcagaagagcctctccctgtctccgggtaaatga
```
(SEQ ID NO: 1)

```
MVSYWDTGVLLCALLSCLLLTGSSSGSDTGRPFVEMYSEIPEIIHMTEGRELVIPCRVTS

PNITVTLKKFPLDTLIPDGKRIIWDSRKGFIISNATYKEIGLLTCEATVNGHLYKTNYLT

HRQTNTIIDVVLSPSHGIELSVGEKLVLNCTARTELNVGIDFNWEYPSSKHQHKKLVNRD

LKTQSGSEMKKFLSTLTIDGVTRSDQGLYTCAASSGLMTKKNSTFVRVHEKDKTHTCPPC

PAPELLGGPSVFLFPPKPKDTLMISRTPEVTCVVVDVSHEDPEVKFNWYVDGVEVHNAKT

KPREEQYNSTYRVVSVLTVLHQDWLNGKEYKCKVSNKALPAPIEKTISKAKGQPREPQVY

TLPPSRDELTKNQVSLTCLVKGFYPSDIAVEWESNGQPENNYKTTPPVLDSDGSFFLYSK

LTVDKSRWQQGNVFSCSVMHEALHNHYTQKSLSLSPGK
```

(SEQ ID NO: 2; signal sequence underscored-missing in mature, processed aflibercept; optionally, lacking C-terminal Lysine)

In an embodiment of the invention, aflibercept is N-glycosylated at any one or more of Asparagines 62, 94, 149, 222 and 308.

In an embodiment of the invention, the VEGF receptor fusion protein comprises

(1) an immunoglobin-like (Ig) domain 2 of a first VEGF receptor (*e.g.*, VEGFR1), and

(2) an Ig domain 3 of a second VEGF receptor (*e.g.*, VEGFR2),

(3) and, optionally, further including an Ig domain 4 of the second VEGF receptor (*e.g.*, VEGFR2) and

(4) a multimerizing component (*e.g.*, Fc domain of IgG including the hinge, CH2 and CH3 domains).

For example, in an embodiment of the invention, the VEGF receptor fusion protein has the following arrangement of said domains:

- [VEGFR1 Ig domain 2]-[VEGFR2 Ig domain 3]-[MC] (*e.g.*, a homodimer thereof) or
- [VEGFR1 Ig domain 2]-[VEGFR2 Ig domain 3]-[VEGFR2 Ig domain 4]-[MC] (*e.g.*, a homodimer thereof).

Note that the present disclosure also includes, within its scope, high concentration formulations including, instead of a VEGF receptor fusion protein, a VEGF binding molecule or anti-VEGF antibody or antigen-binding fragments thereof or biopolymer conjugate thereof (*e.g.*, KSI-301) and uses thereof as discussed, *e.g.*,

- bevacizumab (*e.g.*, at a concentration of about 80-90 or 88 mg/ml),
- ranibizumab (*e.g.*, at a concentration of about 20-40 mg/ml, *e.g.*, 21-35, 21 or 35 mg/ml),
- an anti-VEGF aptamer such as pegaptanib (*e.g.*, pegaptanib sodium),
- a single chain (*e.g.*, $V_L$-$V_H$) anti-VEGF antibody such as brolucizumab (*e.g.*, at a concentration of about 200-400 or 200, 210, 400 or 420 mg/ml),
- an anti-VEGF DARPin such as the Abicipar Pegol DARPin (*e.g.*, at a concentration of about 70-140, 70 or 140 mg/ml), or
- a bispecific anti-VEGF antibody, *e.g.*, which also binds to ANG2, such as RG7716 (faricimab) (*e.g.*, at a concentration of about 100-400, 100, 105, 400 or 420 mg/ml).

In order to minimize the repetitiveness of the embodiments discussed herein, it is contemplated that the scope of the present invention includes embodiments wherein any of the formulations discussed herein include, in place of a VEGF receptor fusion protein, an anti-VEGF antibody or antibody fragment or other VEGF binding molecule as discussed herein (*e.g.*, substituted with an anti-VEGF DARPin) at any of the concentrations discussed herein. For example, the present invention includes a formulation having 35 or 80 mg/ml ranibizumab, a buffer, a thermal stabilizer, a viscosity reducing agent and a surfactant.

DARPins are Designed Ankyrin Repeat Proteins. DARPins generally contain three to four tightly packed repeats of approximately 33 amino acid residues, with each repeat containing a β-turn and two anti-parallel α-helices. This rigid framework provides protein stability whilst enabling the presentation of variable regions, normally comprising six amino acid residues per repeat, for target recognition.

An "anti-VEGF" antibody or antigen-binding fragment of an antibody refers to an antibody or fragment that specifically binds to VEGF.

Illustrative VEGF receptor fusion proteins include aflibercept (EYLEA®, Regeneron Pharmaceuticals, Inc.) or conbercept (sold commercially by Chengdu Kanghong Biotechnology Co., Ltd.). See International patent application publication no. WO2005/121176 or WO2007/112675. The terms "aflibercept" and "conbercept" include biosimilar versions thereof. A biosimilar version of a reference product (*e.g.*, aflibercept) generally refers to a product comprising the identical amino acid sequence but includes products which are biosimilar under the U.S. Biologics Price Competition and Innovation Act.

**Pharmaceutical Formulations**

The present invention includes methods in which the VEGF antagonist (e.g., aflibercept) that is administered to the patient's eye is contained within a pharmaceutical formulation. The pharmaceutical formulation includes a VEGF antagonist along with a pharmaceutically acceptable carrier. Other agents may be incorporated into the pharmaceutical formulation to provide improved transfer, delivery, tolerance, and the like. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the VEGF antagonist is administered. A multitude of appropriate formulations can be found in the formulary known to all pharmaceutical chemists: Remington's Pharmaceutical Sciences (15th ed, Mack Publishing Company, Easton, Pa., 1975), e.g., Chapter 87 by Blaug, Seymour, therein.

Pharmaceutical formulations for use in a method of the present invention can be "high concentration". High concentration pharmaceutical formulations of the present invention include VEGF antagonist, *e.g.*, VEGF receptor fusion protein (e.g., aflibercept), at a concentration of greater than 40 mg/ml, at least 41 mg/ml, of at least 80 mg/ml, of at least 100 mg/ml, of at least 125 mg/ml, of at least 140 mg/ml, of at least 150 mg/ml, of at least 175 mg/ml, of at least 200 mg/ml, of at least 225 mg/ml, of at least 250 mg/ml, or of at least 275 mg/ml. "High concentration" can refer to formulations that include a concentration of VEGF antagonist of from about 140 mg/ml to about 160 mg/ml, at least about 140 mg/ml but less than 160 mg/ml, from about 41 mg/ml to about 275 mg/ml, from about 70 mg/ml to about 75 mg/ml or from about 80 mg/ml to about 250 mg/ml. In some aspects, the VEGF antagonist concentration in the formulation is about any of the following concentrations: 41 mg/ml; 42 mg/ml; 43 mg/ml; 44 mg/ml; 45 mg/ml; 46 mg/ml; 47 mg/ml; 48 mg/ml; 49 mg/ml; 50mg/ml; 51 mg/ml; 52 mg/ml; 53 mg/ml; 54 mg/ml; 55 mg/ml; 56 mg/ml; 57 mg/ml; 58 mg/ml; 59 mg/ml; 60 mg/ml; 61 mg/ml; 62 mg/ml; 63 mg/ml; 64 mg/ml; 65 mg/ml; 66 mg/ml; 67 mg/ml; 68 mg/ml; 69 mg/ml; 70 mg/ml; 71 mg/ml; 72 mg/ml; 73 mg/ml; 74 mg/ml; 75 mg/ml; 76 mg/ml; 77 mg/ml; 78 mg/ml; 79 mg/ml; 80 mg/ml; 81 mg/ml; 82mg/ml; 83 mg/ml; 84 mg/ml; 85 mg/ml; 86 mg/ml; 87mg/ml; 88 mg/ml; 89 mg/ml; 90 mg/ml; 91 mg/ml; 92 mg/ml; 93 mg/ml; 94 mg/ml; 95mg/ml; 96 mg/ml; 97 mg/ml; 98 mg/ml; 99 mg/ml; 100 mg/ml; 101 mg/ml; 102 mg/ml; 103 mg/ml; 104 mg/ml; 105 mg/ml; 106mg/ml; 107 mg/ml; 108 mg/ml; 109 mg/ml; 110 mg/ml; 111 mg/ml; 112 mg/ml; 113 mg/ml; 113.3 mg/ml; 114 mg/ml; 114.1 mg/ml; 114.2 mg/ml; 114.3 mg/ml; 114.4 mg/ml; 114.5 mg/ml; 114.6 mg/ml, 114.7 mg/ml; 114.8 mg/ml; 114.9 mg/ml; 115 mg/ml; 116 mg/ml; 117 mg/ml; 118 mg/ml; 119 mg/ml; 120 mg/ml; 121 mg/ml; 122 mg/ml; 123 mg/ml; 124 mg/ml; 125 mg/ml; 126 mg/ml; 127mg/ml; 128 mg/ml; 129 mg/ml; 130 mg/ml; 131 mg/ml; 132 mg/ml; 133 mg/ml; 133.3 mg/ml; 133.4 mg/ml, 134 mg/ml; 135 mg/ml; 136 mg/ml; 137 mg/ml; 138 mg/ml; 139 mg/ml; 140 mg/ml; 141 mg/ml; 142 mg/ml; 143 mg/ml; 144 mg/ml; 145 mg/ml; 146 mg/ml; 147 mg/ml; 148 mg/ml; 149 mg/ml; 150 mg/ml; 151 mg/ml; 152 mg/ml; 153 mg/ml; 154mg/ml; 155 mg/ml; 156 mg/ml; 157mg/ml; 158 mg/ml; 159 mg/ml; 160 mg/ml; 161 mg/ml; 162 mg/ml; 163 mg/ml; 164 mg/ml; 165 mg/ml; 166 mg/ml; 167 mg/ml; 168 mg/ml; 169 mg/ml; 170 mg/ml; 171 mg/ml; 172 mg/ml; 173 mg/ml; 174 mg/ml; 175 mg/ml; 176 mg/ml; 177 mg/ml; 178 mg/ml; 179 mg/ml; 180 mg/ml; 181 mg/ml; 182 mg/ml; 183 mg/ml; 184 mg/ml; 185 mg/ml; 186 mg/ml; 187 mg/ml; 188 mg/ml; 189 mg/ml; 190 mg/ml; 191 mg/ml; 192 mg/ml; 193 mg/ml; 194 mg/ml; 195 mg/ml; 196 mg/ml; 197 mg/ml; 198 mg/ml; 199 mg/ml; 200 mg/ml; 201 mg/ml; 202 mg/ml; 203 mg/ml; 204 mg/ml; 205 mg/ml; 206 mg/ml; 207 mg/ml; 208 mg/ml; 209 mg/ml; 210 mg/ml; 211 mg/ml; 212 mg/ml; 213 mg/ml; 214 mg/ml; 215 mg/ml; 216 mg/ml; 217 mg/ml; 218 mg/ml; 219 mg/ml; 220 mg/ml; 221 mg/ml; 222 mg/ml; 223 mg/ml; 224 mg/ml; 225 mg/ml; 226 mg/ml; 227 mg/ml; 228 mg/ml; 229 mg/ml; 230 mg/ml; 231 mg/ml; 232 mg/ml; 233 mg/ml; 234 mg/ml; 235 mg/ml; 236 mg/ml; 237mg/ml; 238 mg/ml; 239 mg/ml; 240 mg/ml; 241 mg/ml; 242 mg/ml; 243 mg/ml; 244 mg/ml; 245 mg/ml; 246 mg/ml; 247 mg/ml; 248 mg/ml; 249 mg/ml; 250 mg/ml; 251 mg/ml; 252 mg/ml; 253 mg/ml; 254 mg/ml; 255 mg/ml; 256 mg/ml; 257 mg/ml; 258 mg/ml; 259 mg/ml; 260 mg/ml; 261 mg/ml; 262 mg/ml; 263 mg/ml; 264 mg/ml; 265 mg/ml; 266 mg/ml; 267 mg/ml; 268 mg/ml; 269 mg/ml; 270 mg/ml; 271 mg/ml; 272 mg/ml; 273 mg/ml; 274 mg/ml; or 275 mg/ml. Other VEGF antagonist concentrations are contemplated herein, as long as the concentration functions in accordance with embodiments herein.

In an embodiment of the invention, a pharmaceutical formulation for use in a method of the present invention is of such a concentration as to contain about 4, 6, 8, 10, 12, 14, 16, 18 or 20 mg VEGF receptor fusion protein (*e.g.*, aflibercept), or the amount of such protein in any of the acceptable doses thereof which are discussed herein, in about 100 μl or less, about 75 μl or less or about 70 μl or less, *e.g.*, about 50 μl; 51 μl; 52 μl; 53 μl; 54 μl; 55 μl; 56 μl; 57 μl; 58 μl; 59 μl; 60 μl; 61 μl; 62 μl; 63 μl; 64 μl; 65 μl; 66 μl; 67 μl; 68 μl; 69 μl; 70 μl; 71 μl; 72 μl; 73 μl; 74 μl; 75 μl; 76 μl; 77 μl; 78 μl; 79 μl; 80 μl; 81 μl; 82 μl; 83 μl; 84 μl; 85 μl; 86 μl; 87 μl; 88 μl; 89 μl; 90 μl; 91 μl; 92 μl; 93 μl; 94 μl; 95 μl; 96 μl; 97 μl; 98 μl; 99 μl; or 100 μl.

The present invention includes methods of using (as discussed herein) any of the formulations set forth under *"Illustrative Formulations"* herein, but wherein the concentration of the VEGF receptor fusion protein (*e.g.*, aflibercept) is substituted with a concentration which is set forth in this section ("VEGF Receptor Fusion Proteins and Other VEGF inhibitors").

Buffers for use in pharmaceutical formulations herein that may be used in a method of the present invention refer to solutions that resist pH change by use of acid-base conjugates. Buffers are capable of maintaining pH in the range of from about 5.0 to about 6.8, and more typically, from about 5.8 to about 6.5, and most typically, from about 6.0 to about 6.5. In some cases, the pH of the formulation of the present invention is about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, or about 6.8. Example buffers for inclusion in formulations herein include histidine-based buffers, for example, histidine, histidine hydrochloride, and histidine acetate. Buffers for inclusion in formulations herein can alternatively be phosphate-based buffers, for example, sodium phosphate, acetate-based buffers, for example, sodium acetate or acetic acid, or can be citrate-based, for example, sodium citrate or citric acid. It is also recognized that buffers can be a mix of the above, as long as the buffer functions to buffer the formulations in the above-described pH ranges. In some cases, the buffer is from about 5 mM to about 25 mM, or more typically, about 5 mM to about 15 mM. Buffers can be about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, or about 25 mM.

In an embodiment of the invention, a histidine-based buffer is prepared using histidine and histidine monohydrochloride.

Surfactant for use herein refers to ingredients that protect the higher concentration of VEGF antagonist, *e.g.*,

VEGF receptor fusion protein (e.g., aflibercept), from various surface and interfacial induced stresses. As such, surfactants can be used to limit or minimize VEGF receptor fusion protein aggregation and promote protein solubility. Suitable surfactants herein have been shown to be non-ionic, and can include surfactants that have a polyoxyethylene moiety. Illustrative surfactants in this category include: polysorbate 20, polysorbate 80, poloxamer 188, polyethylene glycol 3350, and mixtures thereof. Surfactants in the formulations can be present at from about 0.02% to about 0.1% weight per volume (w/v), and more typically, about 0.02% to about 0.04% (w/v). In some cases, the surfactant is about 0.02% (w/v), about 0.03% (w/v), about 0.04% (w/v), about 0.05% (w/v), about 0.06% (w/v), about 0.07% (w/v), about 0.08% (w/v), about 0.09% (w/v), or about 0.1% (w/v).

Thermal stabilizers for use in pharmaceutical formulations that may be used in methods set forth herein refers to ingredients that provide thermal stability against thermal denaturation of the VEGF antagonist, *e.g.*, VEGF receptor fusion protein (e.g., aflibercept), as well as protect against loss of VEGF receptor fusion protein potency or activity. Suitable thermal stabilizers include sugars, and can be sucrose, trehalose, sorbitol or mannitol, or can be amino acids, for example L-proline, L-arginine (*e.g.*, L-arginine monohydrochloride), or taurine. Additionally, thermal stabilizers may also include substituted acrylamides or propane sulfonic acid, or may be compounds like glycerol.

In some cases, the pharmaceutical formulations for use in a method herein include both a sugar and taurine, a sugar and an amino acid, a sugar and propane sulfonic acid, a sugar and taurine, glycerol and taurine, glycerol and propane sulfonic acid, an amino acid and taurine, or an amino acid and propane sulfonic acid. In addition, formulations can include a sugar, taurine and propane sulfonic acid, glycerol, taurine and propane sulfonic acid, as well as L-proline, taurine and propane sulfonic acid.

Embodiments herein may have thermal stabilizers present alone, each independently present at a concentration of, or present in combination at a total concentration of, from about 2% (w/v) to about 10% (w/v) or 4% (w/v) to about 10% (w/v), or about 4% (w/v) to about 9% (w/v), or about 5% (w/v) to about 8% (w/v). Thermal stabilizers in the formulation can be at a concentration of about 2% (w/v), about 2.5% (w/v), about 3% (w/v), about 4% (w/v), about 5% (w/v), about 6% (w/v), about 7% (w/v), about 8% (w/v), about 9% (w/v), about 10% (w/v) or about 20% (w/v).

With respect to taurine and propane sulfonic acid, in an embodiment of the invention, these thermal stabilizers can be present in the formulations at about from 25 mM to about 100 mM, and more typically from about 50 mM to about 75 mM (as compared to the other thermal stabilizers).

Viscosity reducing agents typically are used to reduce or prevent protein aggregation. Viscosity reducing agents for inclusion herein include: sodium chloride, magnesium chloride, D- or L-arginine (*e.g.*, L-arginine monohydrochloride), lysine, or mixtures thereof. When present herein, viscosity reducing agents can be present at from about 10 mM to about 100 mM, and more typically from about 30 mM to about 75 mM, and even more typically from about 40 mM to about 70 mM. In some cases, the viscosity reducing agent is present at about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM or about 100 mM.

Pharmaceutical formulations for use in a method as set forth herein can also have a pharmaceutically acceptable viscosity for ocular administration, for example, intravitreal injection. Viscosity generally refers to the measure of resistance of a fluid which is being deformed by either shear stress or tensile stress (typically measured by techniques known in the art, viscometer or rheometer, for example). Typical viscosities of formulations for use in a method set forth herein are from about 5.0 cP (centipoise) to about 15 cP, from about 11 cP to about 14 cP, from about 12 cP to about 15 cP or from about 11 cP to about 12 cP. As such, formulation viscosity herein can be about 5.0 cP, about 6.0, about 7.1 cP, about 7.2 cP, about 7.3 cP, about 7.4 cP, about 7.5 cP, about 7.6 cP, about 10 cP, about 10.5 cP, about 11.0 cP, about 11.5 cP, about 12.0 cP, about 12.5 cP, about 13.0 cP, about 13.5 cP, about 14.0 cP, about 14.5 cP, or about 15.0 cP (*e.g.*, when measured at 20°C).

Various embodiments herein do not require inclusion of an inorganic salt, or other viscosity reducing agent, to maintain these highly useful viscosities. Typically, high concentration protein solutions require viscosity reducing agents to avoid protein aggregation and higher viscosity, making the formulations difficult for intravitreal injection and reducing the potency of the VEGF receptor fusion protein. As such, embodiments herein include methods of using formulations that have had substantially no, or no added, sodium chloride (NaCl), magnesium chloride .($MgCl_2$), D- or L-arginine hydrochloride, lysine or other viscosity reducing agent.

Osmolality is a critical attribute for injectable pharmaceutical formulations for use in a method of the present invention. It is desirable to have products match physiological osmotic conditions. Furthermore, osmolality provides confirmation of soluble content in solution. In an embodiment of the invention, the osmolality of a formulation for use in a method of the present invention is less than or equal to about 506 mmol/Kg or from about 250 to about 506 mmol/Kg., *e.g.*, about 250, 260, 270, 280, 290, 299, 300, 310, 314, 315, 316, 324, 343, 346, 349, 369, 384, 403, 426, 430 or 506 mmol/Kg. In an embodiment of the invention, the osmolality is lower than about 250 mmol/Kg.

Illustrative pharmaceutical formulations for use in the methods of the present invention include the following:

Formulation A: 80 mg/ml aflibercept, 10 mM histidine-based buffer, 5 % (w/v) sucrose, 0.03% (w/v) polysorbate 20, and 40 mM sodium chloride, with a pH of 5.8 to 6.2.

Formulation B: 80 mg/ml aflibercept, 10 mM phosphate-based buffer, 5 % (w/v) sucrose, 0.03% (w/v) polysorbate 20, and 40 mM sodium chloride, with a pH of 5.8 to 6.2.

Formulation C: 80 mg/ml aflibercept, 10 mM citrate-based buffer, 5 % (w/v) sucrose, 0.03% (w/v) polysorbate 20, and 40 mM sodium chloride, with a pH of 5.8 to 6.2.

Formulation D: 80 mg/ml aflibercept, 10 mM histidine-based buffer, 5 % (w/v) sucrose, 0.03 % (w/v) polysorbate 80, and 40 mM sodium chloride, with a pH of 6.2.

Formulation E: 80 mg/ml aflibercept, 10 mM phosphate-based buffer, 5 % (w/v) sucrose, 0.03 % (w/v) polysorbate 80, and 40 mM sodium chloride, with a pH of 5.8 to 6.2.

Formulation F: 80 mg/ml aflibercept, 10 mM citrate-based buffer, 5 % (w/v) sucrose, 0.03 % (w/v) polysorbate 80, and 40 mM sodium chloride, with a pH of 5.8 to 6.2.

Formulation G: 80 mg/ml aflibercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.

Formulation H: 80 mg/ml aflibercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.

Formulation I: 80 mg/ml aflibercept, 10 mM citrate-based buffer, 8 % (w/v) sucrose, and 0.0 3% (w/v) polysorbate 20, with a pH of 5.8 to 6.2, and, optionally, specifically excluding a viscosity reducing agent.

Formulation J: 80 mg/ml aflibercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.

Formulation K: 80 mg/ml aflibercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.

Formulation L: 80 mg/ml aflibercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.

Formulation M: 150 mg/ml aflibercept, 10 mM histidine-based buffer, 5 % (w/v) sucrose, 0.03 % (w/v) polysorbate 20, and 40 mM sodium chloride, with a pH of 5.8 to 6.2.

Formulation N: 150 mg/ml aflibercept, 10 mM phosphate-based buffer, 5 % (w/v) sucrose, 0.03 % (w/v) polysorbate 20, and 40 mM sodium chloride, with a pH of 5.8 to 6.2.

Formulation O: 150 mg/ml aflibercept, 10 mM citrate-based buffer, 5 % (w/v) sucrose, 0.03 % (w/v) polysorbate 20, and 40 mM sodium chloride, with a pH of 5.8 to 6.2.

Formulation P: 150 mg/ml aflibercept, 10 mM histidine-based buffer, 5 % (w/v) sucrose, 0.03 % (w/v) polysorbate 80, and 40 mM sodium chloride, with a pH of 6.2.

Formulation Q: 150 mg/ml aflibercept, 10 mM phosphate-based buffer, 5 % (w/v) sucrose, 0.03 % (w/v) polysorbate 80, and 40 mM sodium chloride, with a pH of 5.8 to 6.2.

Formulation R: 150 mg/ml aflibercept, 10 mM citrate-based buffer, 5 % (w/v) sucrose, 0.03 % (w/v) polysorbate 80, and 40 mM sodium chloride, with a pH of 5.8 to 6.2.

Formulation S: 150 mg/ml aflibercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.

Formulation T: 150 mg/ml aflibercept, 10 mM phosphate-based buffer, 8 % (w/v) sucrose, and 0.03 % (w/v) polysorbate 20, with a pH of 5.8 to 6.2 (e.g., 6.2), and, optionally, specifically excluding a viscosity reducing agent.

Formulation U: 150 mg/ml aflibercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.

Formulation V: 150 mg/ml aflibercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.

Formulation W: 150 mg/ml aflibercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.

Formulation X: 150 mg/ml aflibercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.

Formulation Y: 80 mg/ml conbercept, 10 mM histidine-based buffer, 5 % (w/v) sucrose, 0.03 % (w/v) polysorbate 20, and 40 mM sodium chloride, with a pH of 5.8 to 6.2.

Formulation Z: 80 mg/ml conbercept, 10 mM phosphate-based buffer, 5 % (w/v) sucrose, 0.03 % (w/v) polysorbate 20, and 40 mM sodium chloride, with a pH of 5.8 to 6.2.

Formulation AA: 80 mg/ml conbercept, 10 mM citrate-based buffer, 5 % (w/v) sucrose, 0.03 % (w/v) polysorbate 20, and 40 mM sodium chloride, with a pH of 5.8 to 6.2.

Formulation BB: 80 mg/ml conbercept, 10 mM histidine-based buffer, 5 % (w/v) sucrose, 0.03 % (w/v) polysorbate 80, and 40 mM sodium chloride, with a pH of 6.2.

Formulation CC: 80 mg/ml conbercept, 10 mM phosphate-based buffer, 5 % (w/v) sucrose, 0.03 % (w/v) polysorbate 80, and 40 mM sodium chloride, with a pH of 5.8 to 6.2.

Formulation DD: 80 mg/ml conbercept, 10 mM citrate-based buffer, 5 % (w/v) sucrose, 0.03 % (w/v) polysorbate 80, and 40 mM sodium chloride, with a pH of 5.8 to 6.2.

Formulation EE: 80 mg/ml conbercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.

Formulation FF: 80 mg/ml conbercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.

Formulation GG: 80 mg/ml conbercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.

Formulation HH: 80 mg/ml conbercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.

Formulation II: 80 mg/ml conbercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.

Formulation JJ: 80 mg/ml conbercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.

Formulation KK: 150 mg/ml conbercept, 10 mM histidine-based buffer, 5 % (w/v) sucrose, 0.03 % (w/v) polysorbate 20, and 40 mM sodium chloride, with a pH of 5.8 to 6.2.

Formulation LL: 150 mg/ml conbercept, 10 mM phosphate-based buffer, 5 % (w/v) sucrose, 0.03 % (w/v) polysorbate 20, and 40 mM sodium chloride, with a pH of 5.8 to 6.2.

Formulation MM: 150 mg/ml conbercept, 10 mM citrate-based buffer, 5 % (w/v) sucrose, 0.03 % (w/v) polysorbate 20, and 40 mM sodium chloride, with a pH of 5.8 to 6.2.

Formulation NN: 150 mg/ml conbercept, 10 mM histidine-based buffer, 5 % (w/v) sucrose, 0.03 % (w/v) polysorbate 80, and 40 mM sodium chloride, with a pH of 6.2.

Formulation OO: 150 mg/ml conbercept, 10 mM phosphate-based buffer, 5 % (w/v) sucrose, 0.03 % (w/v) polysorbate 80, and 40 mM sodium chloride, with a pH of 5.8 to 6.2.

Formulation PP: 150 mg/ml conbercept, 10 mM citrate-based buffer, 5 % (w/v) sucrose, 0.03 % (w/v) polysorbate 80, and 40 mM sodium chloride, with a pH of 5.8 to 6.2.

Formulation QQ: 150 mg/ml conbercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.

Formulation RR: 150 mg/ml conbercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.

Formulation SS: 150 mg/ml conbercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.

Formulation TT: 150 mg/ml conbercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.

Formulation UU: 150 mg/ml conbercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.

Formulation VV: 150 mg/ml conbercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.

Formulation WW: 140 mg/ml VEGF receptor fusion protein (e.g., aflibercept), 10 mM histidine-based buffer, 5 % (w/v) sucrose, 0.03 % (w/v) polysorbate 20, and 50 mM taurine, with a pH of 5.8.

Formulation XX: 140 mg/ml VEGF receptor fusion protein (e.g., aflibercept), 20 mM histidine-based buffer, 4 % (w/v) proline, 0.03 % (w/v) polysorbate 20, and 50 mM arginine hydrochloride, with a pH of 5.8.

Formulation YY: 140 mg/ml VEGF receptor fusion protein (e.g., aflibercept), 20 mM histidine-based buffer, 2.5 % (w/v) sucrose, 2.0 % (w/v) proline, 0.03 % (w/v) polysorbate 20, and 50 mM taurine, with a pH of 5.8.

Formulation ZZ: 140 mg/ml VEGF receptor fusion protein (e.g., aflibercept), 10 mM histidine-based buffer, 2.5 % (w/v) sucrose, 2.0 % (w/v) proline, 0.03 % (w/v) polysorbate 20, and 50 mM arginine hydrochloride, with a pH of 5.8.

Formulation AAA: 140 mg/ml VEGF receptor fusion protein (e.g., aflibercept), 20 mM histidine-based buffer, 5 % (w/v) sucrose, 0.03% (w/v) polysorbate 20, and 50 mM PSA, with a pH of 5.8.

Formulation BBB: 140 mg/ml VEGF receptor fusion protein (e.g., aflibercept), 20 mM histidine-based buffer, 2.5 % (w/v) sucrose, 2.0 % (w/v) proline, 0.03 % (w/v) polysorbate 20, and 50 mM PSA, with a pH of 5.8.

Formulation CCC: 80, 100, 120 or 140 mg/ml VEGF receptor fusion protein (e.g., aflibercept), 20 mM histidine-based buffer, 5 % (w/v) sucrose, 0.03 % (w/v) polysorbate 20, and 50 mM arginine hydrochloride, with a pH of 5.8.

Formulation DDD: 140 mg/ml VEGF receptor fusion protein (e.g., aflibercept), 10 mM histidine-based buffer, 4 % (w/v) proline, 0.03 % (w/v) polysorbate 20, and 50 mM PSA, with a pH of 5.8.

Formulation EEE: 140 mg/ml VEGF receptor fusion protein (e.g., aflibercept), 20 mM histidine-based buffer, 5 % (w/v) sucrose, and 0.03 % (w/v) polysorbate 20 and, optionally, no thermal stabilizer, with a pH of 5.8.

Formulation FFF: 140 mg/ml VEGF receptor fusion protein (e.g., aflibercept), 10mM sodium phosphate, 5 % (w/v) sucrose and 0.03 % polysorbate 20 with a pH of 6.2.

Formulation GGG: 140 mg/ml VEGF receptor fusion protein (e.g., aflibercept); 20 mM histidine, pH 5.8; 5% sucrose;

0.03 % polysorbate 20; 50 mM sodium sulfate

Formulation HHH: 140 mg/ml VEGF receptor fusion protein (*e.g.*, aflibercept); 20 mM histidine, pH 5.8; 5% sucrose; 0.03 % polysorbate 20; 50 mM sodium thiocyanate

Formulation III: 140 mg/ml VEGF receptor fusion protein (*e.g.*, aflibercept); 20 mM histidine, pH 5.8; 5 % sucrose, 0.03 % polysorbate 20; 40 mM sodium citrate

Formulation JJJ: 140 mg/ml VEGF receptor fusion protein (*e.g.*, aflibercept); 20 mM histidine, pH 5.8; 5% Sucrose, 0.03 % polysorbate 20; 50 mM glycine

Formulation KKK: 140 mg/ml VEGF receptor fusion protein (*e.g.*, aflibercept); 20 mM histidine, pH 5.8; 5 % sucrose, 0.03 % polysorbate 20; 50 mM sodium chloride

Formulation LLL: 140 mg/ml VEGF receptor fusion protein (*e.g.*, aflibercept); 20 mM histidine, pH 5.8; 5 % sucrose; 0.03 % polysorbate 20; 50 mM lysine

Formulation MMM: 140 mg/ml VEGF receptor fusion protein (*e.g.*, aflibercept); 20 mM histidine, pH 5.8; 5 % sucrose; 0.03% polysorbate 20; 50 mM sodium aspartate

Formulation NNN: 140 mg/ml VEGF receptor fusion protein (*e.g.*, aflibercept); 20 mM histidine, pH 5.8; 5 % sucrose; 0.03 % polysorbate 20; 50 mM sodium glutamate

Formulation OOO: 140 mg/ml VEGF receptor fusion protein (*e.g.*, aflibercept); 20 mM histidine, pH 5.8; 5 % sucrose; 0.03% polysorbate 20; 50 mM sodium citrate; 50 mM arginine hydrochloride

Formulation PPP: 140 mg/ml VEGF receptor fusion protein (*e.g.*, aflibercept); 20 mM histidine, pH 5.8; 5 % sucrose; 0.03% polysorbate 20; 50 mM glycine; 50 mM arginine hydrochloride

Formulation QQQ: 140 mg/ml VEGF receptor fusion protein (*e.g.*, aflibercept); 20 mM histidine, pH 5.8; 5 % sucrose; 0.03% polysorbate 20; 50 mM sodium aspartate; 50 mM arginine hydrochloride

Formulation RRR: 140 mg/ml VEGF receptor fusion protein (*e.g.*, aflibercept); 20 mM histidine, pH 5.8; 5 % sucrose; 0.03% polysorbate 20; 50 mM sodium glutamate; 50 mM arginine hydrochloride

Formulation SSS: 140 mg/ml VEGF receptor fusion protein (*e.g.*, aflibercept); 20 mM His, pH 5.8; 5 % sucrose; 0.03 % polysorbate 20; 10 mM L-arginine hydrochloride

Formulation TTT: 140 mg/ml VEGF receptor fusion protein (*e.g.*, aflibercept); 20 mM His, pH 5.8; 5 % sucrose; 0.03 % polysorbate 20; 100 mM L-arginine hydrochloride

Formulation UUU: 30 mg/ml VEGF receptor fusion protein (*e.g.*, aflibercept), 10 % sucrose, 10 mM phosphate, 0.03 % polysorbate 20, pH 6.2

Formulation VVV: 30 mg/ml VEGF receptor fusion protein (*e.g.*, aflibercept), 20 % sucrose, 10 mM phosphate, 0.03 % polysorbate 20, pH 6.2

Formulation WWW: 60 mg/ml VEGF receptor fusion protein (*e.g.*, aflibercept), 10 % sucrose, 10 mM phosphate, 0.03 % polysorbate 20, pH 6.2

Formulation XXX: 60 mg/ml VEGF receptor fusion protein (*e.g.*, aflibercept), 20 % sucrose, 10 mM phosphate, 0.03 % polysorbate 20, pH 6.2

Formulation YYY: 120 mg/ml VEGF receptor fusion protein (*e.g.*, aflibercept), 10 % sucrose, 10 mM phosphate, 0.03 % polysorbate 20, pH 6.2

Formulation ZZZ: 120 mg/ml VEGF receptor fusion protein (*e.g.*, aflibercept), 20 % sucrose, 10 mM phosphate, 0.03 % polysorbate 20, pH 6.2

Formulation AAAA: 120 mg/ml VEGF receptor fusion protein (*e.g.*, aflibercept), 10 % sucrose, 10 mM phosphate, 0.03 % polysorbate 20, 50 mM NaCl, pH 6.2

Formulation BBBB: 120 mg/ml VEGF receptor fusion protein (*e.g.*, aflibercept), 20 % sucrose, 10 mM phosphate, 0.03 % polysorbate 20, 50 mM NaCl, pH 6.2

Formulation CCCC: 140 mg/ml VEGF receptor fusion protein (*e.g.*, aflibercept), 10 mM sodium phosphate, 5 % sucrose, 40 mM sodium chloride, 0.03 % PS20, pH 6.2

Formulation DDDD: 80 mg/ml VEGF receptor fusion protein (*e.g.*, aflibercept), 20 mM histidine-based buffer, 5 % (w/v) sucrose, 0.03 % (w/v) polysorbate 20, and 50 mM L-arginine monohydrochloride, with a pH of 5.8.

Formulation EEEE: 120.0 mg/ml VEGF receptor fusion protein (*e.g.*, aflibercept) (*e.g.*, ± 12 mg/ml), 20 mM histidine-based buffer (*e.g.*, ± 2 mM), 5 % (w/v) sucrose (*e.g.*, ± 0.5%), 0.03 % (w/v) polysorbate 20 (*e.g.,* 0.02-0.04%), and 50 mM L-arginine monohydrochloride (*e.g.*, ± 5 mM), with a pH of 5.8 (*e.g.*, 5.6-6.0 or 5.5-6.1).

Formulation FFFF: 113.3 mg/ml VEGF receptor fusion protein (*e.g.*, aflibercept) (*e.g.*, 102-125 mg/ml), 20 mM histidine-based buffer (*e.g.*, ± 2 mM), 5 % (w/v) sucrose (*e.g.*, ± 0.5%), 0.03 % (w/v) polysorbate 20 (*e.g.,* 0.02-0.04%), and 50 mM L-arginine monohydrochloride (*e.g.,* ± 5 mM), with a pH of 5.8 (*e.g.*, 5.6-6.0 or 5.5-6.1).

Formulation GGGG: 114.3 mg/ml VEGF receptor fusion protein (*e.g.*, aflibercept) (*e.g.*, 103-126 mg/ml), 10 mM histidine-based buffer (*e.g.*, ± 1 mM), 5 % (w/v) sucrose (*e.g.*, ± 0.5%), 0.03 % (w/v) polysorbate 20 (*e.g.,* 0.02-0.04%), and 50 mM L-arginine (*e.g.*, L-arginine monohydrochloride) (*e.g.*, ± 5 mM), with a pH of 5.8 (*e.g.*, 5.6-6.0 or 5.5-6.1).

Formulation HHHH: 100.0 mg/ml VEGF receptor fusion protein (*e.g.*, aflibercept) (*e.g.*, ± 10 mg/ml), 20 mM histidine-based buffer (*e.g.*, ± 2 mM), 5 % (w/v) sucrose (*e.g.*, ± 0.5%), 0.03 % (w/v) polysorbate 20 (*e.g.*, 0.02-0.04%), and 50 mM L-arginine monohydrochloride (*e.g.*, ± 5 mM), with a pH of 5.8 (*e.g.*, 5.6-6.0 or 5.5-6.1).

Formulation IIII: 133.3 mg/ml VEGF receptor fusion protein (*e.g.*, aflibercept) (*e.g.*, ± 13 mg/ml), 20 mM histidine-based buffer (*e.g.*, ± 2 mM), 5 % (w/v) sucrose (*e.g.*, ± 0.5%), 0.03 % (w/v) polysorbate 20 (*e.g.*, 0.02-0.04%), and 50 mM L-arginine monohydrochloride (*e.g.*, ± 5 mM), with a pH of 5.8 (*e.g.*, 5.6-6.0 or 5.5-6.1).

Formulation JJJJ: 150 mg/ml aflibercept (*e.g.*, aflibercept) (*e.g.*, ± 15 mg/ml), 10 mM sodium phosphate, 8% (w/v) sucrose (*e.g.*, ± 0.8%), 0.03% (w/v) polysorbate 20 (*e.g.*, 0.02-0.04%) and 50 mM L-arginine hydrochloride, pH 6.2 (*e.g.*, 6.0-6.4 or 5.9-6.5).

Formulation KKKK: 114.3 mg/ml VEGF receptor fusion protein (*e.g.*, aflibercept) (*e.g.*, ± 14 mg/ml), 20 mM histidine-based buffer (*e.g.*, ± 2 mM), 5% (w/v) sucrose (*e.g.*, ± 0.5%), 0.03% (w/v) polysorbate 20 (*e.g.*, 0.02-0.04%), and 50 mM L-arginine monohydrochloride (*e.g.,* ± 5 mM), with a pH of 5.8 (*e.g.*, 5.6-6.0 or 5.5-6.1).

In an embodiment of the invention the formulation that can be administered, *e.g.*, intravitreally, to a subject is an aqueous pharmaceutical formulation comprising:

at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component (*e.g.*, aflibercept);
about L-arginine (*e.g.*, at a concentration of about 10-100 mM);
sucrose;
a histidine-based buffer; and
a surfactant;
wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.

See International Patent Application Publication No. WO2019/217927 and US Patent No. 11103552.

## Treatment and Administration

The present invention provides methods for treating angiogenic eye disorders by sequentially administering initial loading doses of greater than or equal to (≥) about 8 mg (*e.g.*, about every 2-4 or 3-5 weeks) of VEGF antagonist (*e.g.*, aflibercept) followed by additional doses of ≥ about 8 mg every 12 weeks of VEGF antagonist (*e.g.*, aflibercept). For example, in an embodiment of the invention, the methods of the present invention include treating or preventing angiogenic eye disorders, such as diabetic retinopathy, diabetic macular edema or neovascular AMD, by administering, sequentially, one or more (*e.g.*, 3, 4 or 5) doses (*e.g.*, ≥ about 8 mg) about every month (or about every 28 days, 28 ± 5 days or about every 4 weeks), followed by one or more doses (*e.g.*, ≥ about 8 mg) about every 12 weeks. For example, in an embodiment of the invention, about 8 mg is 7.2 mg, 8.8 mg or 7.2-8.8 mg or 8 mg ± about 10%.

The terms "initial dose," "secondary doses," and "tertiary doses," refer to the temporal sequence of administration of the VEGF antagonist (e.g., aflibercept). Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial dose occurs on day 1 for the purposes of counting or numbering days thereafter (see *e.g.*, Tables 1-1 and 1-2 herein). The initial, secondary, and tertiary doses may all contain the same amount of VEGF antagonist, but will generally differ from one another in terms of frequency of administration. In certain embodiments, however, the amount of VEGF antagonist contained in the initial, secondary and/or tertiary doses will vary from one another (*e.g.*, adjusted up or down as appropriate) during the course of treatment. Thus, a dosing regimen of the present invention may be expressed as follows:

a single initial dose (*e.g.*, ≥ about 8 mg) of a VEGF antagonist (*e.g.*, aflibercept), followed by one or more (*e.g.*, 2, or 3 or 4) secondary doses of the VEGF antagonist, followed by one or more tertiary doses of the VEGF antagonist;
wherein each secondary dose is administered 2 to 4 weeks after the immediately preceding dose; and
wherein each tertiary dose is administered about 12 weeks after the immediately preceding dose. The initial and secondary doses administered before the tertiary doses may be referred to, generally, as "loading" doses. The tertiary doses may be referred to as "maintenance" doses.

The present invention includes methods wherein one or more additional, non-scheduled, *pro re nata* (PRN) doses, in addition to any of the scheduled initial, secondary and/or tertiary doses of VEGF antagonist (*e.g.*, aflibercept) are administered to a subject. Such PRN doses are typically administered at the discretion of the treating physician depending on the particular needs of the subject.

In an embodiment of the invention,

- the method comprises administering the secondary doses to a subject who has received the initial dose
- the method comprises administering the remaining secondary doses to a subject who has already received one or more secondary doses;
- the method comprises administering one or more tertiary doses to a subject who has already received the secondary doses
- the method comprises administering one or more tertiary doses to a subject who has already received one or more tertiary doses;

optionally wherein the subject receives doses, earlier in the regimen, in one pharmaceutical formulation and additional doses, later in the regimen, in a different pharmaceutical formulation, for example, comprising a different buffer (*e.g.*, wherein one or more of the secondary doses are in one pharmaceutical formulation and the tertiary doses are in a different pharmaceutical formulation).

Dosing every "month" refers to dosing about every 28 days, about every 4 weeks, or about every 28 $\pm$ 5 days and may encompass up to every 5 weeks. Dosing every "4 weeks" refers to dosing about every 28 days, about every month or about every 28 $\pm$ 5 days, and may encompass up to every 5 weeks.

Dosing every "2-4 weeks" refers to dosing about every 2 weeks, 3 weeks or 4 weeks. Dosing every "8 weeks" refers to dosing about every 2 months, about every 56 days, 56 $\pm$ 5 days.

Dosing every "12 weeks" refers to dosing about every 3 months, about every quarter year, about every 84, 90 days, 84 $\pm$ 5 days, or 90 $\pm$ 5 days.

A dose of greater than or equal to about 8 mg or $\geq$ about 8 mg VEGF antagonist (e.g., aflibercept) includes 7.2 mg; 7.2-8.8 mg; 8.0 mg; 8.01 mg; 8.1 mg; 8.2 mg; 8.3 mg; 8.4 mg; 8.5 mg; 8.6 mg; 8.7 mg; 8.8 mg; 8.9 mg; 9 mg; 9.1 mg; 9.2 mg; 9.3 mg; 9.4 mg; 9.5 mg; 9.6 mg; 9.7 mg; 9.8 mg; 9.9 mg, 10.0 mg, 10.1 mg; 10.2 mg; 10.3 mg; 10.4 mg; 10.5 mg; 10.6 mg; 10.7 mg; 10.8 mg; 10.9 mg; 11 mg; 11.1 mg; 11.2 mg; 11.3 mg; 11.4 mg; 11.5 mg; 11.6 mg; 11.7 mg; 11.8 mg; 11.9 mg; 12 mg; 12.1 mg; 12.2 mg; 12.3 mg; 12.4 mg; 12.5 mg; 12.6 mg; 12.7 mg; 12.8 mg; 12.9 mg; 13 mg; 13.1 mg; 13.2 mg; 13.3 mg; 13.4 mg; 13.5 mg; 13.6 mg; 13.7 mg; 13.8 mg; 13.9 mg; 14 mg; 14.1 mg; 14.2 mg; 14.3 mg; 14.4 mg; 14.5 mg; 14.6 mg; 14.7 mg; 14.8 mg; 14.9 mg; 15 mg; 15.1 mg; 15.2 mg; 15.3 mg; 15.4 mg; 15.5 mg; 15.6 mg; 15.7 mg; 15.8 mg; 15.9 mg; 16 mg; 16.1 mg; 16.2 mg; 16.3 mg; 16.4 mg; 16.5 mg; 16.6 mg; 16.7 mg; 16.8 mg; 16.9 mg; 17 mg; 17.1 mg; 17.2 mg; 17.3 mg; 17.4 mg; 17.5 mg; 17.6 mg; 17.7 mg; 17.8 mg; 17.9 mg; 18 mg; 18.1 mg; 18.2 mg; 18.3 mg; 18.4 mg; 18.5 mg; 18.6 mg; 18.7 mg; 18.8 mg; 18.9 mg; 19 mg; 19.1 mg; 19.2 mg; 19.3 mg; 19.4 mg; 19.5 mg; 19.6 mg; 19.7 mg; 19.8 mg; 19.9 mg; or 20 mg ($\pm$ about 10%, $\pm$ about 0.5, or $\pm$ about 0.51 mg of any of the foregoing). In an embodiment of the invention, a dosage of $\geq$ 8 mg VEGF antagonist is administered in a dose having a volume of about 100 $\mu$l or less, about 75 $\mu$l or less or about 70 $\mu$l or less, *e.g.*, about 50 $\mu$l; 51 $\mu$l; 52 $\mu$l; 53 $\mu$l; 54 $\mu$l; 55 $\mu$l; 56 $\mu$l; 57 $\mu$l; 58 $\mu$l; 59 $\mu$l; 60 $\mu$l; 61 $\mu$l; 62 $\mu$l; 63 $\mu$l; 64 $\mu$l; 65 $\mu$l; 66 $\mu$l; 67 $\mu$l; 68 $\mu$l; 69 $\mu$l; 70 $\mu$l; 71 $\mu$l; 72 $\mu$l; 73 $\mu$l; 74 $\mu$l; 75 $\mu$l; 76 $\mu$l; 77 $\mu$l; 78 $\mu$l; 79 $\mu$l; 80 $\mu$l; 81 $\mu$l; 82 $\mu$l; 83 $\mu$l; 84 $\mu$l; 85 $\mu$l; 85-87 $\mu$l; 86 $\mu$l; 87 $\mu$l; 88 $\mu$l; 89 $\mu$l; 90 $\mu$l; 91 $\mu$l; 92 $\mu$l; 93 $\mu$l; 94 $\mu$l; 95 $\mu$l; 96 $\mu$l; 97 $\mu$l; 98 $\mu$l; 99 $\mu$l; or 100 $\mu$l ($\pm$ about 4, 4.45, 4.5, or 5 microliters).

Any dosing frequency specified herein may, in an embodiment of the invention, be expressed as the specific frequency "$\pm$ 5 days" (*e.g.*, where "4 weeks" is stated, the present invention also includes embodiments such as 4 weeks $\pm$ 5 days).

"Sequentially administering" means that each dose of VEGF antagonist (e.g., aflibercept) is administered to the eye of a patient at a different point in time, *e.g.*, on different days separated by a predetermined interval (*e.g.*, hours, days, weeks or months). The present invention includes methods which comprise sequentially administering, to the eye of a patient, a single initial dose of a VEGF antagonist, followed by one or more secondary doses of the VEGF antagonist, followed by one or more tertiary doses of the VEGF antagonist.

An effective or therapeutically effective dose of VEGF antagonist, e.g., aflibercept, for treating or preventing an angiogenic eye disorder refers to the amount of VEGF antagonist sufficient to alleviate one or more signs and/or symptoms of the disease or condition in the treated subject, whether by inducing the regression or elimination of such signs and/or symptoms or by inhibiting the progression of such signs and/or symptoms. In an embodiment of the invention, an effective or therapeutically effective dose of VEGF antagonist is $\geq$ about 8 mg every month followed by once every 12 weeks.

An "angiogenic eye disorder" means any disease of the eye which is caused by or associated with the growth or proliferation of blood vessels or by blood vessel leakage. Non-limiting examples of angiogenic eye disorders that are treatable or preventable using the methods of the present invention include:

- age-related macular degeneration (neovascular (nAMD)),
- macular edema (ME),

- macular edema following retinal vein occlusion (ME-RVO),
- retinal vein occlusion (RVO),
- central retinal vein occlusion (CRVO),
- branch retinal vein occlusion (BRVO),
- diabetic macular edema (DME),
- choroidal neovascularization (CNV),
- iris neovascularization,
- neovascular glaucoma,
- post-surgical fibrosis in glaucoma,
- proliferative vitreoretinopathy (PVR),
- optic disc neovascularization,
- corneal neovascularization,
- retinal neovascularization,
- vitreal neovascularization,
- pannus,
- pterygium,
- vascular retinopathy,
- diabetic retinopathies (DR) (*e.g.*, non-proliferative diabetic retinopathy (*e.g.*, characterized by a Diabetic Retinopathy Severity Scale (DRSS) level of about 47 or 53) or proliferative diabetic retinopathy; *e.g.*, in a subject that does not suffer from DME), and
- diabetic retinopathy in a patient who has diabetic macular edema (DME).

In an embodiment of the invention, a subject receiving a treatment for an angiogenic eye disorder as set forth herein (*e.g.*, three monthly doses of about 8 mg aflibercept followed by doses of about 8 mg aflibercept every 12 weeks) achieves one or more of the following:

- with respect to visual acuity (VA) or best corrected visual acuity (BCVA), achieving:

  ◦ No loss in visual acuity or BCVA or a gain in visual acuity or BCVA;
  ◦ no loss of visual acuity or BCVA, for example, by about week 4, 8, 9, 12, 16, 20, 24, 28, 32, 36, 40 or 44, following the initial dose *e.g.*, according to ETDRS (Early Treatment Diabetic Retinopathy Study) chart or Snellen equivalent (*e.g.*, no loss of 5, 10, or 15 or more ETDRS letters (*e.g.*, no loss of 5 (or more), 6 (or more), 7 (or more), 8 (or more), 9 (or more), 10 (or more), 11 (or more), 12 (or more), 13 (or more), 14 (or more) or 15 (or more) letters) or Snellen equivalent),
  ◦ a gain in visual acuity or BCVA, for example, by about week 4, 8, 9, 12, 16, 20, 24, 28, 32, 36, 40 or 44 following the initial dose, *e.g.*, according to ETDRS chart or Snellen equivalent (e.g., gaining 5 or more, 10 or more or 15 or more ETDRS letters (*e.g.*, a gain of 5 (or more), 6 (or more), 7 (or more), 8 (or more), 9 (or more), 10 (or more), 11 (or more), 12 (or more), 13 (or more), 14 (or more) or 15 (or more) letters)); and/or
  ◦ a gain in BCVA of about 6 or 7 or 8 letters (or more) (*e.g.*, according to ETDRS chart or Snellen equivalent) by week 8 and maintaining a gain of about 6 or 7 or 8 letters until at least about week 44;

- with respect to central retinal thickness (CRT), achieving:

  o a decrease in central retinal thickness;
  o a decrease in central retinal thickness by at least about 123, 125, 131, 142, 147, 149, 150, 151, 156, 157, 158, 159, 161, 162, 166, 167, 168, 172, 173, 175, 177, 178 or 183 micrometers (or more), for example, by about week 4, 8, 9, 12, 16, 20, 24, 28, 32, 36, 40 or 44 following the initial dose, for example, wherein the baseline (prior to treatment) CRT, is about 488, 492, 497 or 516 micrometers;
  ◦ a decrease in CRT of about 47 micrometers (or more) from about week 12 to about week 20 following the initial dose, *e.g.*, wherein the baseline CRT is about 516 micrometers;
  ◦ a decrease in CRT of about 17 micrometers (or more) from about week 24 to about week 32 following the initial dose, *e.g.*, wherein the baseline CRT is about 516 micrometers;
  ◦ a decrease in CRT of about 18 micrometers (or more) from about week 36 to about week 44 following the initial dose, *e.g.*, wherein the baseline CRT is about 516 micrometers;
  ◦ a decrease in central retinal thickness by at least about 4 or 24.3 micrometers (or more) from about week 12 to about week 16, for example, wherein the baseline CRT, prior to treatment, is about 516 micrometers;
  ◦ a decrease in CRT of about 123, 131 161 micrometers (or more) (*e.g.*, by about week 4, 8, 12, 16 or 20) and maintaining the decrease until at least about week 44 following the initial dose; and/or

○ a reduction in CRT of about 159, 160, 161 or 162 micrometers (or more) by about week 4 or 8 or 12 and maintaining a reduction of about 159, 160, 161 or 162 micrometers (or more) until at least about week 44;

• with respect to retinal fluid, achieving:

○ a dry retina (*e.g.*, no IRF and no SRF; or no IRF; or no SRF, *e.g.*, in the center subfield or in the macula, *e.g.*, on SD-OCT);
○ no fluid in the center subfield (*e.g.*, no IRF and no SRF; or no IRF; or no SRF) by about week 4, 8, 9, 12, 16, 20, 24, 28, 32, 36, 40 or 44 following the initial dose (*e.g.*, as measured by SD-OCT);
○ no sub-retinal pigment epithelium (RPE) fluid; *e.g.*, by about week 16, *e.g.*, until at least about week 44 following the initial dose (*e.g.*, as measured by SD-OCT);
○ no SRF and IRF in the macula, *e.g.*, by SD-OCT, *e.g.*, by week 16 or week 44 following the initial dose, and/or
○ maintenance of a dry retina, *e.g.*, once achieved (*e.g.*, at 16 weeks following the initial dose); until at least about week 44 (*e.g.*, as measured by SD-OCT);

• achieving:

○ reduction in total lesion size by at least about 3.3 $\mu$m; and/or choroidal neovascularization (CNV) size of at least about 3.2 $\mu$m from baseline by about week 4, 8, 9, 12, 16, 20, 24, 28, 32, 36, 40 or 44, *e.g.*, wherein baseline total lesion size is about 7.7 $\mu$m and/or baseline CNV size is about 7.5 $\mu$m;
○ no significant increase in intraocular pressure from baseline by about week 4, 8, 9, 12, 16, 20, 24, 28, 32, 36, 40 or 44 or later (*e.g.*, by no more than about 0.2 or 0.5 mmHg); and/or
○ no significant increase in blood pressure (*e.g.*, systolic (S) or diastolic (D)) from baseline by about week 4, 8, 9, 12, 16, 20, 24, 28, 32, 36, 40 or 44 or later (*e.g.*, by no more than about 0.1 (S) or 0.9 (S), 1.0 (D) or 1.4 (D) mmHg); *e.g.*, wherein baseline systolic pressure is about 125 mmHg or 129 mmHg and/or baseline diastolic pressure is about 72 mmHg or 74 mmHg;
and/or

• with respect to efficacy or safety:

○ Efficacy and/or safety, in a subject suffering from DR or DME, similar to or greater than that of aflibercept which is intravitreally dosed at 2 mg approximately every 4 weeks for the first 5 injections followed by 2 mg approximately once every 8 weeks or once every 2 months, *e.g.*, wherein efficacy is measured as an increase in visual acuity or BCVA and/or a reduction in central retinal thickness, achievement of dry retina (*e.g.*, no IRF and/or SRF), *e.g.*, wherein safety is as measured as the incidence of adverse events (treatment-emergent adverse events occurring anytime within 30 days of any injection) such as intraocular inflammation, clinically significant blood pressure increase, clinically significant intraocular pressure increase, visual impairment, vitreous floaters, vitreous detachment, iris neovascularization and/or vitreous hemorrhage; and/or
○ Efficacy and/or safety, in a subject suffering from nAMD, similar to or greater than that of aflibercept which is intravitreally dosed at 2 mg approximately every 4 weeks for the first 3 injections followed by 2 mg approximately once every 8 weeks or once every 2 months, *e.g.*, wherein efficacy is measured as an increase in visual acuity or BCVA and/or a reduction in central retinal thickness, achievement of dry retina (*e.g.*, no IRF and/or SRF), *e.g.*, wherein safety is as measured as the incidence of adverse events (treatment-emergent adverse events occurring anytime within 30 days of any injection) such as intraocular inflammation, clinically significant blood pressure increase, clinically significant intraocular pressure increase, visual impairment, vitreous floaters, vitreous detachment, iris neovascularization and/or vitreous hemorrhage;

for example, wherein such an effect (*e.g.*, improvement of BCVA, CRT and/or retinal fluid) are achieved and/or maintained for as long as the subject is receiving the treatment regimen.

The center subfield of the retina is a 1 mm diameter area around the macula. The macula itself is about 6 mm in diameter.

The present invention also includes methods for achieving any one or more of the foregoing in a subject (*e.g.*, increase in VA or BCVA, or decrease in CRT) suffering from an angiogenic eye disorder, *e.g.*, nAMD, DR or DME, comprising administering to an eye of the subject, a single initial dose of about 8 mg or more of a VEGF antagonist (e.g., aflibercept), followed by one or more (*e.g.*, 3, 4 or 5) secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 12 weeks after the immediately preceding dose.

The present invention includes methods for treating or an angiogenic eye disorder (*e.g.*, nAMD, DR, DME or ME-RVO), in a subject in need thereof, comprising administering to an eye of the subject, a single initial dose of about 8 mg or more of a VEGF antagonist (e.g., aflibercept), followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 12 weeks after the immediately preceding dose;

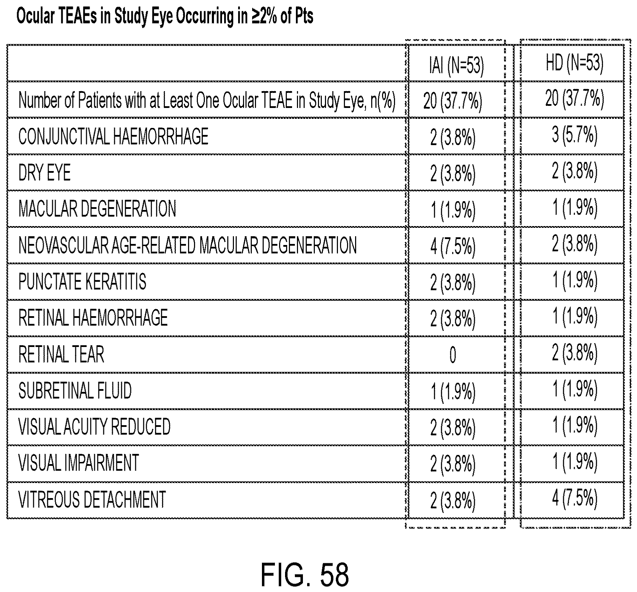

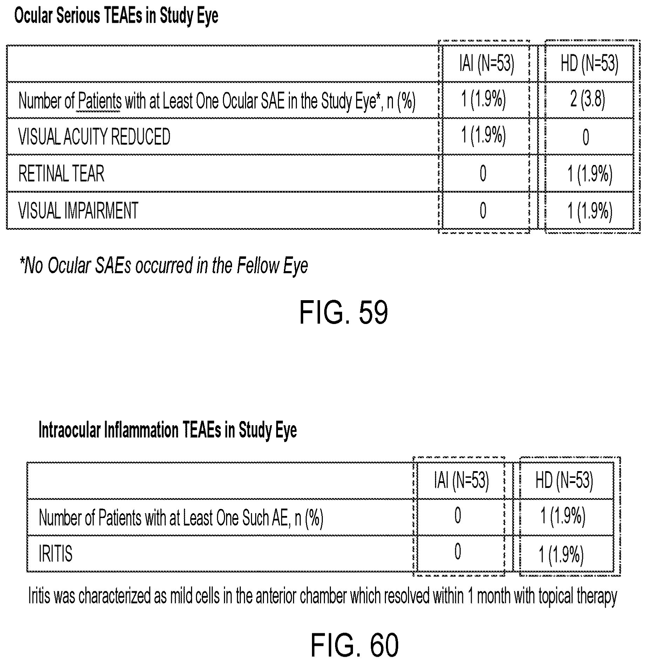

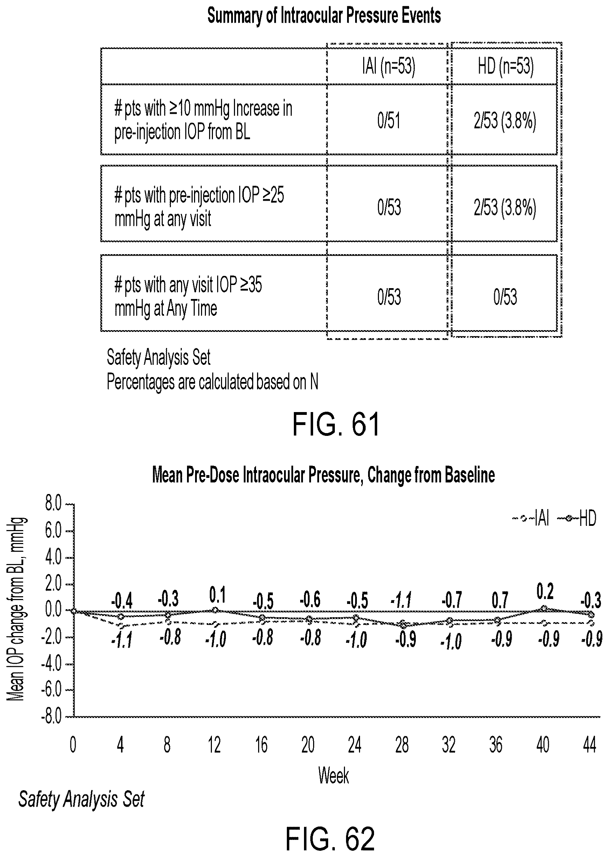

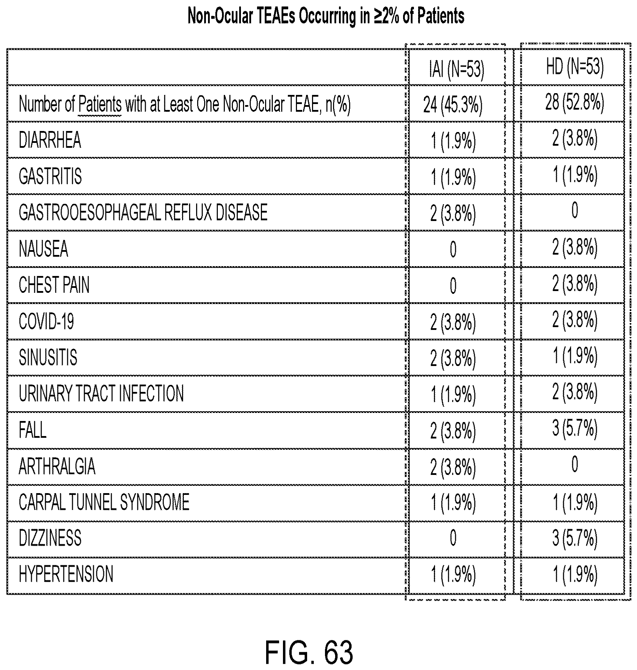

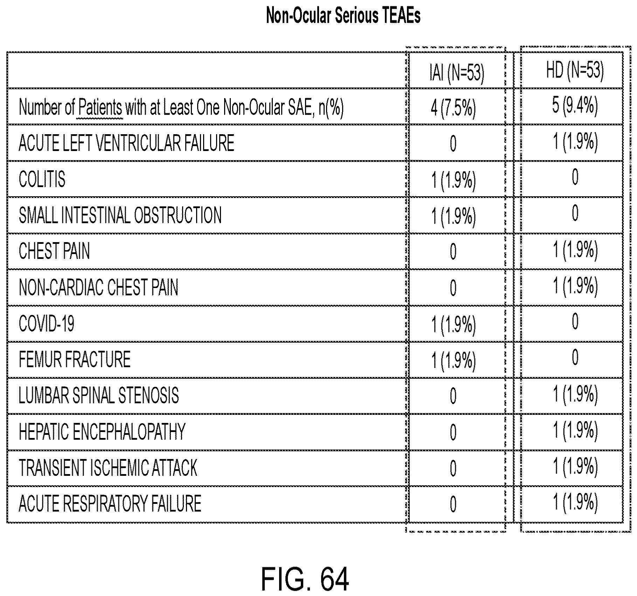

> wherein the subject achieves a change in central retinal thickness, from baseline at the initiation of treatment, as depicted in Figure 54 or Figure 55 (or a greater reduction), e.g., by the timepoint shown for such a change; and/or
> wherein the subject achieves a change in central retinal thickness, during the time period as set forth in the inset box of Figure 54 (or a greater reduction),
> and/or
> wherein the subject achieves a change in BCVA, from baseline at the initiation of treatment, as depicted in Figure 56 (or a greater increase), e.g., by the timepoint shown for such a change (such change in BCVA can be in terms of ETDRS letters gained or in the Snellen equivalent); and/or
> wherein the subject does not experience any one or more of the ocular TEAEs set forth in Figure 58, ocular serious TEAEs set forth in Figure 59, intraocular inflammation, any of the intraocular pressure events set forth in Figure 61, non-ocular TEAEs set forth in Figure 63, non-ocular serious TEAEs set forth in Figure 64, hypertension TEAEs set forth in Figure 65 and/or an APTC event.

**Best Corrected Visual Acuity (BCVA)**

Best corrected visual acuity (BCVA) can be measured in various methods known in the art. First, determining the proper level of lens refraction needed to best correct the visual acuity (VA) of a subject is determined before testing best corrected visual acuity (BCVA) with a visual acuity chart.

Two separate VA charts used for testing the right and left eye (*e.g.*, Sloan Letter ETDRS Chart 1 and Sloan Letter ETDRS Chart 2, respectively), and a third refraction chart is used for testing appropriate refraction (*e.g.*, Sloan Letter ETDRS Chart R). The features of the Sloan charts are high-contrast Sloan letters of equal difficulty, 5 letters in each of 14 rows, and a geometric progression of letter size (and, thus, an arithmetic progression of the logarithm of minimum angle of resolution [LogMAR]) from row to row. The charts have different letter sequences.

There are three basic components to determining refraction according to ETDRS protocol-determining spherical power, determining cylindrical axis and determining cylindrical power using methods known by practitioners in the art. For assessing refraction, if the subject wears contact lenses and has glasses, he or she should be told not to wear the contact lenses on the day of the examination or remove them 30-60 minutes before refraction is done. A trial frame is placed and adjusted on the subject's face so that lens cells placed in the frame are parallel to the anterior plane of the orbits and centered in front of the pupils. (It is permissible to use a phoroptor for subjective refraction. However, for testing visual acuity, the lenses from the final phoroptor refraction must be placed in a frame, and the final sphere must be rechecked).

BCVA can be measured first in one eye with a visual acuity chart, and then in the other eye with another visual acuity chart (*e.g.*, Charts 1 and 2 as discussed herein), wherein each chart remains hidden from view until the eye in question is ready for testing. The distance from the subject's eyes to the visual acuity chart is typically 4 meters (13 feet and 1.5 inches, or 157.5 inches). The subject should be asked to read slowly (*e.g.*, at a rate not faster than about one letter per second). Eyes reading 19 or fewer letters correctly at 4 meters can be tested at 1 meter.

Two commonly used tools for testing visual acuity (VA) or BCVA include the Snellen and the Early Treatment Diabetic Retinopathy Study (ETDRS) VA charts (Early Treatment Diabetic Retinopathy Study research group. Photocoagulation for diabetic macular edema. Early Treatment Diabetic Retinopathy Study report number 1, Arch Ophthalmol. 1985 Dec;103(12):1796-806; Chen et al., Comparison of visual acuity estimates using three different letter charts under two ambient room illuminations. 2012;60(2):101-104; Bailey & Lovie, New design principles for visual acuity letter charts, 1976;53(11):740-745; Shamir et al., Comparison of Snellen and Early Treatment Diabetic Retinopathy Study charts using a computer simulation, Int. J. Opthamology 9(1): 119-123 (2016); Kaiser, Prospective Evaluation of Visual Acuity Assessment: A Comparison of Snellen Versus ETDRS Charts in Clinical Practice (An AOS Thesis), Trans Am Ophthalmol Soc 2009;107:311-324). A version of the Bailey-Lovie chart (Bailey & Lovie, New design principles for visual acuity letter charts. Am J Optometry Physiol Opt 1976;53:740-745) was modified in 1982 based on the recommendations of the Committee on Vision of the National Academy of Sciences, National Research Council, and Working Group 39, and by Dr. Rick Ferris for use in the Early Treatment Diabetic Retinopathy Study (ETDRS). The "ETDRS chart" and the protocol to test vision with the chart is commonly used in clinical trials. The ETDRS chart is typically tested from a shorter distance (13 feet (or 4 meters) rather than 20 feet) than Snellen, but does not allow the use of mirrors to simulate the correct distance, has the same amount of letters in every row (five letters each), and has an equal spacing of both the letters and the rows on a logarithmic scale. The Snellen Chart uses a geometric scale to measure visual acuity, with normal vision at a distance being set at 20/20. In an embodiment of the invention, VA or BCVA can be expressed in terms of ETDRS or Snellen. ETDRS VA values can be converted to a corresponding Snellen equivalent using methods known in the art. In an embodiment of the invention, VA or BCVA is measured with an ETDRS chart or with a Snellen chart.

**Precision Dose Drug Delivery**

The present invention provides methods as set forth herein wherein a VEGF antagonist (*e.g.,* aflibercept) is delivered with a high amount of precision, *e.g.*, with a drug delivery device (DDD) (*e.g.*, with a 0.5 mL volume), whether pre-filled or capable of being filled from a vial, and delivering a volume of between 70 and 100 microliter with an average volume of about 81 or 82 or 81-82 microliters, *e.g.*, with a standard deviation of about 4 or 5 or 4-5 microliters (*e.g.*, about 4.5 or 4.46 microliters) or less. In an embodiment of the invention, the DDD is a syringe, *e.g.*, with a 30 gauge, ½ inch needle.

One means for ensuring precision of a dose to be delivered with a device, such as a syringe, is by employing a syringe wherein the dose volume is device-determined. If the dose volume is device-determined, the device is designed only to deliver a single volume (*e.g.*, 87 microliters) or a single volume with a limited amount of acceptable error (± 4-5 microliters). Thus, if used properly, the user cannot deliver the wrong dose (*e.g.*, cannot deliver more than the intended volume from the device).

The present invention includes embodiments wherein, a precise dosage of about 8 mg or more is a dose of about 9, 9.3, 9.33, 9.7, 9.8, 9.9, 9.7-9.9 mg or more ± about 0.5, or ± about 0.51 mg is delivered to a subject's eye. The volume in which a dose is delivered can be, for example, about 70, 81, 82, 81.7, 85, 86, 87, 85-87 microliters ± about 4, 4.45, 4.5, or 5 microliters. Doses may be delivered with a dose delivery device (DDD) which is a syringe.

Highly precise doses of VEGF antagonist (*e.g.*, aflibercept) may be delivered, for example, in a volume that is device-determined (wherein the device is a syringe), by a method that includes the steps: (a) priming the syringe (*e.g.*, a pre-filled syringe), thereby removing air from the syringe and, thus avoiding injection of air into the eye, by advancing the plunger rod by a predetermined distance into the syringe body until advancement of the plunger rod is resisted by a stop; (b) rotating the plunger rod about a longitudinal axis; and (c) actuating the plunger rod to dispense a predetermined (device-determined) volume (*e.g.*, about 70, 81, 82, 81.7, 85, 86, 87, 85-87 microliters, ± about 4, 4.45, 4.5, or 5 microliters) of the formulation.

In an embodiment of the invention, the drug delivery device (DDD), comprises:

- a barrel including a longitudinal axis, a proximal end region, and a distal end region, the proximal end region including an opening, wherein the barrel is configured to receive a drug therein;
- a plunger rod disposed at least partially inside the barrel and protruding from the opening, wherein the plunger rod includes a rack having a plurality of teeth; and
- a pinion having a plurality of teeth configured to engage with the plurality of teeth of the rack,

wherein rotation of the pinion against the rack moves at least a part of the plunger rod along the longitudinal axis of the barrel; for example, which further comprises a shaft affixed to the pinion, wherein rotation of the shaft rotates the pinion against the rack which may include a knob affixed to the shaft. In an embodiment of the invention, the DDD further includes a magnifier disposed on the distal end region of the barrel. In an embodiment of the invention, the DDD further includes a stopper inside the barrel, wherein the stopper is affixed to a distal end of the plunger rod. In an embodiment of the invention, the DDD further includes a circular ratchet disposed coaxially with the pinion, wherein the circular ratchet has a diameter smaller than a diameter of the pinion; a spring-loaded pawl disposed on an internal circumference of the pinion, wherein the pawl is configured to engage the ratchet; and a shaft affixed to the ratchet, wherein rotation of the shaft in one direction causes rotation of the pinion, and rotation of the shaft in a second direction does not cause rotation of the pinion for example wherein the ratchet is disposed inside the pinion. In an embodiment of the invention, the pinion includes a plurality of teeth having a first height, and a stopper tooth having a second height greater than the first height, for example, wherein the second height of the stopper tooth prevents the pinion from engaging the plurality of teeth of the rack, and/or wherein the second height of the stopper tooth is configured to contact one of the plunger rod and the rack to stop rotation of the pinion. In an embodiment of the invention, the plunger rod includes an inner column and an outer lumen, and wherein the rack is disposed on the inner column, *e.g.*, wherein rotation of the pinion against the rack moves the inner column of the plunger rod independently of the outer lumen, and/or further including a shaft removably affixed to the pinion, wherein the shaft prevents movement of the outer lumen of the plunger rod relative to the barrel, and wherein removal of the shaft allows for movement of the outer lumen of the plunger rod relative to the barrel. In an embodiment of the invention, the plunger rod further includes a body and a flange, the flange extending partially along a longitudinal length of the body and having a width greater than a width of the body; wherein the barrel further comprises a plunger lock, the plunger lock including a through hole configured to allow the flange to pass through the second plunger lock in a specific orientation.

In an embodiment of the invention, the drug delivery device (DDD), comprises:

- a barrel including a longitudinal axis, a proximal end region, a distal end region, and an interior, the proximal end region including an opening and the interior including a threaded region; and
- a plunger rod disposed at least partially inside the barrel and protruding from the opening, the plunger rod including a threaded region configured to engage the threaded region of the barrel interior,

wherein rotation of the plunger rod about the longitudinal axis of the drug delivery device moves the plunger rod along the longitudinal axis. In an embodiment of the invention, the plunger rod further includes a tab protruding from the plunger rod in a first direction and located proximally from the threaded region of the plunger rod, and wherein the threaded region in the interior of the barrel further includes a slot sized and configured to allow for the tab to pass through the threaded region in the interior of the barrel, *e.g.*, wherein the slot includes a first segment parallel to the longitudinal axis of the drug delivery device and a second segment perpendicular to the longitudinal axis of the drug delivery device-the slot may include a third segment parallel to the longitudinal axis of the drug delivery device, wherein the second segment is in between the first segment and the third segment. In an embodiment of the invention, the tab is a first tab, and wherein the plunger rod further includes a second tab protruding from the plunger rod in a second direction opposite to the first direction, and wherein the threaded region in the interior of the barrel further includes a second slot sized and configured to allow for the second tab to pass through the threaded region in the interior of the barrel.

In an embodiment of the invention, the drug delivery device, includes:

- a barrel having a proximal end region, a distal end region, an opening in the proximal end region, an interior, and a threaded region in the interior;
- a sleeve disposed partly inside the barrel and protruding from the opening in the proximal end region of the barrel, the sleeve including a threaded region engaged with the threaded region of the barrel interior;
- a plunger rod disposed at least partially inside the sleeve; and
- a stopper inside the barrel and located distally from the sleeve, the stopper connected to a distal end of the plunger rod,

wherein rotation of the sleeve in a first direction around a longitudinal axis of the drug delivery device moves the sleeve towards the distal end region of the barrel. In an embodiment of the invention, rotation of the sleeve in the first direction moves the stopper towards the distal end region of the barrel. In an embodiment of the invention; the sleeve includes an inner passage, and the stopper has a diameter larger than a diameter of the inner passage; and/or the sleeve includes a tab disposed on an exterior of the sleeve, the tab located proximally from the threaded region of the barrel interior, and wherein the tab stops movement of the sleeve towards the distal end region of the barrel, *e.g.*, wherein the tab is configured to stop movement of the sleeve towards the distal end region of the barrel after the drug delivery device has been primed or wherein the tab is a first tab, and wherein the sleeve further includes a second tab disposed on an exterior of the sleeve, the second tab located distally from the threaded region of the barrel interior, wherein the second tab stops movement of the sleeve towards the proximal end region of the barrel.

In an embodiment of the invention, the drug delivery device, comprises:

- a barrel including a proximal end region and a distal end region, the proximal end region including an opening;
- a plunger rod including a body and a flange, the flange extending partially along a longitudinal length of the body and having a width greater than a width of the body, the plunger rod disposed at least partially inside the barrel and protruding from the opening;
- a first plunger lock disposed on the barrel, the first plunger lock configured to block the flange from entering the barrel; and
- a second plunger lock disposed in the barrel, the second plunger lock including a through hole configured to allow the flange to pass through the second plunger lock in a specific orientation.

For example, in an embodiment of the invention, the first plunger lock is removable and/or frangible. In an embodiment of the invention, a distance between the first plunger lock and the second plunger lock is equivalent to the distance that the stopper must travel to prime the drug delivery device; and/or the plunger rod is rotatable around a longitudinal axis of the drug delivery device.

Substances from such a DDD (*e.g.*, a formulation including aflibercept as described herein), having a plunger rod and a barrel, may be dispensed as follows:

- advancing the plunger rod by a predetermined distance into the barrel until advancement of the plunger rod is resisted by a stop;
- deactivating the stop; and
- actuating the plunger rod (*e.g.*, which includes a flange, wherein the stop includes a lock that prevents the flange from entering the barrel; or which includes a flange, wherein the stop comprises a lock that prevents the flange from entering the barrel) to deliver the substance.

Advancing the plunger rod may include the step of rotating a pinion against a rack disposed on the plunger rod, *e.g.*, wherein the stop comprises a shaft removably affixed to the pinion, and wherein deactivating the stop comprises removing the shaft from the pinion.

Deactivating the stop may include the step of rotating the plunger rod. In an embodiment of the invention, deactivating the stop includes the step of removing the lock and/or breaking the lock.

In an embodiment of the invention, the drug delivery device, includes:

- a barrel including a longitudinal axis, a proximal end region, and a distal end region, the proximal end region including an opening and a rack disposed on the interior of the barrel, the rack having a plurality of teeth, wherein the barrel is configured to receive a drug therein;
- a plunger rod disposed at least partially inside the barrel and protruding from the opening, wherein the plunger rod includes a rack having a plurality of teeth; a pinion having a plurality of teeth configured to engage with the plurality of teeth of the plunger rod rack; and
- an inner plunger coupled to the pinion by a rod, wherein rotation of the pinion against the plunger rod rack results in movement of the inner plunger along the longitudinal axis of the barrel;

for example, wherein the teeth of the pinion are further configured to engage with the plurality of teeth of the rack disposed on the barrel. In an embodiment of the invention, the pinion is a first pinion, and further includes: a second pinion disposed coaxially with the first pinion, the second pinion having a diameter smaller than a diameter of the first pinion and a plurality of teeth configured to engage with the plurality of the teeth of the rack disposed on the barrel, wherein rotation of the first pinion results in rotation of the second pinion against the rack disposed on the barrel and in movement of the inner plunger along the longitudinal axis of the barrel.

See International patent application publication no. WO2019/118588.

In an embodiment of the invention, the drug delivery device (DDD), includes:

- a body;
- a plunger rod disposed partially inside the body;
- a protrusion extending from the plunger rod; and
- a blocking component coupled to a proximal end portion of the body, wherein the blocking component is a flange piece,

wherein, when the protrusion is in a first position relative to the blocking component, the blocking component restricts distal movement of the plunger rod to a first stopping point, and when the protrusion is in a second position relative to the blocking component, the blocking component restricts distal movement of the plunger rod to a second stopping point. In an embodiment of the invention, the DDD further includes: a stopper disposed in the body, wherein distal movement of the plunger rod distally moves the stopper; and a drug substance disposed in the body in between the stopper and a distal end of the body, wherein distal movement of the plunger rod to the first stopping point primes the drug delivery device, and distal movement of the plunger rod to the second stopping point dispenses a predetermined volume of the drug substance from a distal end of the device.

In an embodiment of the invention, moving the protrusion from the first position to the second position includes twisting the plunger rod relative to the blocking component. In an embodiment of the invention, the DDD further includes: a cavity in a proximal side of the blocking component, the cavity sized and configured to receive a portion of the protrusion, wherein when the protrusion is in the second position relative to the blocking component, the protrusion is positioned proximally from the cavity, such that distal movement of the plunger rod moves the protrusion into the cavity; *e.g.*, wherein the cavity is a first cavity, and further includes: a second cavity in a proximal side of the blocking component, the second cavity sized and configured to receive a portion of the protrusion, wherein the first and second cavity are located on opposite sides of a central longitudinal axis of the drug delivery device. In an embodiment of the invention, the plunger rod passes through an opening in the blocking component. In an embodiment of the invention the DDD further includes an actuation portion at a proximal end portion of the plunger rod, wherein the protrusion extends from the actuation portion, *e.g.*, wherein the actuation portion includes a generally cylindrical shape having a diameter greater than a width of the remainder of the plunger rod, wherein the protrusion extends from a side of the generally cylindrical shape, and wherein the actuation portion further comprises: a thumb pad on a proximal end of the actuation portion; and a ring on an exterior surface on the side of the generally cylindrical shape; *e.g.*, further including a proximal collar on the blocking component, wherein the actuation portion partially fits inside the proximal collar; *e.g.*, wherein the plunger rod further includes a pair of extensions protruding distally from the actuation portion and the blocking component (*e.g.*, which includes one or more indents formed along a bottom wall of the blocking component; and wherein a portion of each extension is configured to be received by the one or more indents upon distal movement of the plunger rod relative to the blocking component to allow distal movement of the plunger rod to the second stopping point; or, which includes one or more indents formed along a bottom wall of the blocking component; and wherein a portion of each extension is configured to be received by the one or more indents upon distal movement of the plunger rod relative to the blocking component to allow distal movement of the plunger rod to the second stopping point; or, which includes a pair of internal grooves formed along a sidewall of the blocking component; and wherein a portion of each extension is configured to be received by at least one of the pair of internal grooves upon rotation of the plunger rod relative to the blocking component to expand the extensions radially-outward from a compressed state to a relaxed state) includes a pair of openings; and wherein a portion of each extension is configured to be received by one of the pair of openings in the first stopping point. In an embodiment of the invention, the protrusion is a first protrusion, and further includes a second protrusion extending from the plunger rod in a direction opposite to the first protrusion. In an embodiment of the invention, the blocking component is slidably coupled to the body and includes a third cavity and a pair of ribs that extend into the third cavity, wherein the body includes a top flange and the pair of ribs are configured to engage the top flange received in the third cavity; wherein the pair of internal ribs are configured to apply a distally-directed force onto the top flange. In an embodiment of the invention, the blocking component is slidably coupled to the body and includes a pair of movable tabs that are configured to engage the body; and the pair of movable tabs are laterally deflectable upon receiving the body in the blocking component and are configured to apply a radially-inward directed force onto the body. In an embodiment of the invention, the blocking component further includes a pair of finger flanges, and each of the finger flanges includes a textured surface having a predefined pattern that increases a grip of the blocking component.

In an embodiment of the invention, the drug delivery device (DDD), includes:

- a body;
- a plunger rod having a distal end contacting a stopper inside the body, and a proximal end including an actuation portion with a thumb pad;
- a plurality of protrusions extending from the actuation portion; and
- a blocking component disposed on the body, the blocking component including a proximal collar having a plurality of slots,

wherein, when the protrusions and the slots are in a first configuration relative to one another, the blocking component restricts distal movement of the plunger rod to a first stopping point, and when the protrusions and the slots are in a second configuration, the blocking component restricts distal movement of the plunger rod to a second stopping point, wherein, in the second configuration, the slots are configured to receive the protrusions upon distal movement of the plunger rod. In an embodiment of the invention, the protrusions and the slots are movable from the first configuration to the second configuration by rotation of the actuation portion about a longitudinal axis in relation to the blocking component, and wherein when the protrusions and the slots are in the second configuration, the protrusions and the slots are not movable to the first configuration; and/or a difference between the first stopping point and the second stopping point is equivalent to a distance that the stopper must travel to expel a predetermined volume of a drug product from a distal end of the body, and wherein the plunger rod is prevented from moving from the second stopping point to the first stopping point; and/or the plurality of protrusions includes two protrusions disposed symmetrically about the actuation portion; and/or the blocking component further comprises a pair of finger flanges; and/or the drug delivery device is a pre-filled syringe; and/or the drug delivery device is changeable: (a) from a pre-use state to a primed state, by longitudinally moving the plunger rod (e.g., wherein the plunger rod includes a neck disposed distally from the actuation portion, wherein the neck interfaces with an opening in the blocking component to prevent proximal movement of the plunger rod, for example, wherein the neck further interfaces with the opening in the blocking component to prevent movement of the drug delivery device from the delivery state to the primed state) until the plunger rod reaches the first stopping point; (b) from the primed state to a delivery state by rotating the plunger rod in relation to the blocking component until the protrusions and the blocking component are in the second configuration; and (c) from a delivery state to a used state by longitudinally moving the plunger rod until the plunger reaches the second stopping point, wherein the drug delivery device is not changeable from the used state to the delivery state, from the delivery state to the primed state, or from the primed state to the pre-use state. In an embodiment of the invention, when the plunger rod is at the second stopping point, the stopper does not contact a distal end of the body.

In an embodiment of the invention, a drug delivery device, includes:

- a body;
- a plunger rod, including:
- a distal portion contacting a stopper inside the body;
- a proximal end including a generally cylindrical actuation portion disposed outside of the body; and
- two protrusions extending from opposite sides of the actuation portion in a symmetrical configuration; and
- a blocking component coupled to the body, the blocking component including: a collar configured to accept a distal part of the actuation portion; and two cavities in the collar having proximally-facing openings, wherein each cavity is configured to accept a distal portion of one of the two protrusions;

wherein the plunger rod is longitudinally movable and rotatable about a longitudinal axis relative to the blocking component, and

wherein, when the drug delivery device is in a pre-use state, the protrusions and the cavity openings are not longitudinally aligned, and when the drug delivery device is in a delivery state, the protrusions and the cavity openings are longitudinally aligned. In an embodiment of the invention, the blocking component further includes a finger flange, and further includes a ribbed surface on a side of the actuation portion. In an embodiment of the invention, the plunger rod further includes: two extensions protruding distally from the actuation portion; and a plurality of openings in the collar of the blocking component,

wherein a portion of each extension is configured to be received by one of the plurality of openings upon distal movement of the plunger rod relative to the blocking component.

In an embodiment of the invention, a drug delivery device includes:

- a body;
- a stopper disposed inside the body;
- a sleeve having a proximal end and a distal end, the distal end being disposed inside the body, proximally from the stopper; and
- a plunger rod disposed at least partially inside the sleeve;

wherein, when the stopper is in a ready position, distal advancement of one of (a) only the sleeve, (b) only the plunger rod, or (c) both the sleeve and the plunger rod together, relative to the body advances the stopper to a primed position, and wherein, when the stopper is in the primed position, distal advancement of another of (a) only the sleeve, (b) only the plunger rod, or (c) both the sleeve and the plunger rod together, relative to the body advances the stopper to a dose completion position. For example, in an embodiment of the invention, a DDD further includes a removable blocking component (e.g., wherein the blocking component is a clip removably secured around at least a portion of the sleeve) disposed between a proximal portion of the sleeve and a proximal end of the body, the blocking component obstructing distal advancement of the sleeve relative to the body, wherein distal advancement of the sleeve relative to the body after removal of the blocking component advances the stopper to the primed position. In an embodiment of the invention, the DDD further includes a removable locking component (e.g., a pin, a tab, or a bar) that couples the plunger rod to the sleeve, wherein distal advancement of both the sleeve and the plunger rod together relative to the body advances the stopper to the primed position, wherein distal advancement of only the plunger rod relative to the body after removal of the locking component advances the stopper to the dose completion position. In an embodiment of the invention, in the dose completion position, a proximal end of the plunger rod abuts against a distal end of the sleeve, such that the plunger rod is prevented from advancing distally any further relative to the body. In an embodiment of the invention, the DDD further includes a protrusion disposed on the plunger rod; and an inner protrusion disposed on an interior wall of the sleeve distally to the protrusion of the plunger rod, wherein distal advancement of only the plunger rod relative to the body advances the stopper to the primed position and causes the protrusion of the plunger rod to contact the inner protrusion of the sleeve, and wherein distal advancement of both the plunger rod and the sleeve relative to the body, after the protrusion of the plunger rod has contacted the inner protrusion of the sleeve, advances the stopper to the dose completion position. In an embodiment of the invention, the sleeve includes a finger flange. In an embodiment of the invention, the DDD further includes a stop disposed at a proximal end of the body, the stop sized to block distal advancement of the sleeve or the plunger rod once the stopper is in the completion position.

In an embodiment of the invention, a drug delivery device, includes:

- a body;
- a plunger rod having a distal portion disposed inside the body and a proximal portion disposed outside a proximal end of the body, the proximal portion having a width greater than a width of the distal portion; and
- an obstruction that, in an obstructing position relative to the plunger rod, prevents distal advancement of the plunger rod from a primed position to a dose completion position,

wherein displacement of the obstruction from the obstructing position permits distal advancement of the plunger rod to the dose completion position, for example, further including a collar affixed to a proximal end portion of the body, the collar surrounding the proximal portion of the plunger rod; and a collar projection extending radially inward from the collar, wherein the proximal portion of the plunger rod includes a channel into which the collar projection protrudes, the channel including a circumferential path and an axial dose completion path, wherein the obstruction comprises the collar projection, which, when disposed in the circumferential path of the channel, prevents distal advancement of the plunger rod to the dose completion position, and wherein displacement of the obstruction from the obstructing position comprises twisting the plunger rod about a longitudinal axis to align the collar projection with the axial dose completion path. For example, in an embodiment of the invention, the channel further includes an axial priming path offset from the axial dose completion path, and connected to the axial dose completion path by the circumferential path, and distal movement of the plunger rod such that the collar projection travels on the axial priming path advances the plunger rod to the primed position. In an embodiment of the invention, the DDD further includes a finger flange. In an embodiment of the invention, the proximal portion of the plunger rod includes a projection extending radially outward, and the drug delivery device further includes: a rotatable alignment component disposed in between the proximal portion of the plunger rod and the body, the alignment component including a channel, the channel sized and configured to accommodate the plunger rod projection, wherein the obstruction comprises a wall of the channel that blocks a distal axial path of the plunger rod projection when the plunger rod is in the primed position, and wherein displacement of the obstruction from the obstructing position comprises rotating the alignment component to remove the wall of the channel from the distal axial path of the plunger rod projection, e.g., further including a finger flange coupled to a proximal end portion of the body, wherein the rotatable alignment component is disposed between the finger flange and the proximal portion of the plunger rod. In an embodiment of the invention, the DDD further includes a flange piece disposed at the proximal end of the body, wherein the obstruction includes a removable cap that, when in the obstructing position relative to the plunger rod, is disposed partially in between the proximal portion of the plunger rod and the flange piece. In an embodiment of the invention, removal of the cap allows the proximal portion of the plunger rod to advance to a dose completion position, wherein, in the dose completion position, the proximal portion of the plunger rod contacts the flange piece. In an embodiment of the invention, the removable cap covers the proximal portion of the plunger rod when in the obstructing position. In an embodiment of the invention, the DDD further includes a collar disposed between the proximal end of the body and the proximal portion of the plunger rod, the collar defining an opening sized to accommodate the proximal portion of the plunger rod upon distal advancement of the plunger rod beyond a primed position; wherein the obstruction comprises a tab protruding radially outward from the proximal portion of the plunger rod, the tab preventing the proximal portion of the plunger rod from fitting into the opening of the collar, and wherein a depth of the collar opening coincides with a distance the plunger rod must travel to advance distally to the dose completion position, e.g., wherein displacement of the obstruction from the obstructing position comprises either removing the tab or compressing the tab into a side of the proximal portion of the plunger rod; and/or wherein the tab is a first tab, and wherein the obstruction further comprises a second tab protruding radially outward from the proximal portion of the plunger rod in a direction opposite the protruding direction of the first tab; and/or wherein the obstruction comprises a tab that, when in the obstructing position, is disposed between the body and the proximal portion of the plunger rod, and wherein the plunger rod includes a geometry disposed proximally from the tab, wherein the geometry cannot advance distally past the tab when the tab is in the obstructing position. For example, displacement of the obstruction may include removing the tab from the drug delivery device by pulling the tab. In an embodiment of the invention, the DDD further includes a flange piece, wherein a portion of the tab is disposed inside a cavity of the flange piece. In an embodiment of the invention, displacement of the obstruction comprises removing the tab from the drug delivery device by breaking the tab. In an embodiment of the invention, the obstruction includes a flange piece that, in the obstructing position, is disposed proximally from the proximal end of the body, between the proximal portion of the plunger rod and the body, and is spaced from the proximal end of the body by a removable blocking component, and wherein displacement of the obstruction from the obstructing position comprises: removing the blocking component; and shifting the flange piece distally towards the proximal end of the body. In an embodiment of the invention, the plunger rod includes a projection extending radially outward, wherein the obstruction includes a lever having an end that, in the obstructing position, is located distally from the projection and blocks distal movement of the projection and thereby distal movement of the plunger rod, and wherein displacement of the obstruction from the obstructing position comprises actuating the lever to remove the end of the lever from its location distal from the projection. In an embodiment of the invention, distal advancement of the plunger rod beyond the dose completion position is prevented by contact between the proximal portion of the plunger rod and a portion of a flange piece coupled to the body.

In an embodiment of the invention, the drug delivery device, includes:

- a body;
- a sleeve affixed to the body, the sleeve including a proximal end, a distal end, and an opening disposed in a circumferential wall of the sleeve;
- a plunger rod passing through the sleeve, the plunger rod including a distal end portion disposed inside the body, and a radially-extending protrusion;

wherein the plunger rod may be distally advanced into the body from a ready position to a primed position, wherein, in the primed position, the protrusion of the plunger rod is disposed inside the opening, and further distal advancement of the plunger rod is resisted by contact between the protrusion and a wall of the opening, and wherein pressure may be exerted on the protrusion to overcome the resistance to further distal advancement of the plunger rod. In an embodiment of the invention, the opening in the sleeve is a second opening, and the sleeve further includes a first opening disposed in the circumferential wall of the sleeve proximally from the second opening, and a third opening disposed in the circumferential wall of the sleeve distally from the second opening, wherein, in the ready position, the protrusion of the plunger rod is disposed in the first opening, and further distal advancement of the plunger rod is resisted by contact between the protrusion and a wall of the first opening, and wherein, after further distal advancement of the plunger rod past the primed position, the protrusion of the plunger rod is disposed in the third opening, and further distal advancement of the plunger rod is prevented. In an embodiment of the invention, the radially-extending protrusion is a first protrusion, and wherein the plunger rod further includes a second radially-extending protrusion opposite the first protrusion, and wherein squeezing the first and second protrusions towards one another while applying axial pressure in the distal direction on the plunger rod overcomes the resistance to further distal advancement of the plunger rod. In an embodiment of the invention, the protrusion includes a distally-tapering profile to aid in distal advancement of the plunger rod.

In an embodiment of the invention, a drug delivery device, includes:

- a body;
- a plunger rod including a distal end portion disposed inside the body and a rotatable element; and
- a sleeve affixed to the body, the sleeve including a proximal opening into which the plunger rod may be advanced,

wherein rotating the rotatable element causes distal advancement of the plunger rod to a primed position, and wherein once the plunger rod is in the primed position, further rotation of the rotatable element is resisted. In an embodiment of the invention, the DDD further includes a collar disposed at a proximal end of the body, an interior of the collar including a proximal threaded portion forming a proximal helical path, wherein the rotatable element comprises a proximal portion of the plunger rod including a protrusion, wherein the proximal portion of the plunger rod may be rotated about a longitudinal axis to cause the protrusion to travel distally along the proximal helical path, and wherein once the protrusion reaches the end of the proximal threaded portion of the collar, the plunger rod is in the primed position, e.g., wherein once the plunger rod is in the primed position, the plunger rod may be depressed axially into the body to distally advance the plunger rod to a dose completion position; and/or wherein the interior of the collar further includes a distal threaded portion, wherein threads of the distal threaded portion form a distal helical path offset from, and opposite to, the proximal helical path, wherein alignment of the protrusion with the distal helical path places the plunger rod in the primed position, and wherein rotation of the proximal portion of the plunger rod to cause the protrusion to travel distally along the distal helical path causes distal advancement of the plunger rod to a dose completion position.

A substance may be dispensed using such a DDD having a plunger rod and a body, may be done by a method including:

(a) advancing the plunger rod by a predetermined distance into the body until advancement of the plunger rod is resisted by a stop;
(b) rotating the plunger rod about a longitudinal axis; and
(c) actuating the plunger rod to dispense a predetermined volume of the substance,

wherein none of steps (a), (b), and (c) are reversible. In an embodiment of the invention, the DDD further includes a flange piece having a collar, and advancing the plunger rod and actuating the plunger rod comprise pressing an actuation portion of the plunger rod into the collar of the flange piece; for example, wherein the plunger rod comprises a protrusion, and wherein the collar of the flange piece abuts against the protrusion to resist advancement of the plunger rod. For example, in an embodiment of the invention, wherein rotating the plunger rod comprises twisting an actuation portion of the plunger rod relative to the flange piece, until a protrusion on the plunger rod becomes longitudinally aligned with a cavity in the collar of the flange piece, which may further include advancing the protrusion into the cavity until the protrusion abuts a distal side of the cavity, wherein the predetermined volume of the substance is dispensed when the protrusion abuts the distal side of the cavity.

See International patent application publication no. WO2020/247686.

## EXAMPLES

### Example 1: A Randomized, Single-Masked, Active-Controlled Phase 2 Study of the Safety, Tolerability, and Efficacy of Repeated Doses of High-Dose Aflibercept in Patients with Neovascular Age-Related Macular Degeneration (CANDELA trial).

This phase 2, multi-center, randomized, single-masked study in patients with nAMD investigated the efficacy, safety, and tolerability of HD (8 mg doses) versus IAI (2mg doses). The Study dosing regimen is summarized in Figure 1. The Study consisted of a screening/baseline period, a treatment period, and an end of study (EOS) visit at week 44. Patients were seen monthly through week 44. One hundred and six eligible patients were randomized into 2 groups in a 1:1 ratio. One group received IAI and the other received HD. The investigational product was administered intravitreally (IVT) monthly for 3 initial injections (baseline, week 4, and week 8), followed by additional doses at weeks 20 and 32. At weeks 24, 28, 36 and 40, patients were evaluated and given a dose (at their randomized dose level) if either of the following criteria are met (PRN criteria):

• Loss of ≥5 letters from week 20 BCVA due to disease progression; or
• Anatomical findings that are considered vision threatening, such as worsening or persistent retinal fluid, new or worsening PED (pigment epithelial detachment), new or persistent hemorrhage, *etc.*

The following are the portions of the protocol by which the CANDELA human clinical trial was conducted.

The study also includes a pharmacokinetic (PK) sub-study, with dense blood sampling (dense PK sub-study) for systemic drug concentrations and PK assessments for approximately 15 patients from each group from selected sites. Additional patients (up to approximately 50% more in each treatment group) may be enrolled in the dense PK sub-study to ensure adequate data is are captured.

Dosing schedule

The dosing schedule for the IAI and HD groups are set forth below in Table 1-1.

**Table 1-1. Dosing schedule of IAI and HD groups in CANDELA study.**

| | | | Wk 4 Safety Analysis | | | | Wk 16 Efficacy Analysis | | | | | | | Wk 44 Analysis ECS |
|---|---|---|---|---|---|---|---|---|---|---|---|---|---|---|
| | Screen 1 & 2 | Day 1 (BL) | Wk 4 | Wk 8 | Wk 8 | Wk 12 | Wk 16' | Wk 20 | Wk 24 | Wk 28 | Wk 32 | Wk 38 | Wk 40 | Wk 44 |
| IAI - 2 mg 50 μl | | X | X | X | BP/ PK | | | X | PRN | PRN | X | PRN | PRN | |
| HD - 8 mg 70 μl | | X | x | X | BP/ PK | | | X | PRN | PRN | X | PRN | PRN | |

Additional visits for Dense PK Substudy:

• Days 2, 3, 5, 8, 15 and 22

• Blood pressure & pharmacokinetic draws at all visits

• Urinalysis (UA) at days 8 & 15

Week 16: Additional treatment allowed after discussion with sponsor

See also Table 1-2 herein. As discussed in this Example, IAI dosing regimens call for 2 mg doses given as defined in Table 1-1; and HD dosing regimens call for 8 mg doses given as defined in Table 1-1.

Primary Endpoints

The co-primary endpoints are:

• Safety, which will be evaluated by assessment of treatment-emergent adverse events (TEAEs) and serious adverse events (SAEs) through week 4; and

• The proportion of patients without retinal fluid in the center subfield at week 16.

Secondary Endpoints

There are no secondary endpoints in this study.

Exploratory Endpoints

The exploratory endpoints are:

- The proportion of patients without retinal fluid in the center subfield at week 44;
- The proportion of patients without retinal fluid in the center subfield at week 16;
- Change in central retinal thickness (CRT) from baseline through week 16 and week 44;
- The proportion of patients without intraretinal fluid (IRF) at week 16 and week 44;
- The proportion of patients without subretinal fluid (SRF) at week 16 and week 44;
- The proportion of patients without sub-retinal pigment epithelium (RPE) fluid at week 16 and week 44;
- The proportion of patients able to maintain dry retina (total fluid, IRF, and/or SRF) through week 44;
- The proportion of patients able to maintain a 12-week dosing interval from week 8 through week 44;
- Change in CRT between dosing visits from week 8 through week 44;
- Change in Best Corrected Visual Acuity (BCVA) from baseline, and proportions of patients gaining and losing vision, through week 16 and week 44;
- Change in lesion size and choroidal neovascularization (CNV) size from baseline through week 20 and week 44;
- Other safety outcomes (e.g., TEAEs, SAEs, vital signs, clinical laboratory values; and intraocular pressure [IOP]) from baseline through week 16 and week 44; and
- Systemic PK of free and bound aflibercept assessed from baseline through week 44.

Efficacy Variables

The efficacy variable relevant to the primary efficacy endpoint is the assessment of retinal fluid. The efficacy variables relevant to the exploratory endpoints are:

- Assessment of retinal fluid levels (total fluid, IRF, and SRF) and retinal thickness on spectral domain optical coherence tomography (SD-OCT, or just OCT)
- Dosing interval;
- Visual acuity;
- Lesion size.

Safety Variables

The safety variable relevant to the primary safety endpoint is the proportion of patients with TEAEs and SAEs.
The safety variables relevant to the exploratory endpoints are:

- Ocular exams
- Vital signs
- Clinical laboratory values
- IOP

Pharmacokinetic Variables

The PK variables are the concentrations of free and bound aflibercept in plasma at each time point using both sparse sampling and dense sampling.

Number of Patients Planned

The study will enroll approximately 100 patients to be randomized in a 1:1 ratio.

Study Population

The study population consists of treatment-naïve patients with nAMD.

Inclusion Criteria

A patient must meet the following criteria at both the screening and/or the randomization visits to be eligible for inclusion in the study:

1. Men or women ≥50 years of age with active subfoveal CNV secondary to nAMD, including juxtafoveal lesions that affect the fovea in the study eye as assessed by an independent reading center.
2. Best Corrected Visual Acuity (BCVA) Early Treatment Diabetic Retinopathy Study (ETDRS) letter score of 78 to 24 (Snellen equivalent of 20/32 to 20/320) in the study eye.
3. Willing and able to comply with clinic visits and study-related procedures.
4. Provide informed consent signed by study patient or legally acceptable representative.

Exclusion Criteria

A patient who meets any of the following criteria at either the screening or randomization visits will be excluded from the study:

1. Evidence of CNV due to any cause other than nAMD in either eye.
2. Subretinal hemorrhage in the study eye that is ≥50% of the total lesion area.
3. Evidence of DME or diabetic retinopathy (defined as more than 1 microaneurysm) in either eye in diabetic patients.
4. Prior use of IVT anti-VEGF agents (aflibercept, ranibizumab, bevacizumab, brolucizumab, pegaptanib sodium) in the study eye.
5. Prior IVT investigational agents in either eye (e.g., anti-ang-2/anti-VEGF bispecific monoclonal antibodies, gene therapy).
6. Previous use of intraocular or periocular corticosteroids within 120 days of screening or treatment with an IVT steroid implant at any time in the study eye.
7. Treatment with ocriplasmin in the study eye at any time.
8. Yttrium-aluminium-garnet capsulotomy in the study eye within 14 days of the screening Visits.
9. History of vitreoretinal surgery (including scleral buckling) in the study eye.
10. Intraocular pressure ≥25 mm Hg in the study eye.
11. Evidence of infectious blepharitis, keratitis, scleritis, or conjunctivitis in either eye.
12. Any intraocular inflammation/infection in either eye within 90 days of the screening visit.
13. Any history of macular hole of stage 2 and above in the study eye.
14. Current iris neovascularization, vitreous hemorrhage, or tractional retinal detachment visible at the screening assessments in the study eye.
15. Only 1 functional eye, even if that eye was otherwise eligible for the study (e.g., BCVA of counting fingers or less in the eye with worse vision).
16. Ocular conditions with poorer prognosis in the fellow eye.
17. Inability to obtain fundus photographs, fluorescein angiography (FA), or OCT (e.g., due to media opacity, allergy to fluorescein dye or lack of venous access) in the study eye.
18. Any prior systemic anti-VEGF administration.
19. Uncontrolled diabetes mellitus in the opinion of the investigator.
20. Uncontrolled BP (defined as systolic >140 mm Hg or diastolic >90 mm Hg). Patients may be treated with up to 3 agents known to have anti-hypertensive effects for arterial hypertension to achieve adequate BP control. This limit applies to drugs that could be used to treat hypertension even if their primary indication in the patient was not for BP control. Any recent changes in medications known to affect BP need to be stable for 90 days prior to the screening visit.
21. Variation by more than 10% in the 3 pre-randomization BP measures recorded at the screening 1, screening 2, and randomization visits.
22. History of cerebrovascular accident/ transient ischemic attack or myocardial infarction/ acute coronary syndrome within 180 days of screening visit.
23. Renal failure, dialysis, or history of renal transplant.
24. Known sensitivity to any of the compounds of the study formulation.
25. Members of the clinical site study team and/or his/her immediate family, unless prior approval granted by the sponsor.
26. Pregnant or breastfeeding women
27. Women of childbearing potential* who are unwilling to practice highly effective contraception prior to the initial dose/start of the first treatment, during the study, and for at least 90 days after the last dose. Highly effective contraceptive measures include:

a. stable use of combined (estrogen and progestogen containing) hormonal contraception (oral, intravaginal, transdermal) or progestogen-only hormonal contraception (oral, injectable, implantable) associated with inhibition of ovulation initiated 2 or more menstrual cycles prior to screening.

b. intrauterine device (IUD); intrauterine hormone-releasing system (IUS)

c. bilateral tubal ligation

d. vasectomized partner

e. and or sexual abstinence†, ‡

*Postmenopausal women must be amenorrhoeic for at least 12 months in order not to be considered of childbearing potential. Pregnancy testing and contraception are not required for women with documented hysterectomy or tubal ligation.

†Sexual abstinence is considered a highly effective method only if defined as refraining from heterosexual intercourse during the entire period of risk associated with the study treatments.

‡Periodic abstinence (calendar, symptothermal, post-ovulation methods), withdrawal (coitus interruptus), spermicides only, and lactational amenorrhea method (LAM) are not acceptable methods of contraception. Female condom and male condom should not be used together.

28. Participation in an investigational study within 30 days prior to screening visit that involved treatment with any drug (excluding vitamins and minerals) or device.

29. Any other intraocular surgery within 12 weeks (84 days) before the screening visit (see Exclusion Criterion #9).

30. History of corneal transplant or corneal dystrophy in study eye.

31. Any concurrent ocular condition in the study eye which, in the opinion of the investigator, could either increase the risk to the patient beyond what is to be expected from standard procedures of IVT injections, or which otherwise may interfere with the injection procedure or with evaluation of efficacy or safety.

32. History of other disease, metabolic dysfunction, physical examination finding, or clinical laboratory finding giving reasonable suspicion of a disease or condition that contraindicates the use of an investigational drug or that might affect interpretation of the results of the study or render the patient at high risk for treatment complications.

Additional Exclusion Criteria for the Dense PK Sub-study

1. Prior IAI in the fellow eye

2. Patients on more than 2 anti-hypertensive medications

3. Patients with known cardiac arrhythmia

4. Patients who, in the opinion of the investigator, are unlikely to have stable BP over the course of the study (e.g., due to known non-compliance with medication)

Investigational and Reference Treatments

The HD will be provided as a liquid formulation in a vial. The target concentration of aflibercept is 114.3 mg/mL. The dose will be delivered in an injection volume of 70 microliters. The IAI will be provided as a liquid formulation in a vial. The target concentration of aflibercept is 40 mg/mL. The dose will be delivered in an injection volume of 50 microliters.

Additional Treatment

Deviation from the treatment schedule defined in the protocol is discouraged. Efforts should be made to ensure adherence to the protocol-specified dosing intervals. If, however, in the investigator's judgement, a patient cannot adhere to the protocol-specified dosing interval due to persistent or worsening disease and requires an interim injection, the patient may receive additional treatment at week 16. The investigator must make reasonable efforts to consult with the study director or sponsor designee prior to additional treatment being allowed.

Patients will receive their randomized dose of aflibercept if it is determined that additional treatment will be administered. Patients who receive additional treatment will continue to receive their randomized treatment at future visits and will remain masked to treatment assignment. Data from patients receiving additional treatment will be censored from the time additional treatment is administered.

Dose Modification

Dose modification for an individual patient is not allowed.

Concomitant Medications

Any treatment administered from the time of informed consent to the final study visit will be considered concomitant medication. This includes medications that were started before the study and are ongoing during the study.
If a pretreatment concomitant medication is administered in the study eye before injection (e.g., antibiotic or anesthetic), it must be administered for fellow eye treatment as well.

Prohibited Medications

Patients are not allowed to receive any standard or investigational treatment for nAMD in the study eye other than their assigned study treatment with HD or IAI, as specified in the protocol. This includes medications administered locally (e.g., IVT, topical, juxtascleral, or periorbital routes), as well as those administered systemically, with the intent of treating nAMD in the study eye or fellow eye.
If the fellow eye has nAMD, or any other approved indication, IAI (2 mg) will be allowed and supplied through the IWRS (Interactive web response system). Patients are not allowed to receive any other anti-VEGF agent in the fellow eye. Patients enrolled in the dense PK sub-study cannot receive IAI (2 mg) in the fellow eye before week 12.
Non-ocular (systemic) standard or investigational treatments for nAMD of the study or fellow eye are not permitted. Systemic anti-angiogenic agents and anti-Ang2 inhibitors are not permitted during the study.

Permitted Medications

Any other medications that are considered necessary for the patient's welfare, and that are not expected to interfere with the evaluation of the study drug, are allowed.

## Table 1-2. Clinical Study Schedule of Events

| Study Procedure | Screening Visit 1 | Screening Visit 2 | Baseline Visit 3 | Visit 4 | Visit 5 | Visit 6 | Visit 7 | Visit 8 | Visit 9 | Visit 10 | Visit 11 | Visit 12 | Visit 13 | Visit 14 | EOS Visit 15 |
|---|---|---|---|---|---|---|---|---|---|---|---|---|---|---|---|
| Month | | | 0 | 1 | 2 | - | 3 | 4 | 5 | 6 | 7 | 8 | 9 | 10 | 11 |
| Week | | | 0 | 4 | 8 | ~9 | 12 | 16 | 20 | 24 | 28 | 32 | 36 | 40 | 44 |
| Day | -21 to -1 | -20 to -1 | 1 | 29 | 57 | 61 | 85 | 113 | 141 | 169 | 197 | 225 | 253 | 281 | 309 |
| Window (day) | | | | ±5[1] | ±5[1] | ±2 | ±5[2] | ±5[2] | ±5[2] | ±5[2] | ±5 | ±5[2] | ±5 | ±5 | ±5[2] |
| **Screening/Baseline:** | | | | | | | | | | | | | | | |
| Informed consent | X | | | | | | | | | | | | | | |
| Dense PK sampling informed consent[3] | X | | | | | | | | | | | | | | |
| Genomic substudy/ FBR Substudy informed consent form[4] | X | | | | | | | | | | | | | | |
| Inclusion/Exclusion | X | | X | | | | | | | | | | | | |
| Medical history | X | | | | | | | | | | | | | | |
| Demographics | X | | | | | | | | | | | | | | |
| Concomitant medications | X | X | X | X | X | X | X | X | X | X | X | X | X | X | X |
| Randomization | | | X | | | | | | | | | | | | |
| **Administer Study Drug[5]** | | | | | | | | | | | | | | | |
| Study drug | | | X | X | X | | | | X | X PRN[6] | X PRN[6] | X | X PRN[6] | X PRN[6] | |
| **Ocular Efficacy and Safety (bilateral unless indicated):** | | | | | | | | | | | | | | | |
| Refraction and BCVA (ETDRS)[7] | X | | X | X | X | | X | X | X | X | X | X | X | X | X |
| IOP[8] | X | | X | X | X | | X | X | X | X | X | X | X | X | X |
| Slit lamp examination | X | | X | X | X | | X | X | X | X | X | X | X | X | X |
| Indirect ophthalmoscopy[9] | X | | X | X | X | | X | X | X | X | X | X | X | X | X |
| FA, FP[10] | X | | | | | | X | | X | | | | | | X |
| SD-OCT[10] | X | | X | X | X | | X | X | X | X | X | X | X | X | X |
| **Non-ocular Safety:** | | | | | | | | | | | | | | | |
| Physical examination | X | | | | | | | | | | | | | | |
| Vital signs[11, 12, 13] | X | X[14] | X | X | X | X[14] | X | X | X | X | X | X | X | X | X |
| ECG | X | | | | | | | | | | | | | | X |
| Adverse events | X | | X | X | X | X | X | X | X | X | X | X | X | X | X |
| **Laboratory Testing[15]:** | | | | | | | | | | | | | | | |
| Hematology | X | | | | | | X | | | | | | | | X |

| Study Procedure | Screening Visit 1 | Screening Visit 2 | Baseline Visit 3 | Visit 4 | Visit 5 | Visit 6 | Visit 7 | Visit 8 | Visit 9 | Visit 10 | Visit 11 | Visit 12 | Visit 13 | Visit 14 | EOS Visit 15 |
|---|---|---|---|---|---|---|---|---|---|---|---|---|---|---|---|
| Month | | | 0 | 1 | 2 | - | 3 | 4 | 5 | 6 | 7 | 8 | 9 | 10 | 11 |
| Week | | | 0 | 4 | 8 | ~9 | 12 | 16 | 20 | 24 | 28 | 32 | 36 | 40 | 44 |
| Day | -21 to -1 | -20 to -1 | 1 | 29 | 57 | 61 | 85 | 113 | 141 | 169 | 197 | 225 | 253 | 281 | 309 |
| Blood chemistry | X | | | | | | X | | | | | | | | X |
| Pregnancy test (women of childbearing potential)[16] | X Serum | | X Urine | X Urine | X Urine | | | X Urine | X Urine | X Urine | X Urine | X Urine | X Urine | | |
| Urinalysis/UPCR[17] | X | | X[15] | | | | X | | | | | | | | X |
| **Pharmacokinetic and Research Sampling** | | | | | | | | | | | | | | | |
| PK samples (dense)[18] | | | See schedule below | X | X | X | X | X | X | X | | X | | | X |
| PK samples (sparse)[20] | | | X | | X | X | X | | | X | | X | | | X |
| Research sample[21] | | | X | | | | | | | | | | | | X |
| Genomic DNA sample (optional)[4] | | | X | | | | | | | | | | | | |

BCVA=Best Corrected Visual Acuity, ECG=electrocardiogram, EOS=end of study, ETDRS=Early Treatment Diabetic Retinopathy Study, FA=fluorescein angiography, FBR=future biomedical research, FP=fundus photography, IOP=Intraocular pressure, PK=pharmacokinetics, PRN=*pro re nata* (as needed), SDOCT= spectral domain optical coherence tomography, UPCR=urine protein:creatinine ratio.

Footnotes:

1. For patients in the dense PK sub-study, the visit window is ± 0 days.
2. For patients in the dense PK sub-study, the visit window is ± 2 days.
3. Signed only by patients participating in the dense PK sub-study and in addition to the study ICF.
4. The optional genomic and FBR sub-study ICF (informed consent form) should be presented to patients at the screening visit and may be signed at any subsequent visit at which the patient chooses to participate after screening. The genomic DNA sample should be collected on day 1/baseline (pre-dose) or at any study visit from patients who have signed the sub-study ICF.
5. Refer to pharmacy manual for study drug injection guidelines. Following study drug injection, patients will be observed for approximately 30 minutes.

6. Patients will be dosed as needed per criteria herein.

7. Patients enrolled at sites participating in the optional visual function sub-study may undergo additional visual function tests. See study procedure manual for details.

8. Intraocular pressure will be measured bilaterally at all study visits. On days when study drug is administered, IOP should also be measured approximately 30 minutes after administration of study drug, in the study eye only. Intraocular pressure will be measured using Goldman applanation tonometry or Tono-pen™ and the same method of measurement must be used in each patient throughout the study.

9. Indirect ophthalmoscopy should be performed bilaterally at all visits. On days when study drug is administered, it should also be performed immediately after administration of study drug (study eye only).

10. The same SD-OCT/FA/FP imaging system used at screening and day 1 must be used at all subsequent visits in each patient. Images will be taken in both eyes before dosing at each required visit.

11. Vital signs (temperature, BP, heart rate) should be measured pre-injection, per the procedure outlined in the study procedure manual. Blood pressure assessments will be taken using automated office blood pressure (AOBP) with the Omron Model HEM 907XL (or comparable). Measures will be taken in triplicate and a mean measure as displayed by the device will be recorded in the EDC. Detailed instructions can be found in the study procedure manual.

12. Timing of BP assessment at all visits must be within 2 hours of planned time of dosing on day 1 for patients in the dense PK sub-study. For all other patients, this window should be adhered to as closely as possible.

13. For patients participating in the dense PK sub-study, HR and BP also will be collected according to the schedule in Table 1-2.

14. Only BP and heart rate will be measured at these visits. No temperature measures are required.

15. All samples collected for laboratory assessments should be obtained prior to administration of fluorescein and prior to administration of study drug.

16. For women of childbearing potential, a negative serum pregnancy test at screening is required for eligibility. A negative urine pregnancy test is required before treatment is administered at subsequent visits.

17. For patients participating in the dense PK sub-study, urinalysis/UPCR will also be collected according to the schedule in Table 1-2.

18. Week 4 collection of urinalysis/UPCR only for patients in the dense PK sub-study.

19. Dense PK sampling will be performed in approximately 30 patients (15 in each group) drawn according to the schedule in Table 1-2. On dosing days, BP (blood pressure) and PK samples must be collected prior to study drug administration. Additional patients (up to approximately 50% more in each treatment group) may be enrolled in the dense PK sub-study to ensure adequate data is are captured.

20. Sparse PK sampling will be performed in all patients not enrolled in the dense PK sub-study according to the schedule defined in Table 1-2. On dosing days, PK samples should be collected prior to study drug administration.

21. Exploratory research serum sample should be drawn prior to the administration of study drug at baseline (visit 3, day 1) and week 44.

**Table 1-3. Dense PK Study Schedule of Events**

| Visit | Dose | Assessment Day and Time (h) | | Dense PK Sample Collection | Heart Rate and Blood Pressure[1,2] | Urinalysis / UPCR |
|---|---|---|---|---|---|---|
| Visit 3 (Baseline) | X | 1 | Time of first dose | X (pre-dose) | X | |
| | | | 4h post-dose ($\pm30$ mm)[3] | X | | |
| | | | 8h post-dose ($\pm2$ h)[4] | X | | |
| | | 2 | $\pm2h$[5] | X | X | |
| | | 3 | $\pm2h$[5] | X | X | |
| | | 5 | $\pm2h$[5] | X | X | |
| | | 8 | $\pm2h$[5] | X | X | X[6] |
| | | 15 | $\pm2h$[5] | X | X | X[6] |
| | | 22 | $\pm2h$[5] | X | X | |

Footnotes:

1. Timing of all BP assessments must be within ±2 hours of the time of dosing on day 1. This may be done at the clinical study site or by the site personnel or another healthcare professional at a remote location (*e.g.*, the patient's home or other appropriate location). Regardless of where BP measurements are taken, the procedures described herein must be followed.

2. Blood pressure assessments will be taken using automated office blood pressure (AOBP) with the Omron Model HEM 907XL (or comparable). Measures will be taken in triplicate and a mean measure as displayed by the device will be recorded in the EDC. Detailed instructions can be found in the study procedure manual.

3. Intraocular pressure will be measured at approximately 4 hours post-dose only if the IOP measurement from approximately 30 minutes to 60 minutes post-dose remains clinically significantly higher than the pre-injection reading.

4. Intraocular pressure will be measured at approximately 8 hours post-dose only if the IOP measurements from approximately 30 minutes to 60 minutes and approximately 4 hours post-dose remain clinically significantly higher than the pre-injection reading.

5. PK draw for all assessment days are to be performed within ±2 hours of the time of dosing on day 1.

6. This may be done at the clinical study site or by the site personnel or another healthcare professional at a remote location (*e.g.,* the patient's home or other appropriate location).

Ocular Procedures

Intraocular Pressure. Intraocular pressure will be measured in both eyes at every visit using Goldmann applanation tonometry or Tono pen™, as specified in Table 1-2. The same method of IOP measurement must be used throughout the study for each individual patient. On dosing visits, IOP will also be measured approximately 30 minutes post-dose (study eye).

For patients in the dense PK sub-study, IOP will also be measured 4 hours post-dose if the reading from approximately 30 minutes to 60 minutes post-dose remains clinically significantly higher than the pre-dose reading, and again at approximately 8 hours post-dose if the reading from approximately 4 hours post-dose remains clinically significantly higher than the pre-dose reading.

Slit Lamp Examination. Patients' anterior eye structure and ocular adnexa will be examined bilaterally pre-dose at each study visit using a slit lamp (see study procedure manual) by the investigator, as specified herein

Indirect Ophthalmoscopy. Patients' posterior pole and peripheral retina will be examined by indirect ophthalmoscopy at each study visit pre-dose (bilateral) and post-dose (study eye) by the investigator, as specified herein. Post-dose evaluation must be performed immediately after injection.

Fundus Photography/Fluorescein Angiography. The anatomical state of the retinal vasculature will be evaluated by FP and FA as specified herein. Fundus photography and FA will be captured and transmitted to an independent reading center for both eyes. For FA, the study eye will be the transit eye.

Fundus and angiographic images will be sent to an independent reading center where images will be read by masked readers. All FPs and FAs will be archived at the site as part of the source documentation. Photographers must be certified by the reading center to ensure consistency and quality in image acquisition. A detailed protocol for image acquisition and transmission can be found in the study procedure manual. Imaging technicians should remain masked to treatment assignment.

Spectral Domain Optical Coherence Tomography. Retinal characteristics will be evaluated at every visit using SD-OCT. Images will be captured and transmitted for both eyes. Images will be sent to an independent reading center where they will be read by masked readers. All OCTs will be electronically archived at the study site as part of the source documentation. Optical coherence tomography technicians must be certified by the reading center to ensure consistency and quality in image acquisition. A detailed protocol for acceptable OCT machines and OCT image acquisition/transmission can be found in the study procedure manual. Imaging technicians should remain masked to treatment assignment.

Best Corrected Visual Acuity. Visual function of the study eye and the fellow eye will be assessed using the ETDRS protocol (Early Treatment Diabetic Retinopathy Study Research Group, 1985) at 4 meters at each study visit, as specified in Table 1-2. Visual acuity examiners must be certified to ensure consistent measurement of BCVA, and must remain masked to treatment assignment, treatment schedule and study eye. Best corrected visual acuity should be done before any other ocular procedures are performed. Patients enrolled at sites participating in the optional visual function sub-study may undergo additional visual function tests.

Definitions

*Adverse Event.* An AE is any untoward medical occurrence in a patient administered a study drug which may or may not have a causal relationship with the study drug. Therefore, an AE is any unfavorable an unintended sign (including abnormal laboratory finding), symptom, or disease which is temporally associated with the use of a study drug, whether or not considered related to the study drug (ICH E2A Guideline. Clinical Safety Data Management: Definitions and Standards for Expedited Reporting, Oct 1994).

*Serious Adverse Event.* An SAE is any untoward medical occurrence that at any dose:

- Results in death - includes all deaths, even those that appear to be completely unrelated to study drug (*e.g.,* a car accident in which a patient is a passenger). Is life-threatening - in the view of the investigator, the patient is at immediate risk of death at the time of the event. This does not include an AE that had it occurred in a more severe form, might have caused death.
- Requires in-patient hospitalization or prolongation of existing hospitalization. Inpatient hospitalization is defined as a hospital admission (any duration) or an emergency room visit for longer than 24 hours. Prolongation of existing hospitalization is defined as a hospital stay that is longer than was originally anticipated for the event or is prolonged due to the development of a new AE as determined by the investigator or treating physician

- Results in persistent or significant disability/incapacity (substantial disruption of one's ability to conduct normal life functions).
- Is a congenital anomaly/birth defect
- Is an important medical event - Important medical events may not be immediately life-threatening or result in death or hospitalization, but may jeopardize the patient or may require intervention to prevent one of the other serious outcomes listed above (*e.g.,* intensive treatment in an emergency room or at home for allergic bronchospasm; blood dyscrasias or convulsions that do not result in hospitalization; or development of drug dependency or drug abuse).

*Ocular important medical event.* An ocular important medical event may include the following:

- An AE that requires either surgical or medical intervention to prevent permanent loss of vision;
- Substantial, unexplained vision loss or an AE that causes substantial vision loss Criteria for reporting SAEs must be followed for these events.

*Severity.* The severity of AEs will be graded according to the following scale:

- Mild: Does not interfere in a significant manner with the patient normal functioning level. It may be an annoyance. Prescription drugs are not ordinarily needed for relief of symptoms, but may be given because of personality of the patient.
- Moderate: Produces some impairment of functioning but is not hazardous to health. It is uncomfortable or an embarrassment. Treatment for symptom may be needed.
- Severe: Produces significant impairment of functioning or incapacitation and is a definite hazard to the patient's health. Treatment for symptom may be given and/or patient hospitalized. If a laboratory value is considered an AE, its severity should be based on the degree of physiological impairment the value indicates.

*Causality.* The investigator must provide causality assessment as whether or not there is a reasonable possibility that the drug caused the adverse event, based on evidence or facts, his/her clinical judgment, and the following definitions. The causality assessment must be made based on the available information and can be updated as new information becomes available.

The following factors should be considered when assessing causality:

- Temporal relationship: time to onset vs time drug was administered;
- Nature of the reactions: immediate vs. long term;
- Clinical and pathological features of the events;
- Existing information about the drug & same class of drugs;
- Concomitant medications;
- Underlying and concurrent illnesses;
- Response to de-challenge (drug discontinuation) or dose reduction;
- Response to re-challenge (re-introduction of the drug) or dose increase, when applicable;
- Patient's medical and social history.

*Causality to the Study Drug.*

- Related: The AE follows a reasonable temporal sequence from study drug administration, and cannot be reasonably explained by the nature of the reaction, patient's clinical (e.g., disease under study, concurrent diseases, concomitant medications), or other external factors; or the AE follows a reasonable temporal sequence from study drug administration, and is a known reaction to the drug under study or its class of drugs, or is predicted by known pharmacology.

- Not Related: The AE does not follow a reasonable sequence from study drug administration, or can be reasonably explained by the nature of the reaction, patient's clinical state (*e.g.,* disease under study, concurrent diseases, and concomitant medications) or other external factors.

*Causality to the Injection Procedure.* The relationship of AEs to the injection procedure is assessed by the investigator, and is a clinical decision based on all available information. The following question is addressed: Is there a reasonable possibility that the AE may have been caused by the injection procedure? The possible answers are:

- Not Related: There is a reasonable possibility that the event may have been caused by the injection procedure
- Related: There is a reasonable possibility that the event may have been caused by the injection procedure *Causality to the Study Conduct (Protocol-Specified Procedure).*
- Related: The AE follows a reasonable temporal sequence from a protocol-specified procedure, and cannot be reasonably explained by the nature of the reaction, patient's clinical (*e.g.,* disease under study, concurrent diseases, concomitant medications), or other external factors.
- Not Related: The AE does not follow a reasonable sequence from a protocol-specified procedure, or can be reasonably explained by the nature of the reaction, patient's clinical state (*e.g.,* disease under study, concurrent diseases, and concomitant medications) or other external factors

Analysis of Drug Concentration Data-Main Study

The concentrations of free and bound aflibercept over time will be summarized by descriptive statistics for each treatment group. No formal statistical hypothesis testing will be performed.

Dense PK Sub-study

The PK parameters to be determined after the first dose for free and bound aflibercept may include, but are not limited to:

- $C_{max}$;
- $C_{max}$/Dose;
- $t_{max}$;
- $t_{last}$;
- $C_{last}$;
- $AUC_{inf}$;
- $AUC_{inf}$/Dose;
- $t_{1/2}$;
- $C_{trough}$;

After repeat dosing in the dense PK sub-study, PK parameters to be determined may include, but are not limited to, $C_{trough}$, time to reach steady-state, and accumulation ratio. The concentrations of free and bound aflibercept over time and selected PK parameters will be summarized by descriptive statistics by treatment group. This descriptive statistical assessment will include the geometric means and ratios of the geometric means for selected PK parameters, as deemed appropriate. No formal statistical hypothesis testing will be performed.

Results at week 16 (n=55)-values as reported at the time for patients reaching week 16.

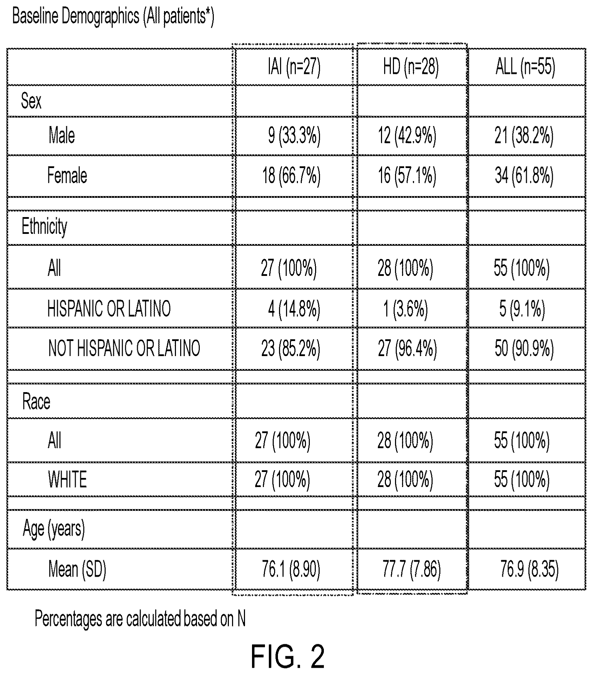

The baseline demographics, eye characteristics, and blood pressure of "All patients" at this point in this trial are set forth in Figures 2-4.

After 16 weeks of study duration, patients receiving the 8 mg doses of aflibercept (HD dosing regimen) maintained (on average) greater vision improvements and anatomical improvements than that of patients receiving the 2 mg dose (IAI dosing regimen) (relative to baseline).

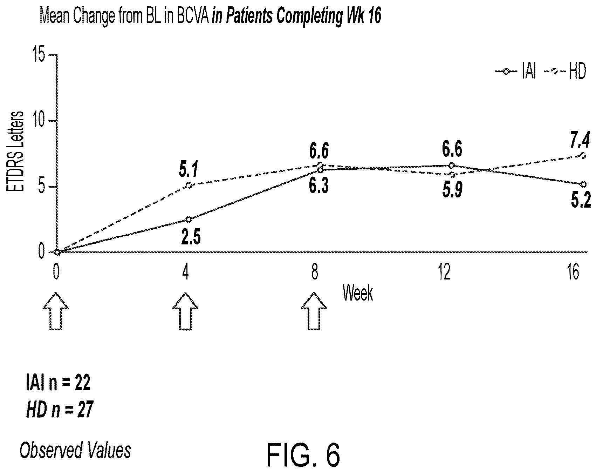

Patients (Completers) receiving the HD dosing regimen maintained a greater mean change in best corrected visual acuity (7.4) than the IAI patients (5.2) at week 16 (Figure 6).

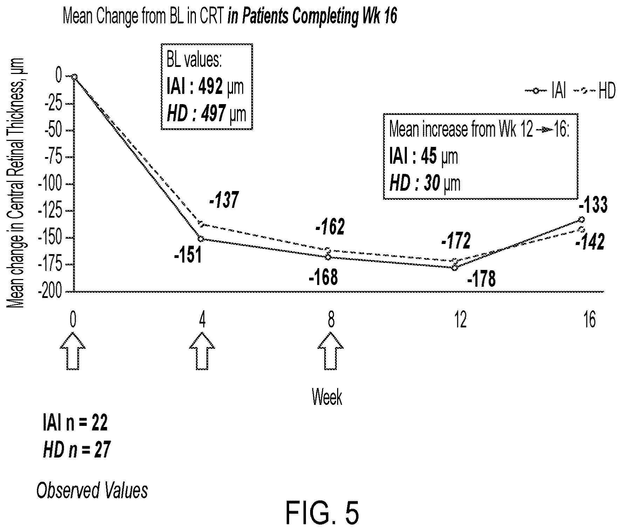

Anatomical improvements were also remarkable at week 16. The central retinal thickness (CRT) of patients receiving the HD dosing regimen remained below that of patients (Completers) receiving the IAI dosing regimen-a mean change of -142 micrometers in HD patients vs -133 micrometers in IAI patients at week 16 (Figure 5).

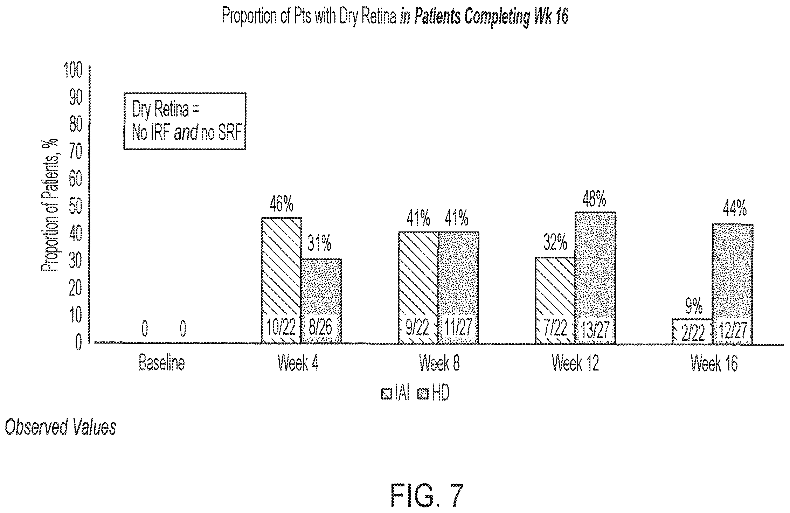

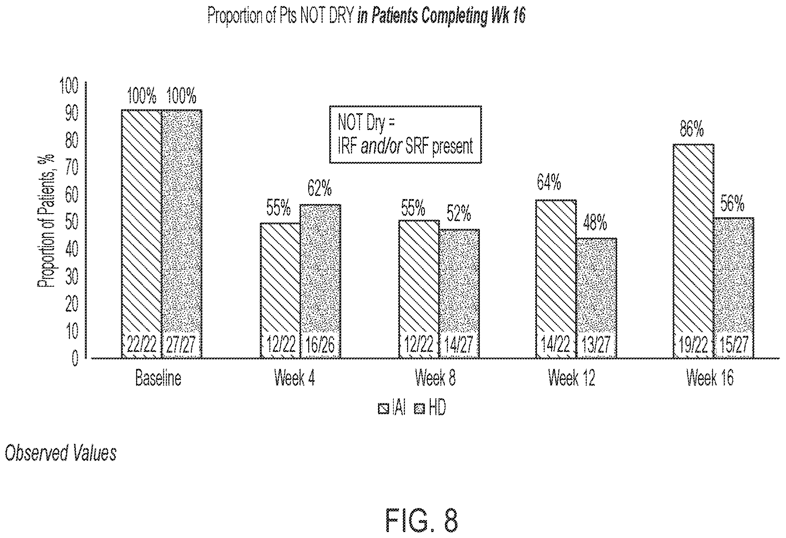

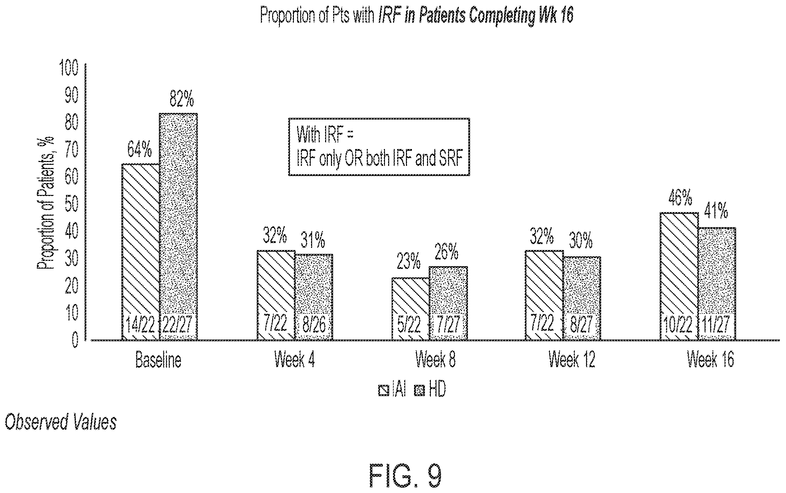

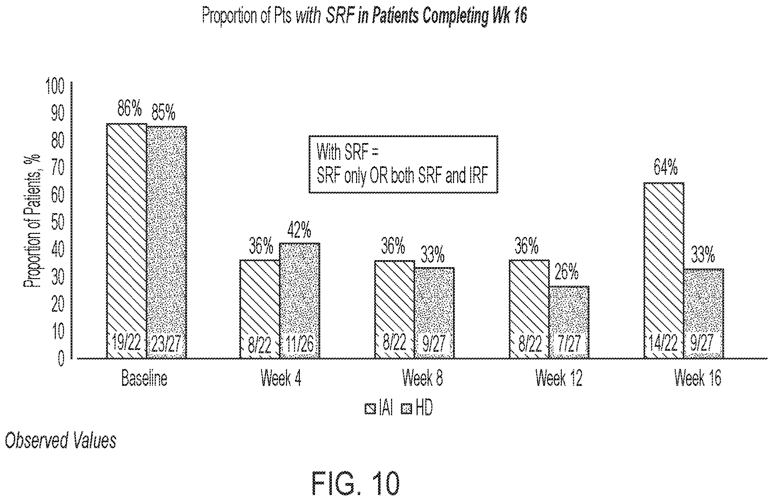

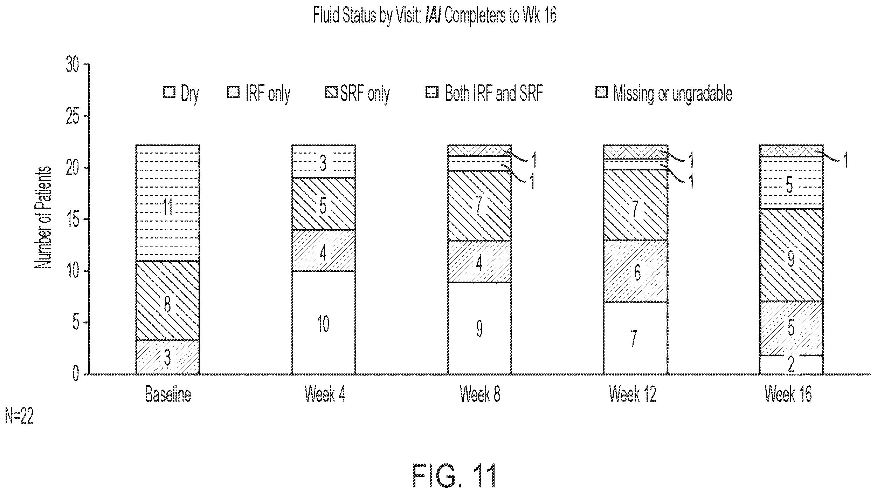

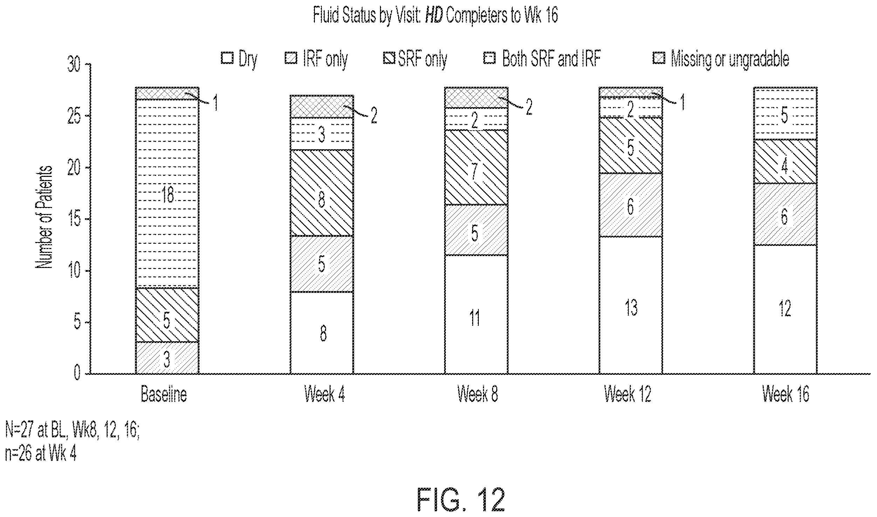

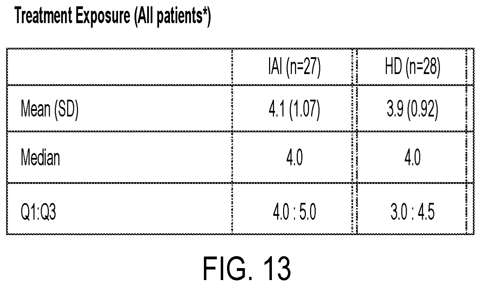

Measures of retinal dryness were also strikingly better in patients receiving the HD dosing regimen. Overall, more patients (Completers) receiving the HD regimen had complete resolution of intraretinal/subretinal fluid at week 16. The proportion of HD patients with dry retinas at week 16 was 44% whereas the proportion of IAI patients with dry retinas was just 9% (Figure 7). A dry retina was regarded as a retina exhibiting no intraretinal fluid (IRF) and subretinal fluid (SRF). Conversely, a greater proportion of patients receiving the IAI dosing regimen had "not-dry" retinas at week 16 than that of HD patients (86% vs 56%, respectively) (Figure 8). Fewer HD patients exhibited IRF (IRF only or both IRF and SRF) or SRF (SRF only or both IRF and SRF) at week 16 than IAI patients (Figures 9-10, respectively). Similar trends were apparent when the retinal fluid status (Dry, IRF only, SRF only or both IRF and SRF) of IAI and HD patients at baseline, week 4, week 8, week 12 and week 16 was observed (Figures 11-12, respectively). The number of treatments in the HD and IAI treatment groups ("All patients") was comparable (Figure 13).

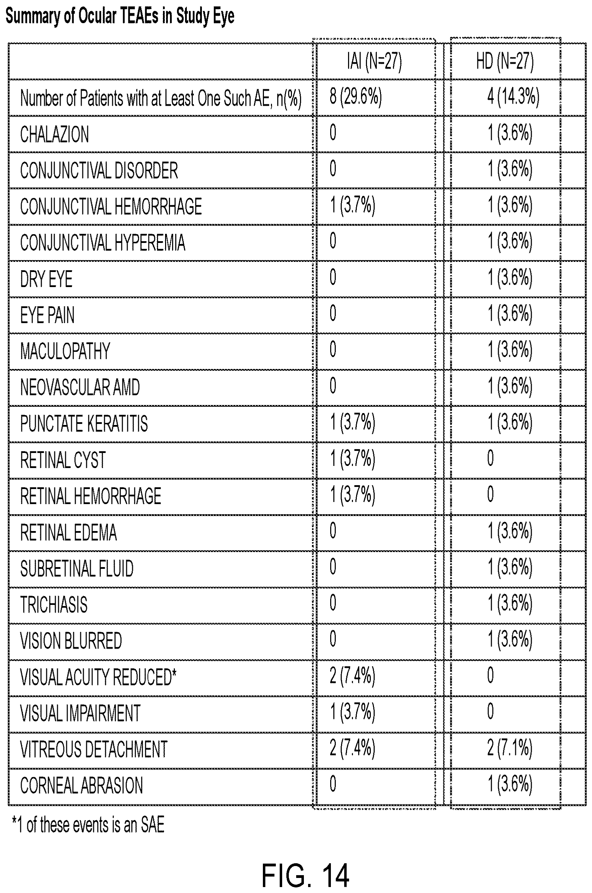

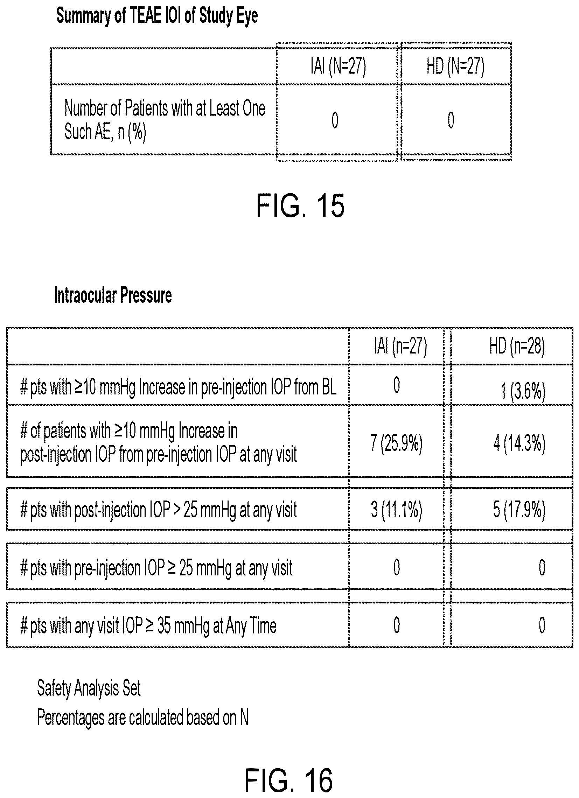

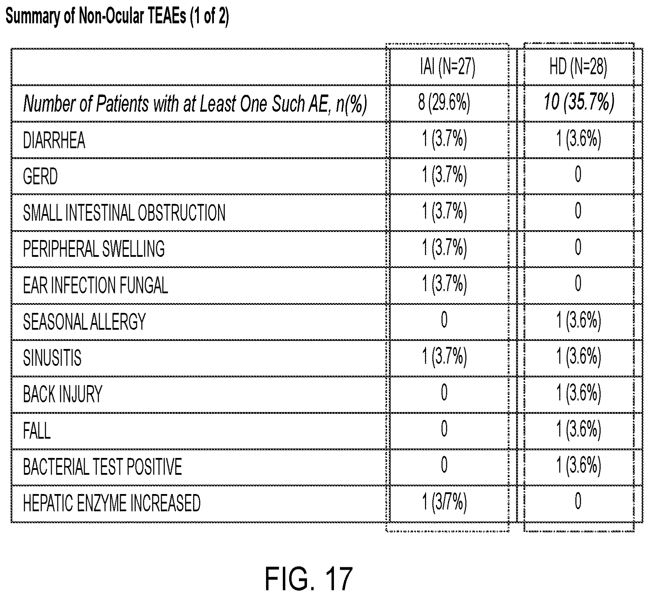

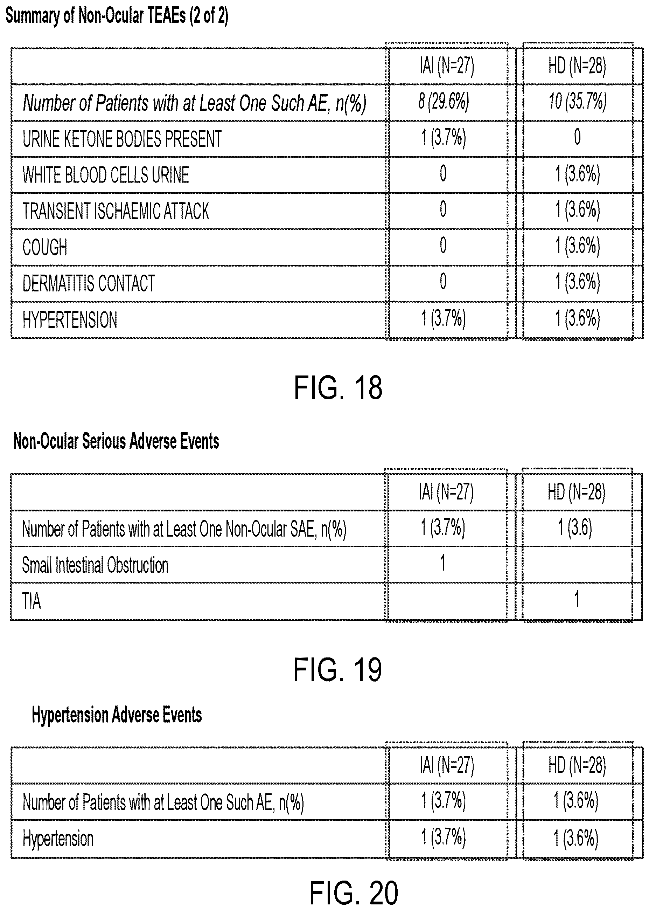

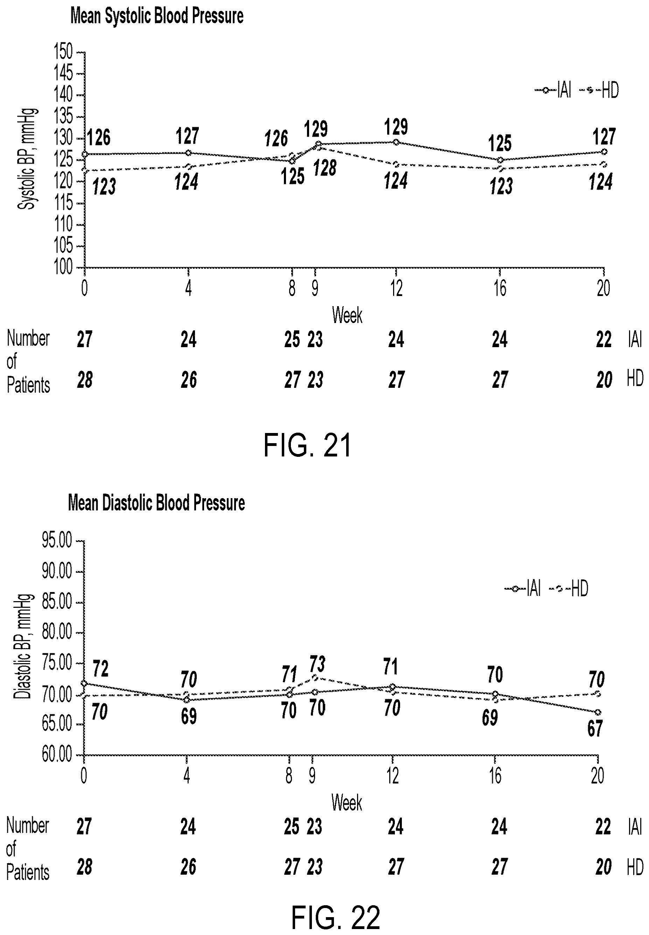

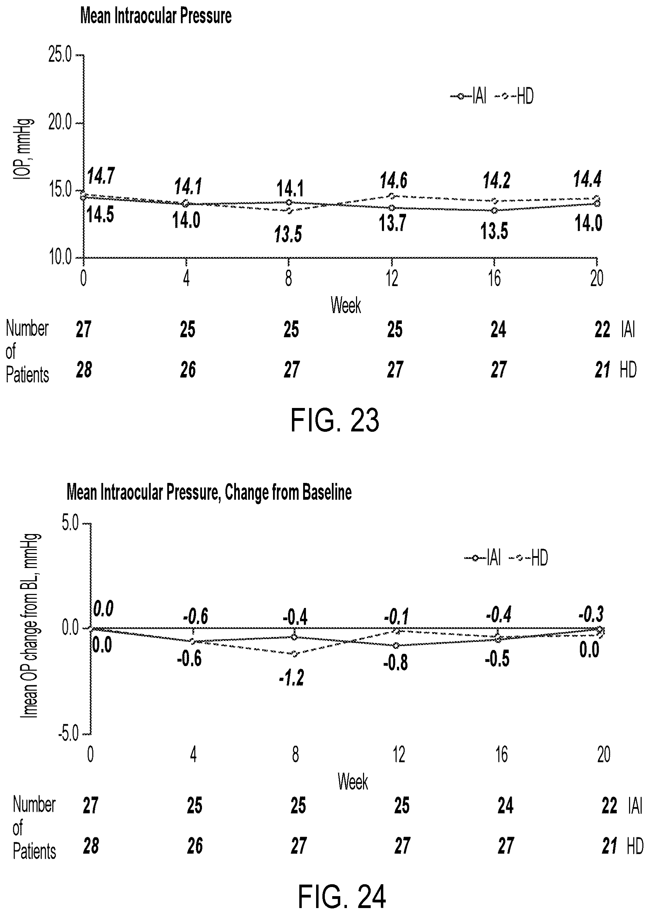

The HD and IAI dosing regimens were generally well tolerated. The ocular treatment-emergent adverse events (TEAEs) (Figure 14-15), intraocular pressure (IOP) (Figure 16), non-ocular TEAEs (Figures 17-18), non-ocular SAEs (Figure 19), hypertension AEs (Figure 20), mean systolic blood pressure (Figure 21), mean diastolic blood pressure (Figure 22), mean IOP (Figure 23), mean change of IOP (Figure 24) were comparable in each treatment group.

Results- additional patients reaching week 16 (n=106) -values as reported at the time for a larger set of patients reaching week 16

At week 16, a higher proportion of these patients in the HD aflibercept 8 mg group had no retinal or subretinal fluid (50.9%, n=27/53) in the center (1 mm) subfield on optical coherence tomography compared to patients treated with IAI EYLEA 2 mg (34.0%, n=18/53) (p=0.08). During the initial 16 weeks of the trial, treatment emergent adverse events (TEAEs) in the study eye occurred in 17.0% (9 of 53) of aflibercept 8 mg patients and 22.6% (12 of 53) of EYLEA 2 mg patients. AEs that occurred more frequently in the aflibercept 8 mg group were conjunctival hemorrhage (5.7% aflibercept 8 mg, 3.8% EYLEA 2 mg) and vitreous detachment (3.8% aflibercept 8 mg, 1.9% EYLEA 2 mg). Serious ocular AEs (SAEs) occurred in two patients overall, one in the aflibercept 8 mg group (retinal tear) and one in the EYLEA 2 mg group (visual acuity reduced). No intraocular inflammation, occlusive vasculitis, arterial thromboembolic events (adjudicated according to the AntiPlatelet Trialists' Collaboration definitions) or death in either patient group were identified through week 16.

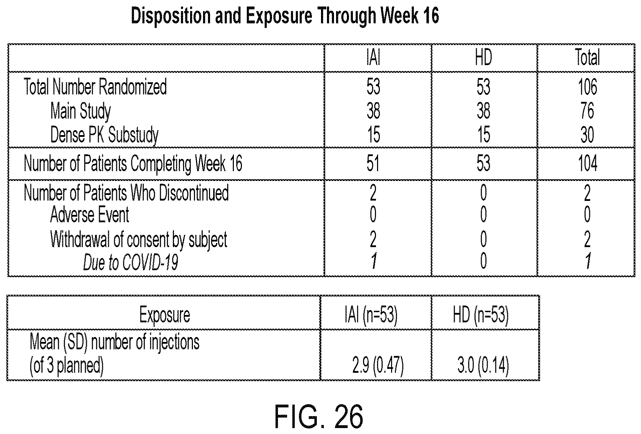

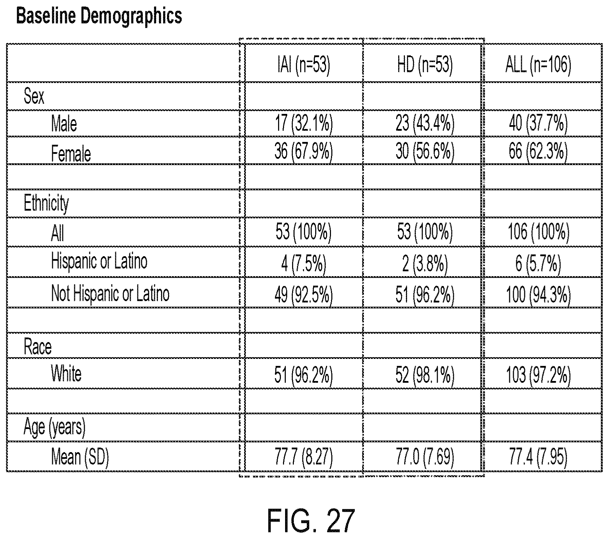

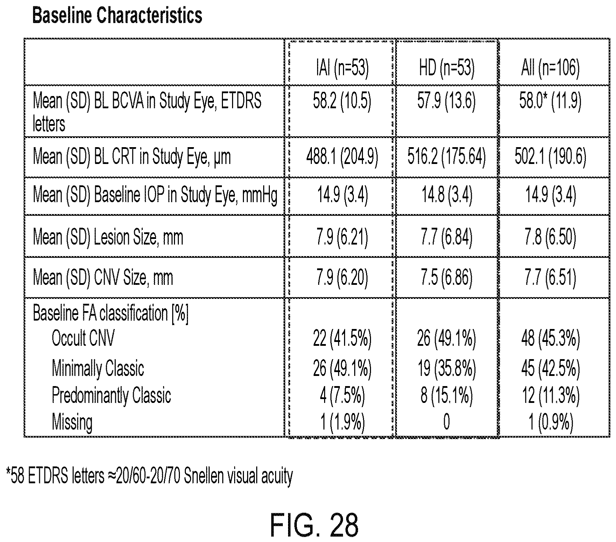

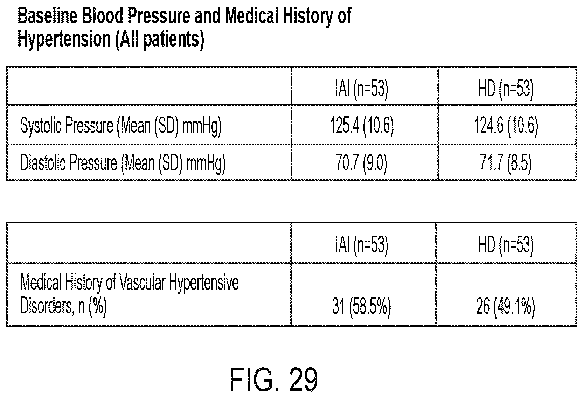

The disposition and exposure, baseline demographics, baseline characteristics, and baseline blood pressure and medical history of patients in this analysis are set forth in Figures 26, 27, 28 and 29.

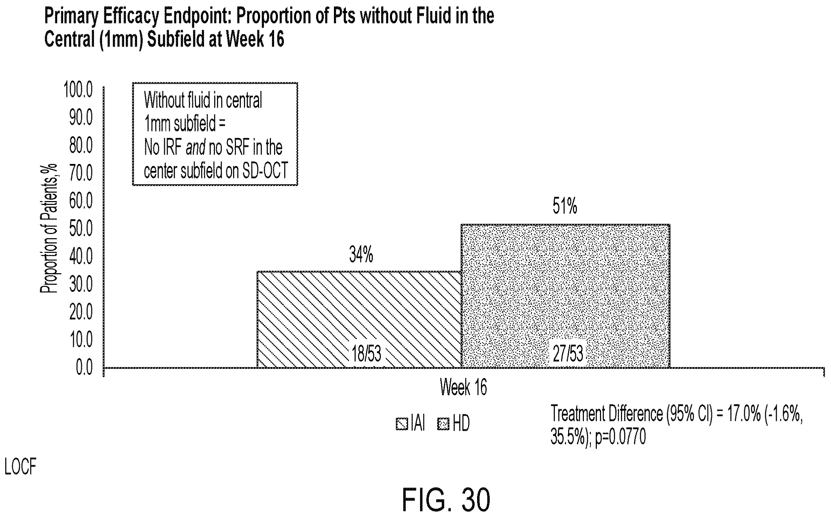

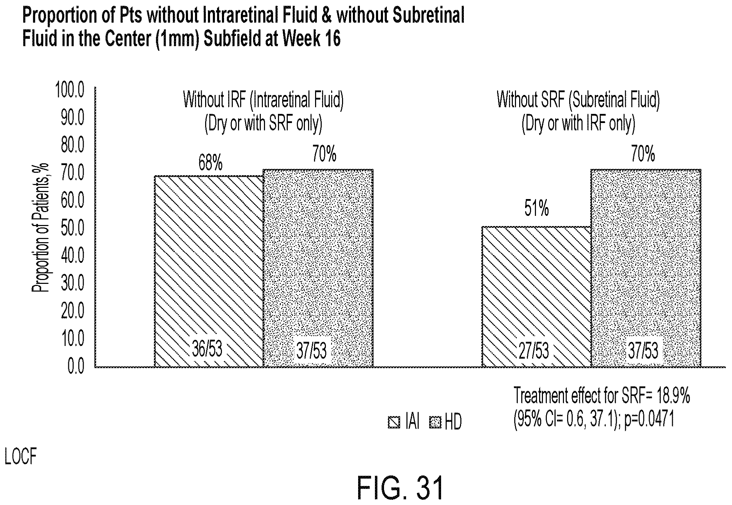

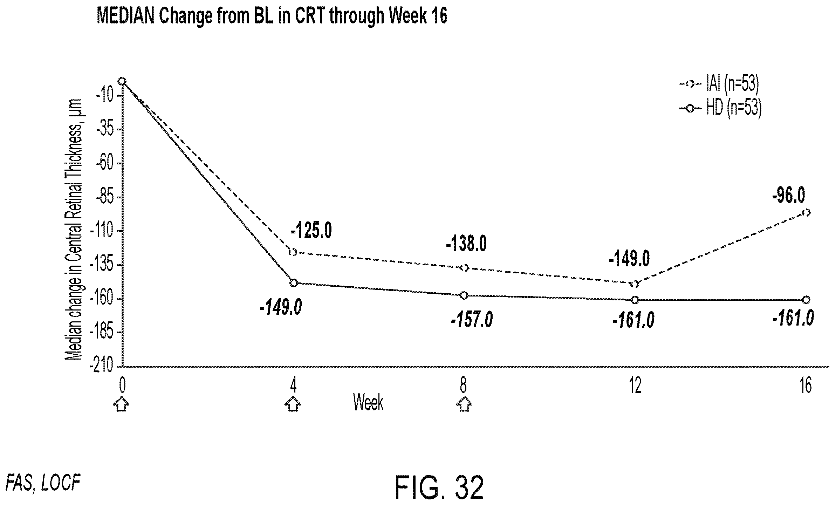

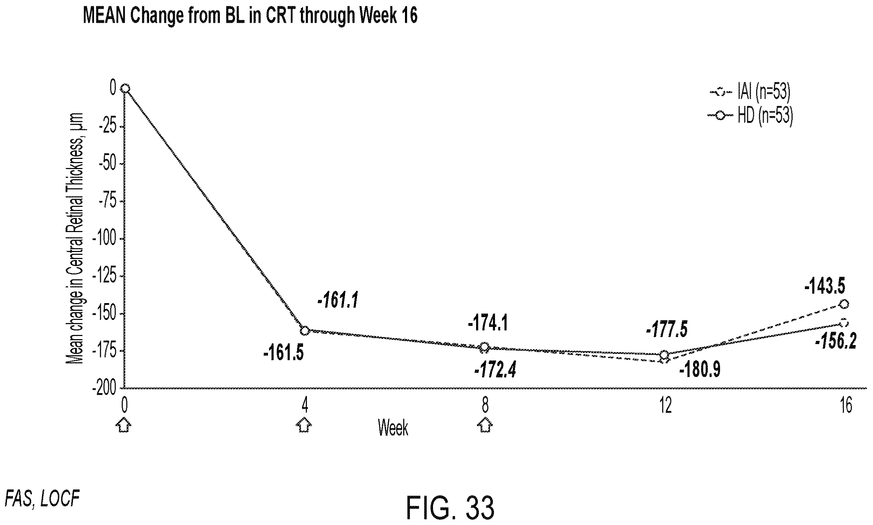

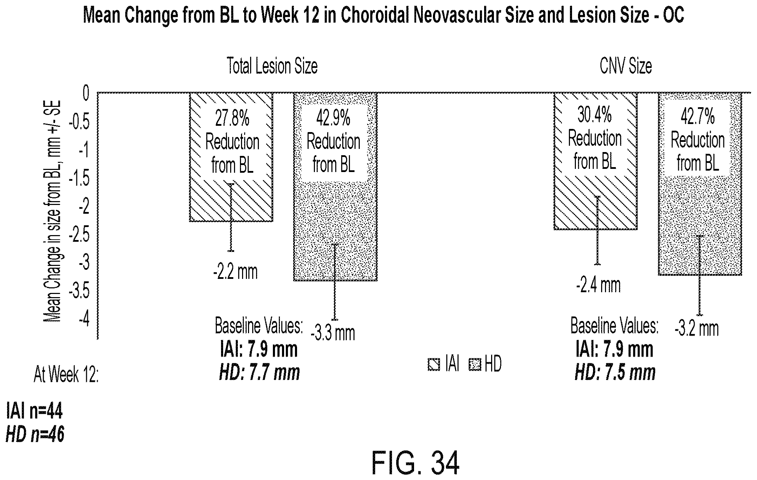

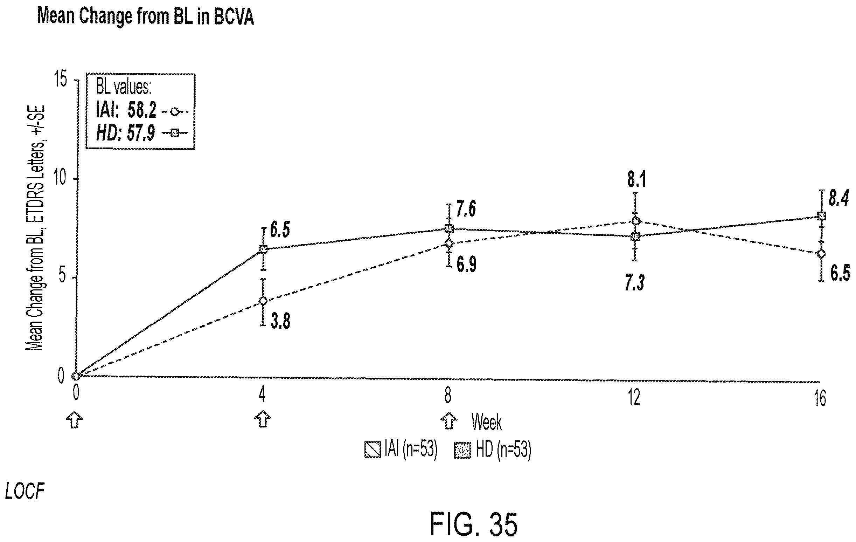

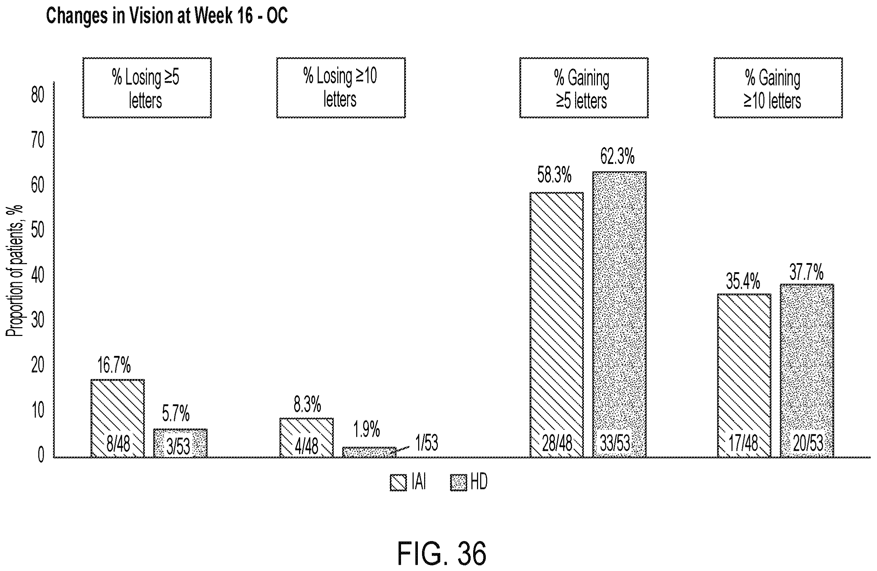

The proportion of HD patients achieving a dry retina (no IRF and no SRF in the central subfield on SD-OCT (spectral domain optical coherence tomography)) was 51% whereas the proportion of IAI patients was 34% (Figure 30) (last observation carried forward (LOCF) values). HD patients without IRF (dry or with SRF only) was 70% (IAI patients: 68%) and without SRF (dry or with IRF only) was 70% (IAI patients: 51%) (Figure 31) (LOCF). At week 16, there was a greater median (Figure 32) and mean (Figure 33) decrease in central retinal thickness from baseline in HD patients (-161.0 micrometers, - 156.2 micrometers, respectively) relative to IAI patients (-96.0 micrometers, -143.5 micrometers, respectively) (LOCF). The mean change from baseline to week 12 in choroidal neovascularization and total lesion size is set forth in Figure 34 showing greater reductions in the HD group relative to the IAI group. Regarding best corrected visual acuity (BCVA), HD patients achieved a mean change, from baseline through week 16, of 8.4 (ETDRS letters) whereas IAI patients achieved 6.5 (Figure 35) (LOCF). A smaller proportion of HD patients lost letters than IAI patients, and a greater proportion of HD patients gained letters than IAI patients at week 16 (Figure 36).

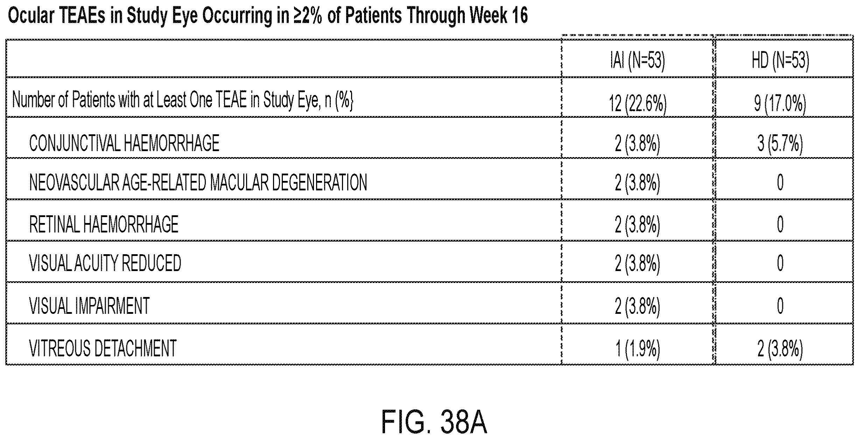

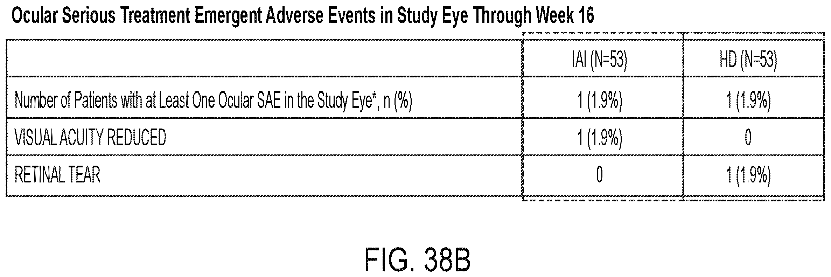

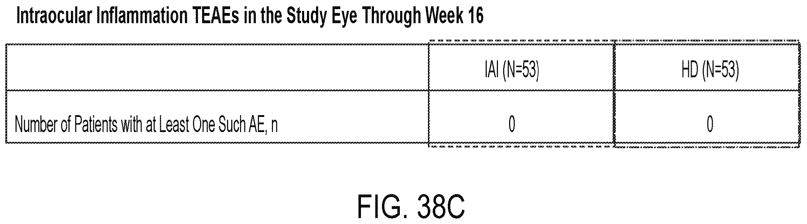

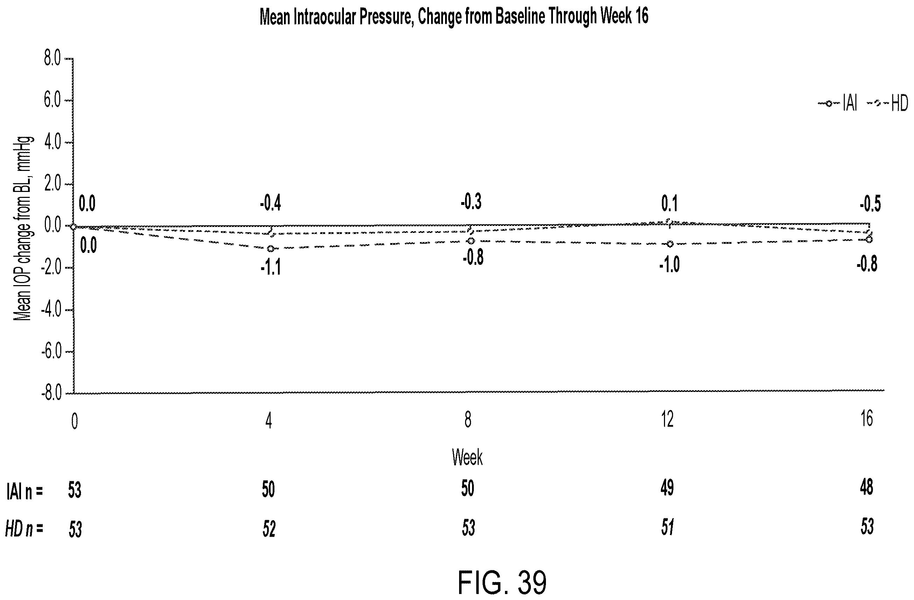

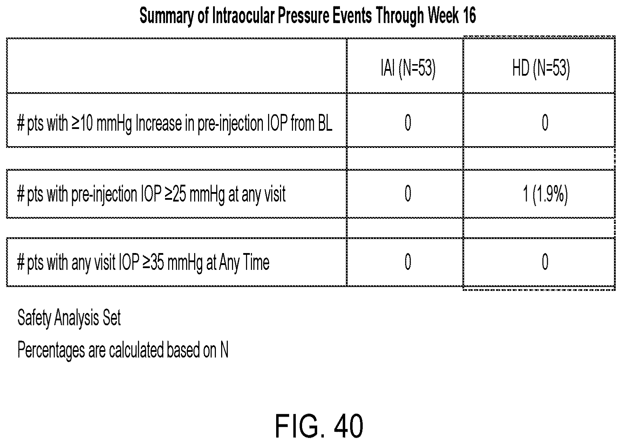

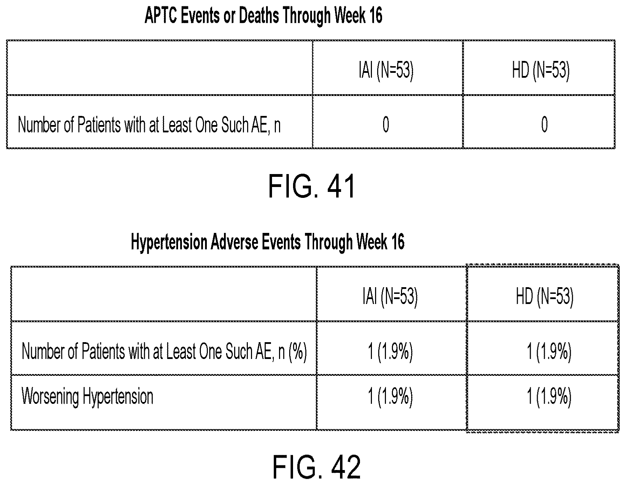

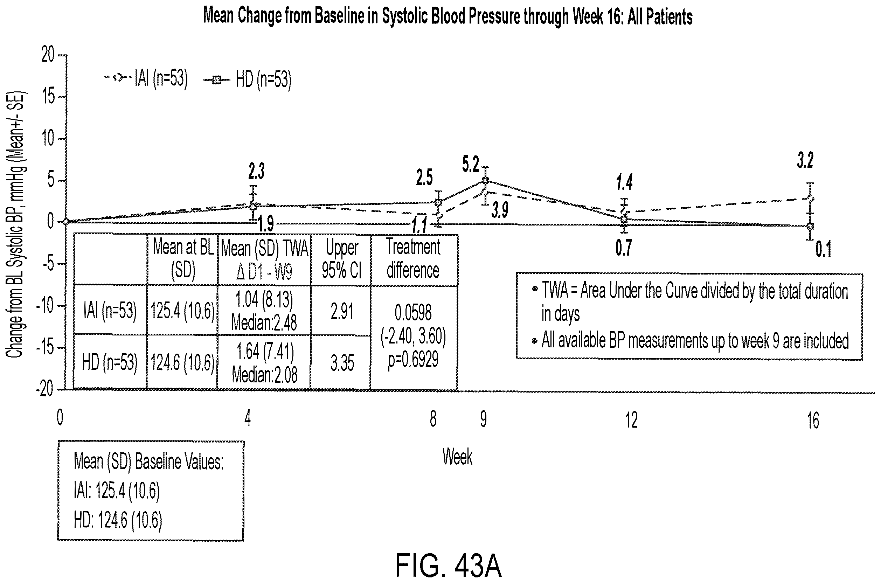

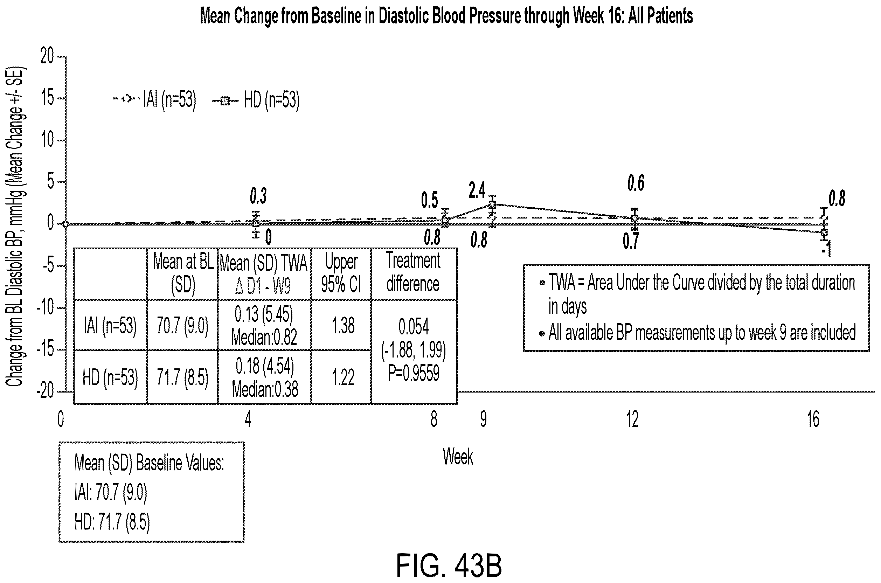

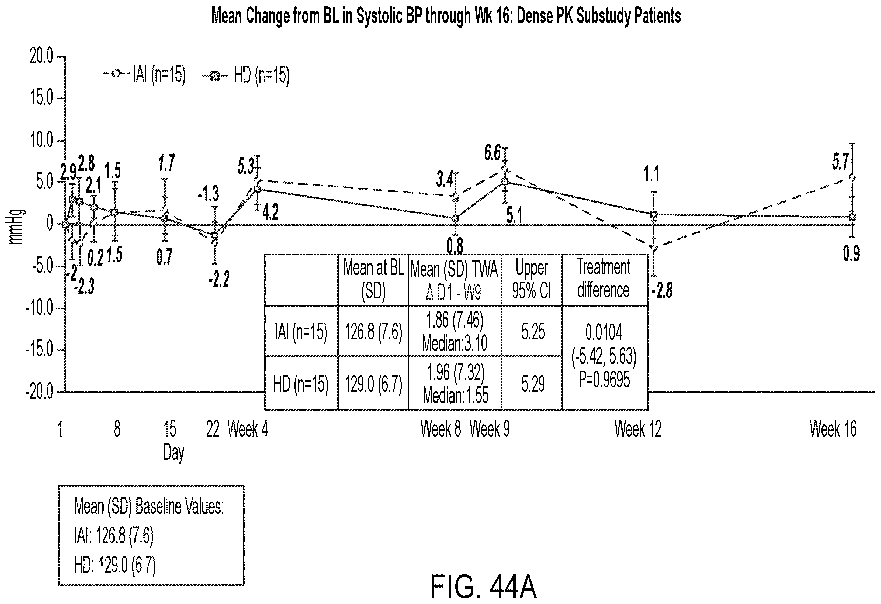

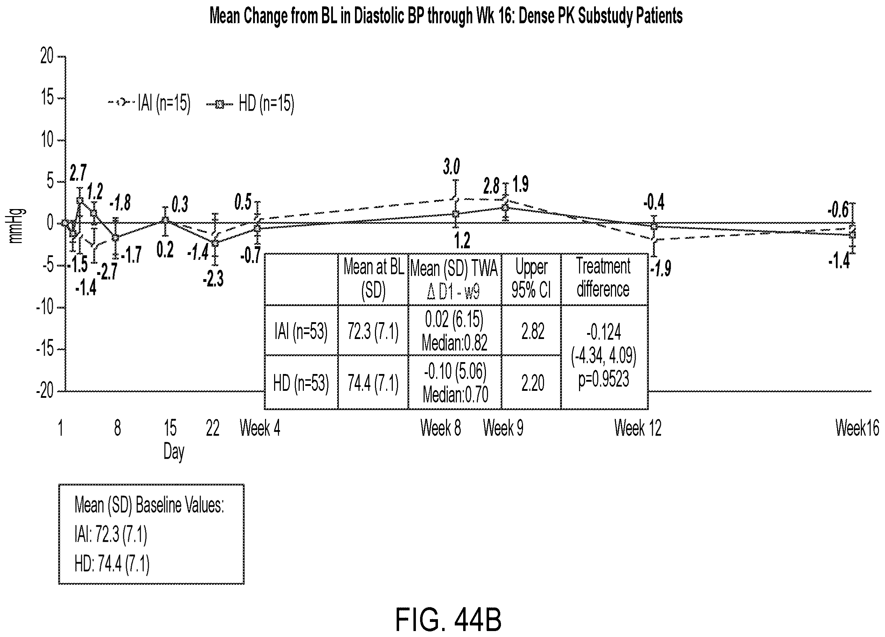

The proportion of HD patients receiving additional treatment at week 16 (19%) was smaller than that of IAI patients (27%). Also, the occurrence of ocular TEAEs through week 16 was 17% among HD patients and 22.6% among IAI patients (Figure 38(A)). The occurrence of ocular serious treatment emergent adverse events and intraocular inflammation TEAEs among HD and IAI patients, through week 16, was comparable (Figure 38(B), Figure 38(C)). Mean intraocular pressure change from baseline, occurrence of intraocular pressure events, occurrence of APTC events or deaths, hypertension adverse events among HD and IAI patients was comparable at week 16 (Figure 39, Figure 40, Figure 41, Figure 42). Mean changes in blood pressure (systolic or diastolic) through week 16 among all HD and IAI patients or patients in a dense PK sub-study was comparable (Figure 43 (A-B), Figure 44(A-B)).

Results- patients reaching week 44 (n=100) -values as reported at the time for patients reaching week 44

The study ended at week 44 with 100 patients. With an identical dosing regimen and slightly fewer rescue and/or PRN doses, a higher proportion of eyes receiving 8 mg aflibercept (HD) were dry in the center subfield relative to the eyes receiving 2 mg aflibercept (IAI). In addition, a change from baseline in central subfield thickness (CST) suggested better anatomic outcomes in the 8 mg HD group relative to the 2 mg IAI group. Changes in visual acuity from baseline favored the 8 mg dosing regimen (HD) over the 2 mg regimen (IAI) (+7.9 letters vs. +5.1 letters).

No new safety signals were seen and the safety profile for the HD group was comparable to that of IAI. There was one case of mild iritis in the HD group that resolved with topical therapy. Changes from baseline blood pressure and intraocular pressure were similar between the groups.

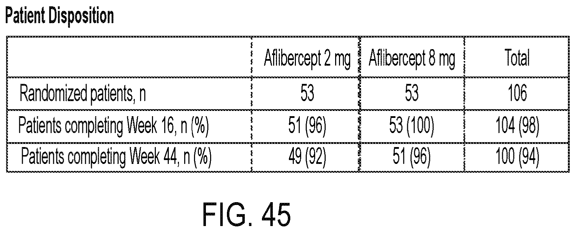

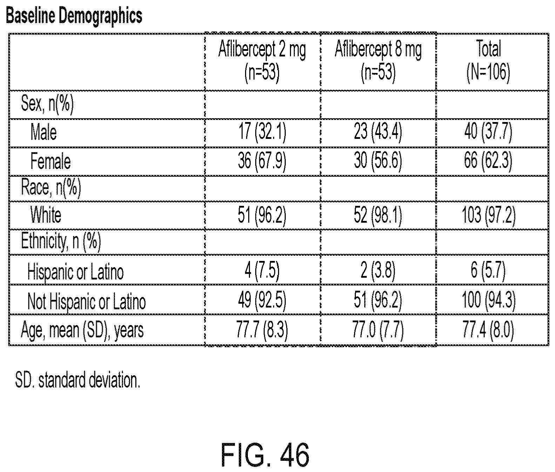

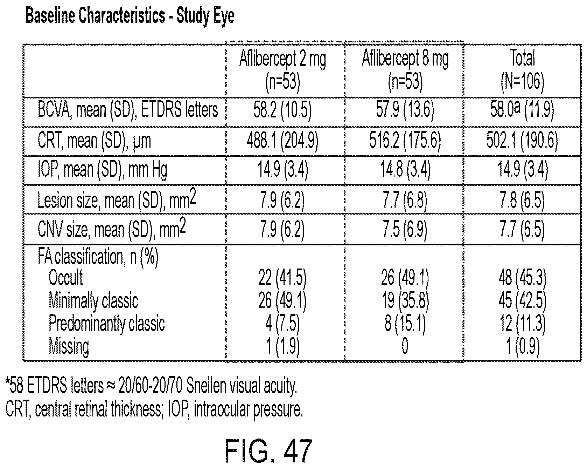

Out of the 106 patients that started in the study, 100 reached the 44-week point-49 in the IAI group and 51 in the HD group (Figure 45). The baseline demographics of the patients in the study were majority white and having more females than males with an average age of about 77 years as set forth in Figure 46. In addition, the baseline characteristics of the study eye in patients are set forth in Figure 47. The IAI and HD groups received the same mean number of injections (5.8) through week 44 (see Figure 48) with slightly more HD patients than IAI patients not receiving additional or PRN treatments.

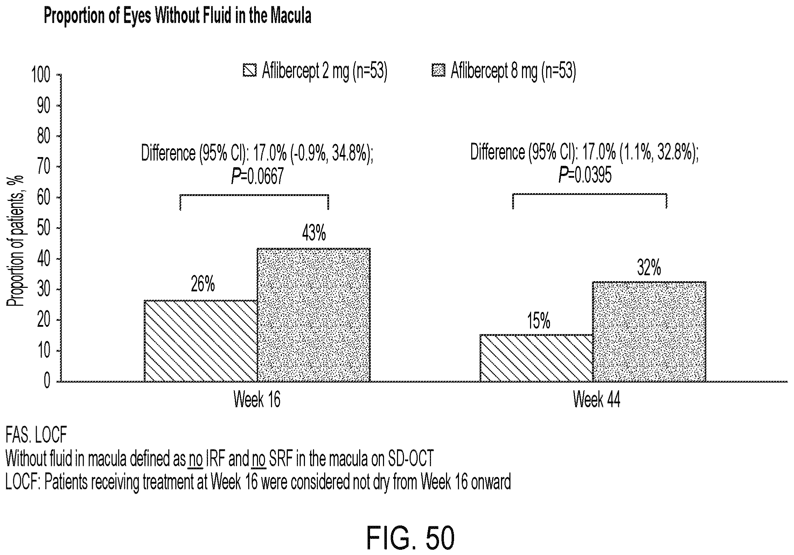

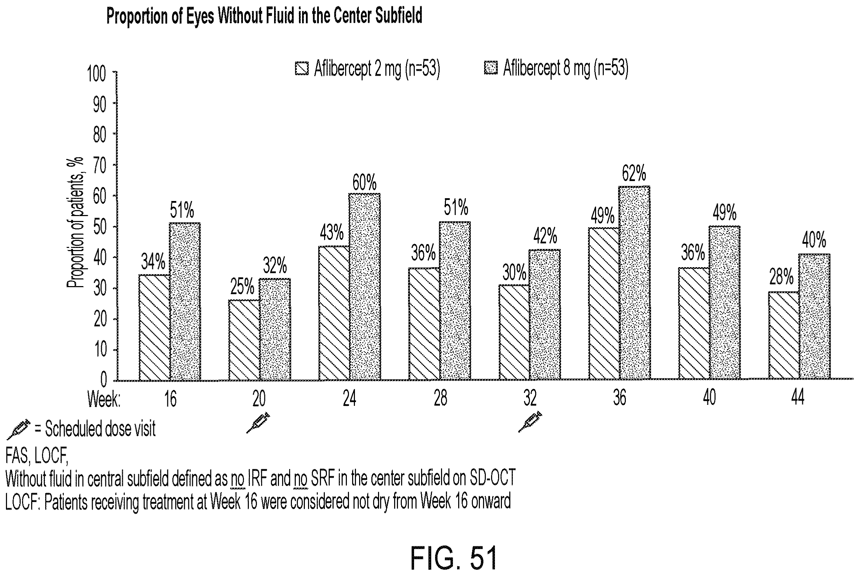

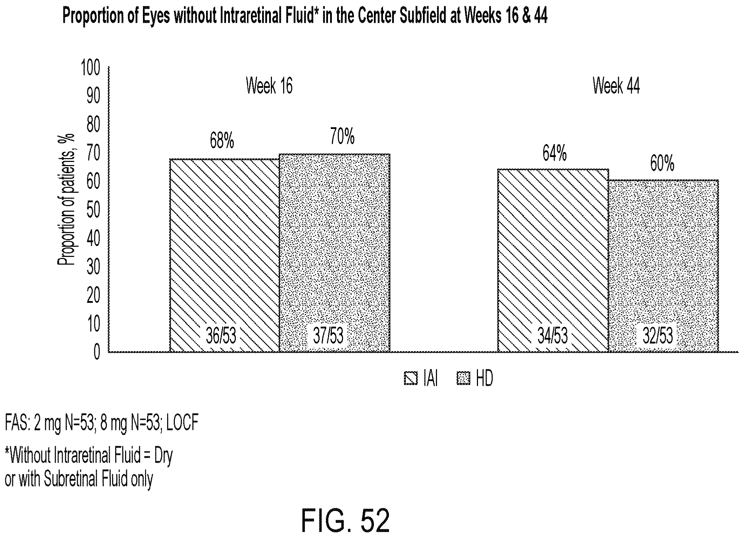

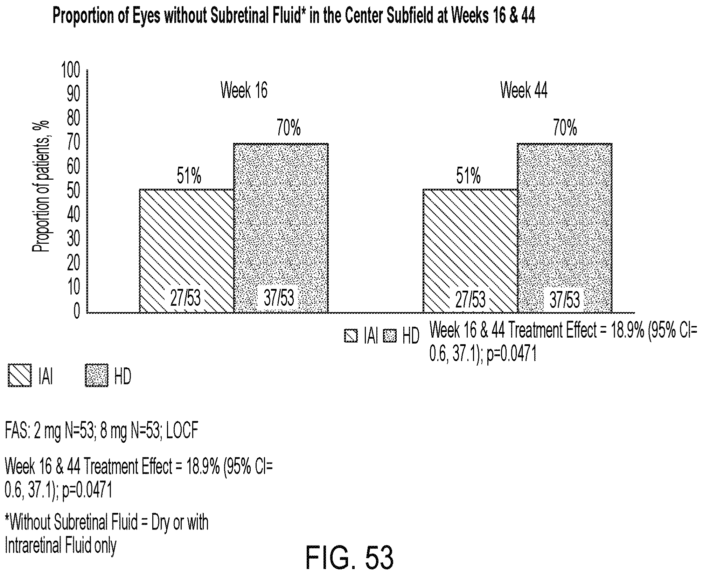

Patients in the HD group achieved numerically superior anatomical improvements in the eye. Retinal drying (lack of fluid in the center subfield-no intraretinal fluid (IRF) and no subretinal fluid (SRF)) at weeks 16 to 44 was higher in the HD group relative to that of the IAI group (Figure 49, Figure 51). Moreover, at weeks 16 and 44, there was a greater proportion of eyes, in the HD group than in the IAI group, without fluid in the macula (no IRF and no SRF in the macula by SD-OCT) (Figure 50). The proportion of patients in each group without IRF (dry or SRF only) at weeks 16 and 44 is set forth in Figure 52. At weeks 16 and 44, 70% of patients in the HD group exhibited no subretinal fluid (SRF) (dry or with IRF only) whereas 51% of the IAI group patients exhibited no SRF (Figure 53). Through much of the 44-week trial, the HD group achieved greater mean and median reductions in central retinal thickness (CRT) from baseline (Figure 54, Figure 55).

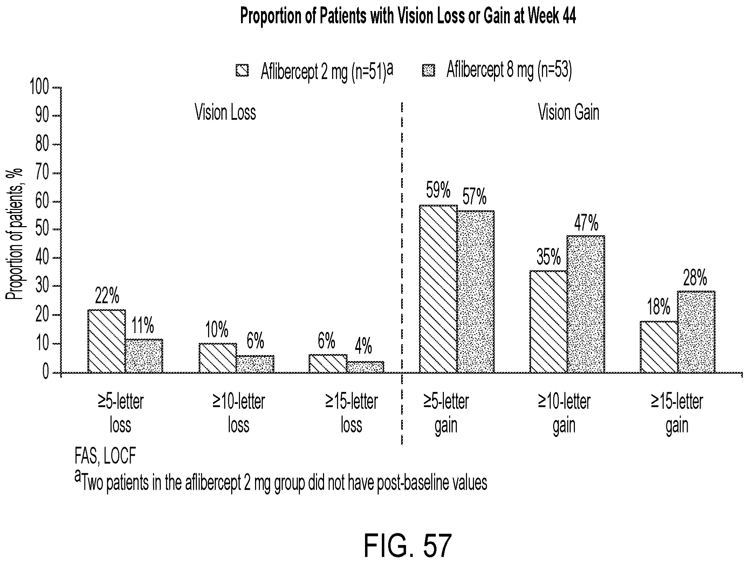

Patients in the HD group also achieved greater gains in vision. By week 44, the mean change, from baseline, in best corrected visual acuity (BCVA) was 7.9 in the HD group and 5.1 in the IAI group (Figure 56). Fewer patients in the HD group than in the IAI group lost vision ($\geq 5$, $\geq 10$ or $\geq 15$ letters lost) by week 44. Moreover, more patients in the HD group gained vision ($\geq 10$ or $\geq 15$ letters gained) (Figure 57) by week 44.

Ocular TEAEs and ocular serious TEAEs, intraocular inflammation TEAEs, intraocular pressure events, non-ocular TEAEs and non-ocular serious TEAEs were comparable between treatment groups (Figure 58, Figure 59 and Figure 60, Figure 61, Figure 63, Figure 64). Intraocular pressure (IOP) observed between treatment groups were also comparable (Figure 62). Moreover, the administration of the 8 mg dose in the HD group was not observed to have an effect on the occurrence of hypertension TEAEs (Figure 65). One patient died during the trial due to glioblastoma (Figure 66).

## Example 2: Characterization of Dose Accuracy

This Example documents the procedure and execution results of deliverable volume characterization testing conducted to compare different presentations of a formulation including aflibercept (REGN3) at a concentration of 114.3 mg/ml.

The following devices will be compared:

- **REGN3-PFS-0.5mL** (see International Design Registration. DM/212509 and International Patent Application Publication No. WO2020/247686) and

- **1mL BD (Becton Dickinson) Luer Lok syringe**.(a plastic disposable syringe that is currently used as part of the commercially available EYLEA Vial Kit presentation).
  PFS=pre-filled syringe

Testing Procedures

Testing was conducted separately for each presentation. All samples were filled with 114.3 mg/mL aflibercept formulated drug substance.

REGN3-PFS-0.5mL testing:

- Sample Size: n = 60
- PFS filled with 187 microliters and primed (air volume expelled) by hand
- A volume of 85-87 microliters was targeted to be delivered from the PFS
- Delivered sample volume was determined by weighing (discussed below)
- PFS was fitted with BD Hypoint 30G ½" needles

1mL BD Luer Lok Syringe testing:

- Sample Size: n = 310
- Syringe fitted with BD PrecisionGlide 30G ½" needles
- Sample was drawn up into the syringe samples from a beaker and manually primed
- A volume of 70 microliters was targeted to be delivered from the syringe

- Delivered sample volume was determined by weighing (discussed below)

<u>Results of Deliverable Volume Testing</u>

Deliverable volume was calculated by collecting the dose delivered through manual injection after the syringe was manually primed and weighing the collected dose on a balance. The collected mass was then divided by the density of the FDS (1.059 g/ml) to calculate the volume delivered.

$$V = (m_{dose}/\rho) * 1000,$$

where $V$ is the delivered volume ($\mu$L), $m_{dose}$ is the dose mass (g), and $\rho$ is the solution density (g/mL). Preconditioning of samples at 5°C was conducted for the testing in order to maintain drug product integrity.
REGN3-PFS-0.5mL:

- Mean delivered dose volume: 81.670 microliters
- Std Dev: 4.458
- Min: 64.684 microliters
- Max: 89.481 microliters

1mL BD (Becton Dickinson) Luer Lok syringe:

- Mean delivered dose volume: 75.740 microliters
- Std Dev: 8.665
- Min: 44.287 microliters
- Max: 97.828 microliters

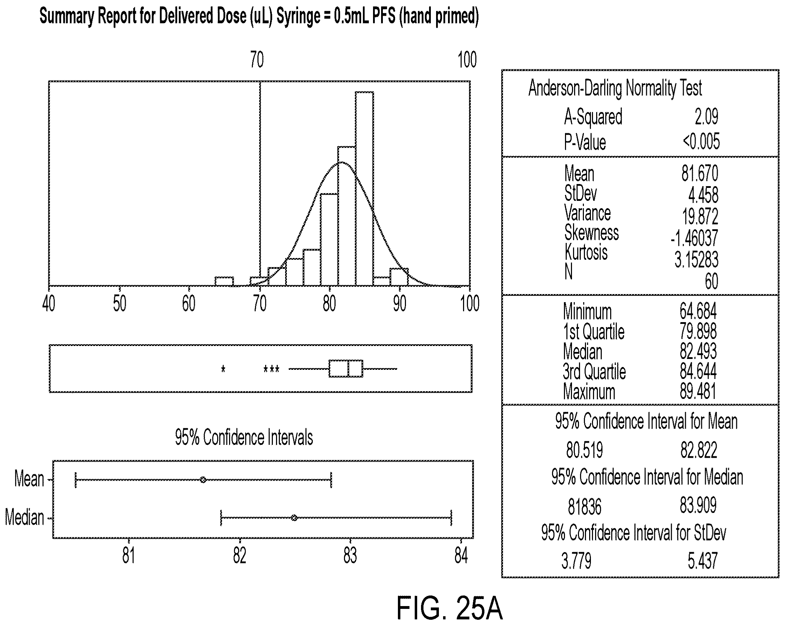

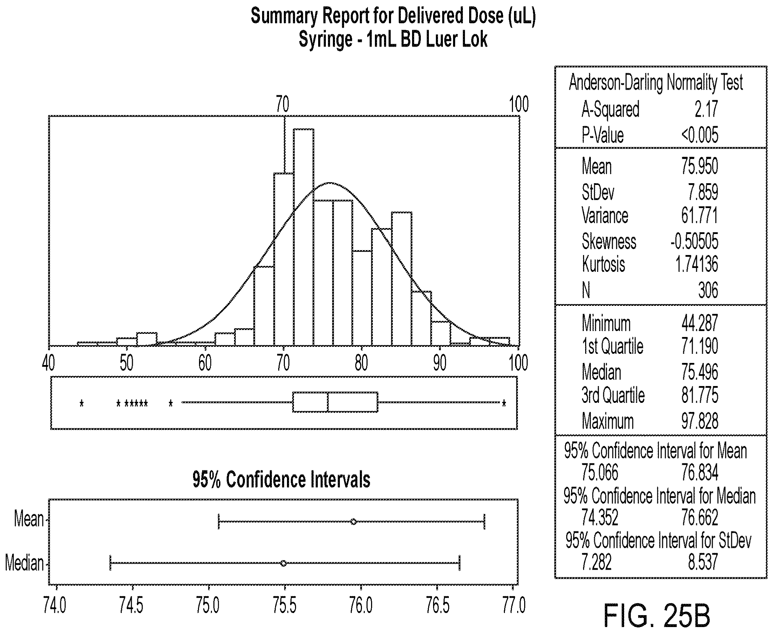

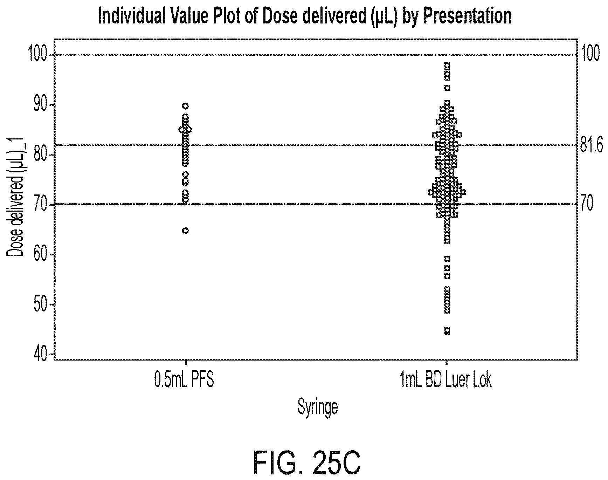

The testing of REGN3-PFS-0.5mL demonstrated that 59 out of 60 samples were able to administer a volume with high precision with 1 sample delivering volume of 64.684 microliters. See the histogram of delivered doses of REGN3-PFS-0.5mL in Figure 25 (A).
Three hundred six (306) samples delivered with the 1mL BD (Becton Dickinson) Luer Lok syringe were utilized for data analysis. Samples 21, 74, 117 and 125 were removed from analysis due to operator error in dose preparation prior to collection. Testing showed that 252 out of 306 samples were 70 microliters -100 microliters. See the histogram of delivered doses in Figure 25(B).
The deliverable volume data of both devices showed the difference in variability and accuracy. The 1mL BD (Becton Dickinson) Luer Lok syringe has a lower average delivered volume at 74.7 microliters than the 0.5mL PFS with 81.6 microliters. However, it has a larger spread of volumes delivered with a range of 53.5 microliters compared to 24.8 microliters for the 0.5 mL PFS. The increased variability depicted in the 1mL BD (Becton Dickinson) Luer Lok syringe may be attributed to user variability in setting the dose to 70 microliters as well as the variability in graduation printing on the syringe. Individual delivered volumes for each device are set forth in Figure 25 (C).
Both devices were able to provide a dose within a 70 microliters -100 microliters range; however, the REGN3-PFS-0.5mL was able to deliver more consistent doses within a limited deliverable volume range with increased precision.

**Numbered further embodiments:**

1. A method for treating or preventing an angiogenic eye disorder, in a subject in need thereof, comprising administering to an eye of the subject, a single initial dose of about 8 mg or more of a VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose.

2. The method of embodiment 1 wherein, while receiving said treatment:

(i) with respect to visual acuity or best corrected visual acuity (BCVA), the subject achieves:

○ no loss in visual acuity or BCVA;

○ a gain in visual acuity or BCVA;

○ maintenance of visual acuity or BCVA

○ no loss of visual acuity or BCVA by about week 4, 8, 9, 12, 16, 20, 24, 28, 32, 36, 40 or 44, following the initial dose wherein visual acuity or BCVA is according to ETDRS or the Snellen equivalent;

o no loss of visual acuity or BCVA of about 5 or more, about 10 or more, or about 15 or more letters by about week 4, 8, 9, 12, 16, 20, 24, 28, 32, 36, 40 or 44, following the initial dose wherein visual acuity or BCVA is according to ETDRS or the Snellen equivalent;

o a gain in visual acuity or BCVA, of about 5 or more, about 6 or more, about 7 or more, about 8 or more, about 9 or more, about 10 or more or about 15 or more letters, by about week 4, 8, 9, 12, 16, 20, 24, 28, 32, 36, 40 or 44 following the initial dose, wherein visual acuity or BCVA is according to ETDRS or the Snellen equivalent; and/or

o a gain in visual acuity or BCVA of about 6 or 7 or 8 letters by about week 8 and maintaining a gain of about 6 or 7 or 8 letters until at least about week 44 wherein visual acuity or BCVA is according to ETDRS or the Snellen equivalent;

(ii) with respect to central retinal thickness (CRT), the subject achieves:

o a decrease in central retinal thickness;

o no increase in central retinal thickness;

o maintenance in central retinal thickness;

○ a decrease in central retinal thickness by at least about 123, 125, 131, 142, 147, 149, 150, 151, 156, 157, 158, 159, 161, 162, 166, 167, 168, 172, 173, 175, 177, 178 or 183 micrometers by about week 4, 8, 9, 12, 16, 20, 24, 28, 32, 36, 40 or 44 following the initial dose;

o a decrease in central retinal thickness of about 47 micrometers from about week 12 to about week 20 following the initial dose;

o a decrease in central retinal thickness of about 17 micrometers from about week 24 to about week 32 following the initial dose;

o a decrease in central retinal thickness of about 18 micrometers from about week 36 to about week 44 following the initial dose; and/or

○ a decrease in central retinal thickness of about 149, about 150, about 160 or about 149-160 micrometers by about week 4 following the initial dose and achieving said decrease ($\pm$1, 2, 3, 4, 5, 10, 12, 13, 14 or 15 micrometers) by about week 44 following the initial dose;

o a decrease ranging from about 131-178 or about 123-175 micrometers from about weeks 4 to 44;

○ a reduction in central retinal thickness of about 160 or 161 or 162 micrometers by about week 12 and maintaining said reduction ($\pm$1, 2, 3, 4 or 5 micrometers) by about week 44;

o a reduction in central retinal thickness of about 156 micrometers by about week 16 and maintaining said reduction ($\pm$1, 2, 3, 4 or 5 micrometers) by about week 44

(iii) with respect to retinal fluid, the subject achieves:

o a dry retina having no intraretinal fluid and no subretinal fluid; or no intraretinal fluid; or no subretinal fluid; in the center subfield or macula as measured by spectral domain optical coherence tomography;

o a dry retina having no intraretinal fluid and no subretinal fluid; or no intraretinal fluid; or no subretinal fluid; in the center subfield as measured by spectral domain optical coherence tomography by about week 4, 8, 9, 12, 16, 20, 24, 28, 32, 36, 40 or 44, following the initial dose;

○ no SRF and IRF in the macula as measured by SD-OCT by week 16 or week 44 following the initial dose;

o no sub-retinal pigment epithelium (RPE) fluid until at least about week 44 following the initial dose as measured by spectral domain optical coherence tomography; and/or

o maintenance of a dry retina once achieved until at least about week 44 following the initial dose as measured by spectral domain optical coherence tomography; and/or

and/or

(iv) the subject achieves:

o a reduction in total choroidal neovascularization (CNV) lesion size by at least about 3.2 or 3.3 micrometers by about week 4, 8, 9, 12, 16, 20, 24, 28, 32, 36, 40 or 44 following the initial dose;

○ no significant increase in intraocular pressure from baseline by about week 4, 8, 9, 12, 16, 20, 24, 28, 32, 36, 40 or 44 or later following the initial dose; and/or

o no significant increase in systolic (S) and/or diastolic (D) blood pressure from baseline by about week 4, 8, 9, 12, 16, 20, 24, 28, 32, 36, 40 or 44 or later following the initial dose.

3. The method of embodiment 2, wherein a dry retina lacks intraretinal fluid and/or subretinal fluid.

4. The method of embodiment 2 or 3, wherein, at or before said initial dose, the subject has one or more of the following characteristics:

- best corrected visual acuity of about 57, 58 or 57-58 ETDRS letters;
- central retinal thickness, as measured by SD-OCT, of about 488, 516, 502 or 488-516 micrometers;
- intraocular pressure of about 14, 15 or 14-15 mmHg;
- neovascular age-related macular degeneration lesion size of about 7, 8 or 7-8 $mm^2$;
- choroidal neovascularization lesion size of about 7, 8 or 7-8 $mm^2$;
- has occult choroidal neovascularization as measured by fluorescein angiography;
- has minimal classic choroidal neovascularization as measured by fluorescein angiography; and/or
- has predominantly classic choroidal neovascularization as measured by fluorescein angiography.

5. A method for improving best corrected visual acuity, decreasing central retinal thickness and/or achieving a dry retina, in an eye of a subject in need thereof suffering from an angiogenic eye disorder, comprising administering to the eye of the subject, a single initial dose of about 8 mg or more of a VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose.

6. A method for promoting retinal drying, in an eye of a subject with an angiogenic eye disorder, comprising administering to the eye of the subject, a single initial dose of about 8 mg or more of a VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose.

7. The method of any one of embodiments 1-6 wherein 4, 8 or 12 weeks is 12 weeks.

8. The method of any one of embodiments 1-7, wherein about 2 to 4 weeks is 2, 3, 4 or 5 weeks.

9. The method of any one of embodiments 1-8 wherein 2 to 4 weeks is 4 weeks.

10. The method of any one of embodiments 1-9 wherein:

- the single initial dose is followed by only 2 secondary doses,
- each secondary dose is administered about 4 weeks after the immediately preceding dose,
- the first tertiary dose is administered about 8 weeks after the immediately preceding dose,
- the first tertiary dose is administered about 12 weeks after the immediately preceding dose,
- each tertiary dose is administered about 12 weeks after the immediately preceding dose.

11. The method of any one of embodiments 1-10 further comprising administering one or more additional *pro re nata* doses.

12. The method of any one of embodiments 1-11 wherein the angiogenic eye disorder is:

- neovascular age-related macular degeneration,
- macular edema (ME),
- macular edema following retinal vein occlusion (ME-RVO),
- retinal vein occlusion (RVO),
- central retinal vein occlusion (CRVO),
- branch retinal vein occlusion (BRVO),

- diabetic macular edema (DME),
- choroidal neovascularization (CNV),
- iris neovascularization,
- neovascular glaucoma,
- post-surgical fibrosis in glaucoma,
- proliferative vitreoretinopathy (PVR),
- optic disc neovascularization,
- corneal neovascularization,
- retinal neovascularization,
- vitreal neovascularization,
- pannus,
- pterygium,
- vascular retinopathy,
- diabetic retinopathy (DR),
- non-proliferative diabetic retinopathy,
- diabetic retinopathy characterized by a Diabetic Retinopathy Severity Scale (DRSS) level of about 47 or 53,
- proliferative diabetic retinopathy,
- proliferative diabetic retinopathy in a subject that does not suffer from DME, and/or
- diabetic retinopathy in a patient who has diabetic macular edema (DME).

13. The method of any one of embodiments 1-12 wherein

- the method comprises administering the secondary doses to a subject who has received the initial dose,
- the method comprises administering the remaining secondary doses to a subject who has already received one or more secondary doses,
- the method comprises administering one or more tertiary doses to a subject who has already received the secondary doses, or
- the method comprises administering one or more tertiary doses to a subject who has already received one or more tertiary doses.

14. The method of embodiment 6, wherein retinal drying is characterized by no intraretinal fluid (IRF) and no subretinal fluid (SRF) in the eye of the subject, after the subject has received three monthly doses of the VEGF antagonist.

15. The method of any one of embodiments 1-14, wherein the VEGF antagonist is a VEGF receptor fusion protein.

16. The method of any one of embodiments 1-15, wherein the VEGF antagonist is:

(i) a VEGF receptor fusion protein comprising two polypeptides that comprise (1) a VEGFR1 component comprising amino acids 27 to 129 of SEQ ID NO: 2; (2) a VEGFR2 component comprising amino acids 130-231 of SEQ **ID** NO: 2; and (3) a multimerization component comprising amino acids 232-457 of SEQ **ID** NO: 2;
(ii) a VEGF receptor fusion protein comprising two polypeptides that comprise an immunoglobin-like (Ig) domain 2 of VEGFR1, an Ig domain 3 of a VEGFR2, and a multimerizing component;
(iii) a VEGF receptor fusion protein comprising two polypeptides that comprise an immunoglobin-like (Ig) domain 2 of VEGFR1, an Ig domain 3 of VEGFR2, an Ig domain 4 of VEGFR2 and a multimerizing component;
(iv) a VEGF receptor fusion protein comprising two VEGFR1R2-FcΔC1(a) polypeptides encoded by the nucleic acid sequence of SEQ ID NO: 1; or
(v) selected from the group consisting of: aflibercept, conbercept, an anti-VEGF antibody or antigen-binding fragment thereof or biopolymer conjugate thereof, bevacizumab, ranibizumab, pegaptanib, brolucizumab, faricimab, abicipar pegol, an anti-VEGF DARPin and a bispecific anti-VEGF/ANG2 antibody.

17. The method of any one of embodiments 1-16, wherein the VEGF antagonist is aflibercept or conbercept.

18. The method of any one of embodiments 1-17 wherein VEGF antagonist is in a pharmaceutical formulation comprising:

at least about 100 mg/ml of a VEGF receptor fusion protein,
L-arginine, and

a histidine-based buffer.

19. The method of any one of embodiments 1-18, wherein the VEGF antagonist is in a pharmaceutical formulation selected from the group consisting of A-KKKK.

20. The method of any one of embodiments 1-19, wherein the VEGF antagonist is in a pre-filled syringe.

21. The method of embodiment 20, wherein the pre-filled syringe is glass or plastic, and/or sterile.

22. The method of any one of embodiments 1-21 wherein said about 8 mg or more is a dose of about 7.2 mg, 8 mg, 8.01 mg, 8.8 mg, 7.2-8.8 mg, 9.3 mg, 9.33 mg, 9.7 mg, 9.8 mg, 9.9 mg, or 9.7-9.9 mg, or more.

23. The method of any one of embodiments 1-22 wherein said dose is ± about 10%, ± about 0.5, or ± about 0.51 mg.

24. The method of any one of embodiments 1-23 wherein said VEGF antagonist is delivered in a volume of about 70, 81, 82, 81.7, 85, 86, 87, 85-87 microliters.

25. The method of any one of embodiments 1-24 wherein VEGF antagonist is delivered in a volume that is ± about 4, 4.45, 4.5, or 5 microliters.

26. The method of any one of embodiments 1-25 wherein said VEGF antagonist is delivered by intravitreal dose with a dose delivery device which is a syringe.

27. The method of any one of embodiments 1-26 wherein the VEGF antagonist is administered by intravitreal injection of a volume that is device-determined.

28. The method of any one of embodiments 1-27 wherein the VEGF antagonist is administered by intravitreal injection, of a formulation comprising the VEGF antagonist, with a pre-filled syringe wherein said method comprises the steps:

(a) priming the syringe by advancing the plunger rod by a predetermined distance into the body until advancement of the plunger rod is resisted by a stop;
(b) rotating the plunger rod about a longitudinal axis; and
(c) actuating the plunger rod to dispense a predetermined volume of the formulation.

## Claims

1. A VEGF antagonist for use in a method for treating or preventing an angiogenic eye disorder, in a subject in need thereof, wherein the method comprises administering, to an eye of the subject, a single initial dose of about 8 mg or more of the VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose.

2. The VEGF antagonist for use according to claim 1 wherein, while receiving said treatment:

(i) with respect to visual acuity or best corrected visual acuity (BCVA), the subject achieves:

◦ no loss in visual acuity or BCVA;
◦ a gain in visual acuity or BCVA;
◦ maintenance of visual acuity or BCVA
◦ no loss of visual acuity or BCVA by about week 4, 8, 9, 12, 16, 20, 24, 28, 32, 36, 40 or 44, following the initial dose wherein visual acuity or BCVA is according to Early Treatment Diabetic Retinopathy Study (ETDRS) or the Snellen equivalent;
◦ no loss of visual acuity or BCVA of about 5 or more, about 10 or more, or about 15 or more letters by about week 4, 8, 9, 12, 16, 20, 24, 28, 32, 36, 40 or 44, following the initial dose wherein visual acuity or BCVA is according to ETDRS or the Snellen equivalent;
◦ a gain in visual acuity or BCVA, of about 5 or more, about 6 or more, about 7 or more, about 8 or more, about 9 or more, about 10 or more or about 15 or more letters, by about week 4, 8, 9, 12, 16, 20, 24, 28, 32, 36, 40 or 44 following the initial dose, wherein visual acuity or BCVA is according to ETDRS or the Snellen equivalent; and/or

o a gain in visual acuity or BCVA of about 6 or 7 or 8 letters by about week 8 and maintaining a gain of about 6 or 7 or 8 letters until at least about week 44 wherein visual acuity or BCVA is according to ETDRS or the Snellen equivalent;

(ii) with respect to central retinal thickness (CRT), the subject achieves:

◦ a decrease in central retinal thickness;
◦ no increase in central retinal thickness;
◦ maintenance in central retinal thickness;
◦ a decrease in central retinal thickness by at least about 123, 125, 131, 142, 147, 149, 150, 151, 156, 157, 158, 159, 161, 162, 166, 167, 168, 172, 173, 175, 177, 178 or 183 micrometers by about week 4, 8, 9, 12, 16, 20, 24, 28, 32, 36, 40 or 44 following the initial dose;
◦ a decrease in central retinal thickness of about 47 micrometers from about week 12 to about week 20 following the initial dose;
◦ a decrease in central retinal thickness of about 17 micrometers from about week 24 to about week 32 following the initial dose;
◦ a decrease in central retinal thickness of about 18 micrometers from about week 36 to about week 44 following the initial dose;
◦ a decrease in central retinal thickness of about 149, about 150, about 160 or about 149-160 micrometers by about week 4 following the initial dose and achieving said decrease (±1, 2, 3, 4, 5, 10, 12, 13, 14 or 15 micrometers) by about week 44 following the initial dose;
◦ a decrease ranging from about 131-178 or about 123-175 micrometers from about weeks 4 to 44;
o◦ a reduction in central retinal thickness of about 160 or 161 or 162 micrometers by about week 12 and maintaining said reduction (±1, 2, 3, 4 or 5 micrometers) by about week 44; and/or
◦ a reduction in central retinal thickness of about 156 micrometers by about week 16 and maintaining said reduction (±1, 2, 3, 4 or 5 micrometers) by about week 44

(iii) with respect to retinal fluid, the subject achieves:

◦ a dry retina having no intraretinal fluid and no subretinal fluid; or no intraretinal fluid; or no subretinal fluid; in the center subfield or macula as measured by spectral domain optical coherence tomography (SD-OCT);
◦ a dry retina having no intraretinal fluid and no subretinal fluid; or no intraretinal fluid; or no subretinal fluid; in the center subfield as measured by spectral domain optical coherence tomography by about week 4, 8, 9, 12, 16, 20, 24, 28, 32, 36, 40 or 44, following the initial dose;
◦ no SRF and IRF in the macula as measured by SD-OCT by week 16 or week 44 following the initial dose;
◦ no sub-retinal pigment epithelium (RPE) fluid until at least about week 44 following the initial dose as measured by spectral domain optical coherence tomography; and/or
◦ maintenance of a dry retina once achieved until at least about week 44 following the initial dose as measured by spectral domain optical coherence tomography;
and/or

(iv) the subject achieves:

◦ a reduction in total choroidal neovascularization (CNV) lesion size by at least about 3.2 or 3.3 micrometers by about week 4, 8, 9, 12, 16, 20, 24, 28, 32, 36, 40 or 44 following the initial dose;
◦ no significant increase in intraocular pressure from baseline by about week 4, 8, 9, 12, 16, 20, 24, 28, 32, 36, 40 or 44 or later following the initial dose; and/or
◦ no significant increase in systolic (S) and/or diastolic (D) blood pressure from baseline by about week 4, 8, 9, 12, 16, 20, 24, 28, 32, 36, 40 or 44 or later following the initial dose.

**3.** The VEGF antagonist for use according to claim 2, wherein a dry retina lacks intraretinal fluid and/or subretinal fluid.

**4.** The VEGF antagonist for use according to claim 2 or 3, wherein, at or before said initial dose, the subject has one or more of the following characteristics:

- best corrected visual acuity of about 57, 58 or 57-58 ETDRS letters;
- central retinal thickness, as measured by SD-OCT, of about 488, 516, 502 or 488-516 micrometers;
- intraocular pressure of about 14, 15 or 14-15 mmHg;
- neovascular age-related macular degeneration lesion size of about 7, 8 or 7-8 mm$^2$;
- choroidal neovascularization lesion size of about 7, 8 or 7-8 mm$^2$;
- has occult choroidal neovascularization as measured by fluorescein angiography;
- has minimal classic choroidal neovascularization as measured by fluorescein angiography; and/or
- has predominantly classic choroidal neovascularization as measured by fluorescein angiography.

5. A VEGF antagonist for use in a method for improving best corrected visual acuity, decreasing central retinal thickness and/or achieving a dry retina, in an eye of a subject in need thereof suffering from an angiogenic eye disorder, wherein the method comprises administering to the eye of the subject, a single initial dose of about 8 mg or more of a VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose.

6. A VEGF antagonist for use in a method for promoting retinal drying, in an eye of a subject with an angiogenic eye disorder, wherein the method comprises administering to the eye of the subject, a single initial dose of about 8 mg or more of a VEGF antagonist, followed by one or more secondary doses of about 8 mg or more of the VEGF antagonist, followed by one or more tertiary doses of about 8 mg or more of the VEGF antagonist; wherein each secondary dose is administered about 2 to 4 weeks after the immediately preceding dose; and wherein each tertiary dose is administered about 4, 8 or 12 weeks after the immediately preceding dose.

7. The method of any one of the preceding claims wherein:

(a) 4, 8 or 12 weeks is 12 weeks; and/or
(b) about 2 to 4 weeks is: (i) 2, 3, 4 or 5 weeks; or (ii) 4 weeks.

8. The VEGF antagonist for use according to any one of the preceding claims wherein:

- the single initial dose is followed by only 2 secondary doses,
- each secondary dose is administered about 4 weeks after the immediately preceding dose,
- the first tertiary dose is administered about 8 weeks after the immediately preceding dose,
- the first tertiary dose is administered about 12 weeks after the immediately preceding dose,
- each tertiary dose is administered about 12 weeks after the immediately preceding dose.

9. The VEGF antagonist for use according to any one of the preceding claims, wherein the method further comprises administering one or more additional *pro re nata* doses.

10. The VEGF antagonist for use according to any one of the preceding claims, wherein the angiogenic eye disorder is:

- neovascular age-related macular degeneration,
- macular edema (ME),
- macular edema following retinal vein occlusion (ME-RVO),
- retinal vein occlusion (RVO),
- central retinal vein occlusion (CRVO),
- branch retinal vein occlusion (BRVO),
- diabetic macular edema (DME),
- choroidal neovascularization (CNV),
- iris neovascularization,
- neovascular glaucoma,
- post-surgical fibrosis in glaucoma,
- proliferative vitreoretinopathy (PVR),
- optic disc neovascularization,
- corneal neovascularization,
- retinal neovascularization,
- vitreal neovascularization,

- pannus,
- pterygium,
- vascular retinopathy,
- diabetic retinopathy (DR),
- non-proliferative diabetic retinopathy,
- diabetic retinopathy **characterized by** a Diabetic Retinopathy Severity Scale (DRSS) level of about 47 or 53,
- proliferative diabetic retinopathy,
- proliferative diabetic retinopathy in a subject that does not suffer from DME, and/or
- diabetic retinopathy in a patient who has diabetic macular edema (DME).

11. The VEGF antagonist for use according to any one of the preceding claims wherein:

   - the method comprises administering the secondary doses to a subject who has received the initial dose;
   - the method comprises administering the remaining secondary doses to a subject who has already received one or more secondary doses;
   - the method comprises administering one or more tertiary doses to a subject who has already received the secondary doses; or
   - the method comprises administering one or more tertiary doses to a subject who has already received one or more tertiary doses.

12. The VEGF antagonist for use according to claim 11, wherein retinal drying is **characterized by** no intraretinal fluid (IRF) and no subretinal fluid (SRF) in the eye of the subject, after the subject has received three monthly doses of the VEGF antagonist.

13. The VEGF antagonist for use according to any one of the preceding claims, wherein the VEGF antagonist is a VEGF receptor fusion protein,
   optionally, wherein the VEGF antagonist is:

   (i) a VEGF receptor fusion protein comprising two polypeptides that comprise (1) a VEGFR1 component comprising amino acids 27 to 129 of SEQ ID NO: 2; (2) a VEGFR2 component comprising amino acids 130-231 of SEQ ID NO: 2; and (3) a multimerization component comprising amino acids 232-457 of SEQ ID NO: 2;
   (ii) a VEGF receptor fusion protein comprising two polypeptides that comprise an immunoglobin-like (Ig) domain 2 of VEGFR1, an Ig domain 3 of a VEGFR2, and a multimerizing component;
   (iii) a VEGF receptor fusion protein comprising two polypeptides that comprise an immunoglobin-like (Ig) domain 2 of VEGFR1, an Ig domain 3 of VEGFR2, an Ig domain 4 of VEGFR2 and a multimerizing component;
   (iv) a VEGF receptor fusion protein comprising two VEGFR1R2-Fc$\Delta$C1(a) polypeptides encoded by the nucleic acid sequence of SEQ ID NO: 1; or
   (v) selected from the group consisting of: aflibercept, conbercept, an anti-VEGF antibody or antigen-binding fragment thereof or biopolymer conjugate thereof, bevacizumab, ranibizumab, pegaptanib, brolucizumab, faricimab, abicipar pegol, an anti-VEGF DARPin and a bispecific anti-VEGF/ANG2 antibody.

14. The VEGF antagonist for use according to any one of the preceding claims, wherein the VEGF antagonist is aflibercept or conbercept.

15. The VEGF antagonist for use according to any one of the preceding claims wherein VEGF antagonist is in a pharmaceutical formulation comprising:

   at least about 100 mg/ml of a VEGF receptor fusion protein,
   L-arginine, and
   a histidine-based buffer.

16. The VEGF antagonist for use according to any one of the preceding claims, wherein:

   (a) the VEGF antagonist is in a pharmaceutical formulation selected from the group consisting of A-KKKK;
   (b) in a pre-filled syringe;
   (c) in a pre-filled syringe, wherein the pre-filled syringe is glass or plastic, and/or sterile;
   (d) said about 8 mg or more is a dose of about 7.2 mg, 8 mg, 8.01 mg, 8.8 mg, 7.2-8.8 mg, 9.3 mg, 9.33 mg, 9.7 mg,

9.8 mg, 9.9 mg, or 9.7-9.9 mg, or more; and/or

(e) said dose is ± about 10%, ± about 0.5, or ± about 0.51 mg;

(f) the VEGF antagonist is delivered in a volume of about 70, 81, 82, 81.7, 85, 86, 87, 85-87 microliters; and/or

(g) the VEGF antagonist is delivered in a volume that is ± about 4, 4.45, 4.5, or 5 microliters.

17. The VEGF antagonist for use according to any one of the preceding claims wherein said VEGF antagonist is:

(a) delivered by intravitreal dose with a dose delivery device which is a syringe; and/or

(b) administered by intravitreal injection of a volume that is device-determined.

18. The VEGF antagonist for use according to any one of the preceding claims wherein the VEGF antagonist is administered by intravitreal injection, of a formulation comprising the VEGF antagonist, with a pre-filled syringe wherein said method comprises the steps:

(a) priming the syringe by advancing the plunger rod by a predetermined distance into the body until advancement of the plunger rod is resisted by a stop;

(b) rotating the plunger rod about a longitudinal axis; and

(c) actuating the plunger rod to dispense a predetermined volume of the formulation.

FIG. 1

Baseline Demographics (All patients*)

| | IAI (n=27) | HD (n=28) | ALL (n=55) |
|---|---|---|---|
| Sex | | | |
| Male | 9 (33.3%) | 12 (42.9%) | 21 (38.2%) |
| Female | 18 (66.7%) | 16 (57.1%) | 34 (61.8%) |
| Ethnicity | | | |
| All | 27 (100%) | 28 (100%) | 55 (100%) |
| HISPANIC OR LATINO | 4 (14.8%) | 1 (3.6%) | 5 (9.1%) |
| NOT HISPANIC OR LATINO | 23 (85.2%) | 27 (96.4%) | 50 (90.9%) |
| Race | | | |
| All | 27 (100%) | 28 (100%) | 55 (100%) |
| WHITE | 27 (100%) | 28 (100%) | 55 (100%) |
| Age (years) | | | |
| Mean (SD) | 76.1 (8.90) | 77.7 (7.86) | 76.9 (8.35) |

Percentages are calculated based on N

# FIG. 2

Baseline Characteristics (All patients*)

|  | IAI (n=27) | HD (n=28) | ALL (n=55) |
|---|---|---|---|
| Mean (SD) BL BCVA in Study Eye (ETDRS letters) | 57.1 (9.24) | 59.3 (13.25) | 58.2 (11.41) |
| Mean (SD) BL CRT in Study Eye (µm) | 474.0 (222.18) | 497.3 (127.66) | |
| Mean (SD) Baseline IOP in Study Eye (mmHg) | 14.5 (3.43) | 14.7 (3.32) | 14.6 (3.35) |

## FIG. 3

Baseline Blood Pressure (All patients*)

|  | IAI (n=27) | HD (n=28) |
|---|---|---|
| Systolic Pressure (Mean (SD) mmHg) | 126.27 (10.1) | 122.8 (11.0) |
| Diastolic Pressure (Mean (SD) mmHg) | 71.73 (9.7) | 69.89 (9.5) |

## FIG. 4

Mean Change from BL in CRT *in Patients Completing Wk 16*

FIG. 5

Mean Change from BL in BCVA *in Patients Completing Wk 16*

IAI n = 22
HD n = 27

*Observed Values*

FIG. 6

Proportion of Pts with Dry Retina *in Patients Completing Wk 16*

Observed Values

FIG. 7

EP 4 647 125 A2

Proportion of Pts NOT DRY *in Patients Completing Wk 16*

Observed Values

FIG. 8

EP 4 647 125 A2

Proportion of Pts with *IRF in Patients Completing Wk 16*

*Observed Values*

FIG. 9

Proportion of Pts *with SRF in Patients Completing Wk 16*

Observed Values

FIG. 10

Fluid Status by Visit. *IAI* Completers to Wk 16

FIG. 11

Fluid Status by Visit: *HD* Completers to Wk 16

FIG. 12

Treatment Exposure (All patients*)

|  | IAI (n=27) | HD (n=28) |
|---|---|---|
| Mean (SD) | 4.1 (1.07) | 3.9 (0.92) |
| Median | 4.0 | 4.0 |
| Q1:Q3 | 4.0 : 5.0 | 3.0 : 4.5 |

## FIG. 13

## Summary of Ocular TEAEs in Study Eye

|  | IAI (N=27) | HD (N=27) |
|---|---|---|
| Number of Patients with at Least One Such AE, n(%) | 8 (29.6%) | 4 (14.3%) |
| CHALAZION | 0 | 1 (3.6%) |
| CONJUNCTIVAL DISORDER | 0 | 1 (3.6%) |
| CONJUNCTIVAL HEMORRHAGE | 1 (3.7%) | 1 (3.6%) |
| CONJUNCTIVAL HYPEREMIA | 0 | 1 (3.6%) |
| DRY EYE | 0 | 1 (3.6%) |
| EYE PAIN | 0 | 1 (3.6%) |
| MACULOPATHY | 0 | 1 (3.6%) |
| NEOVASCULAR AMD | 0 | 1 (3.6%) |
| PUNCTATE KERATITIS | 1 (3.7%) | 1 (3.6%) |
| RETINAL CYST | 1 (3.7%) | 0 |
| RETINAL HEMORRHAGE | 1 (3.7%) | 0 |
| RETINAL EDEMA | 0 | 1 (3.6%) |
| SUBRETINAL FLUID | 0 | 1 (3.6%) |
| TRICHIASIS | 0 | 1 (3.6%) |
| VISION BLURRED | 0 | 1 (3.6%) |
| VISUAL ACUITY REDUCED* | 2 (7.4%) | 0 |
| VISUAL IMPAIRMENT | 1 (3.7%) | 0 |
| VITREOUS DETACHMENT | 2 (7.4%) | 2 (7.1%) |
| CORNEAL ABRASION | 0 | 1 (3.6%) |

*1 of these events is an SAE

# FIG. 14

Summary of TEAE IOI of Study Eye

|  | IAI (N=27) | HD (N=27) |
|---|---|---|
| Number of Patients with at Least One Such AE, n (%) | 0 | 0 |

## FIG. 15

Intraocular Pressure

|  | IAI (n=27) | HD (n=28) |
|---|---|---|
| # pts with ≥10 mmHg Increase in pre-injection IOP from BL | 0 | 1 (3.6%) |
| # of patients with ≥10 mmHg Increase in post-injection IOP from pre-injection IOP at any visit | 7 (25.9%) | 4 (14.3%) |
| # pts with post-injection IOP > 25 mmHg at any visit | 3 (11.1%) | 5 (17.9%) |
| # pts with pre-injection IOP ≥ 25 mmHg at any visit | 0 | 0 |
| # pts with any visit IOP ≥ 35 mmHg at Any Time | 0 | 0 |

Safety Analysis Set
Percentages are calculated based on N

## FIG. 16

Summary of Non-Ocular TEAEs (1 of 2)

| | IAI (N=27) | HD (N=28) |
|---|---|---|
| *Number of Patients with at Least One Such AE, n(%)* | 8 (29.6%) | *10 (35.7%)* |
| DIARRHEA | 1 (3.7%) | 1 (3.6%) |
| GERD | 1 (3.7%) | 0 |
| SMALL INTESTINAL OBSTRUCTION | 1 (3.7%) | 0 |
| PERIPHERAL SWELLING | 1 (3.7%) | 0 |
| EAR INFECTION FUNGAL | 1 (3.7%) | 0 |
| SEASONAL ALLERGY | 0 | 1 (3.6%) |
| SINUSITIS | 1 (3.7%) | 1 (3.6%) |
| BACK INJURY | 0 | 1 (3.6%) |
| FALL | 0 | 1 (3.6%) |
| BACTERIAL TEST POSITIVE | 0 | 1 (3.6%) |
| HEPATIC ENZYME INCREASED | 1 (3/7%) | 0 |

FIG. 17

Summary of Non-Ocular TEAEs (2 of 2)

|  | IAI (N=27) | HD (N=28) |
|---|---|---|
| *Number of Patients with at Least One Such AE, n(%)* | *8 (29.6%)* | *10 (35.7%)* |
| URINE KETONE BODIES PRESENT | 1 (3.7%) | 0 |
| WHITE BLOOD CELLS URINE | 0 | 1 (3.6%) |
| TRANSIENT ISCHAEMIC ATTACK | 0 | 1 (3.6%) |
| COUGH | 0 | 1 (3.6%) |
| DERMATITIS CONTACT | 0 | 1 (3.6%) |
| HYPERTENSION | 1 (3.7%) | 1 (3.6%) |

## FIG. 18

Non-Ocular Serious Adverse Events

|  | IAI (N=27) | HD (N=28) |
|---|---|---|
| Number of Patients with at Least One Non-Ocular SAE, n(%) | 1 (3.7%) | 1 (3.6) |
| Small Intestinal Obstruction | 1 |  |
| TIA |  | 1 |

## FIG. 19

Hypertension Adverse Events

|  | IAI (N=27) | HD (N=28) |
|---|---|---|
| Number of Patients with at Least One Such AE, n(%) | 1 (3.7%) | 1 (3.6%) |
| Hypertension | 1 (3.7%) | 1 (3.6%) |

## FIG. 20

**Mean Systolic Blood Pressure**

FIG. 21

**Mean Diastolic Blood Pressure**

FIG. 22

**Mean Intraocular Pressure**

FIG. 23

**Mean Intraocular Pressure, Change from Baseline**

FIG. 24

Summary Report for Delivered Dose (uL) Syringe = 0.5mL PFS (hand primed)

FIG. 25A

EP 4 647 125 A2

**Summary Report for Delivered Dose (uL)**
**Syringe - 1mL BD Luer Lok**

| Anderson-Darling Normality Test | |
|---|---|
| A-Squared | 2.17 |
| P-Value | <0.005 |
| Mean | 75.950 |
| StDev | 7.859 |
| Variance | 61.771 |
| Skewness | -0.50505 |
| Kurtosis | 1.74136 |
| N | 306 |
| Minimum | 44.287 |
| 1st Quartile | 71.190 |
| Median | 75.496 |
| 3rd Quartile | 81.775 |
| Maximum | 97.828 |

95% Confidence Interval for Mean
75.066    76.834
95% Confidence Interval for Median
74.352    76.662
95% Confidence Interval for StDev
7.282    8.537

**95% Confidence Intervals**

Mean
Median

**FIG. 25B**

**Individual Value Plot of Dose delivered (µL) by Presentation**

FIG. 25C

## Disposition and Exposure Through Week 16

|  | IAI | HD | Total |
|---|---|---|---|
| Total Number Randomized | 53 | 53 | 106 |
|     Main Study | 38 | 38 | 76 |
|     Dense PK Substudy | 15 | 15 | 30 |
| Number of Patients Completing Week 16 | 51 | 53 | 104 |
| Number of Patients Who Discontinued | 2 | 0 | 2 |
|     Adverse Event | 0 | 0 | 0 |
|     Withdrawal of consent by subject | 2 | 0 | 2 |
|     *Due to COVID-19* | *1* | *0* | *1* |

| Exposure | IAI (n=53) | HD (n=53) |
|---|---|---|
| Mean (SD) number of injections (of 3 planned) | 2.9 (0.47) | 3.0 (0.14) |

## FIG. 26

## Baseline Demographics

|  | IAI (n=53) | HD (n=53) | ALL (n=106) |
|---|---|---|---|
| Sex |  |  |  |
| Male | 17 (32.1%) | 23 (43.4%) | 40 (37.7%) |
| Female | 36 (67.9%) | 30 (56.6%) | 66 (62.3%) |
|  |  |  |  |
| Ethnicity |  |  |  |
| All | 53 (100%) | 53 (100%) | 106 (100%) |
| Hispanic or Latino | 4 (7.5%) | 2 (3.8%) | 6 (5.7%) |
| Not Hispanic or Latino | 49 (92.5%) | 51 (96.2%) | 100 (94.3%) |
|  |  |  |  |
| Race |  |  |  |
| White | 51 (96.2%) | 52 (98.1%) | 103 (97.2%) |
|  |  |  |  |
| Age (years) |  |  |  |
| Mean (SD) | 77.7 (8.27) | 77.0 (7.69) | 77.4 (7.95) |

## FIG. 27

**Baseline Characteristics**

|  | IAI (n=53) | HD (n=53) | All (n=106) |
|---|---|---|---|
| Mean (SD) BL BCVA in Study Eye, ETDRS letters | 58.2 (10.5) | 57.9 (13.6) | 58.0* (11.9) |
| Mean (SD) BL CRT in Study Eye, μm | 488.1 (204.9) | 516.2 (175.64) | 502.1 (190.6) |
| Mean (SD) Baseline IOP in Study Eye, mmHg | 14.9 (3.4) | 14.8 (3.4) | 14.9 (3.4) |
| Mean (SD) Lesion Size, mm | 7.9 (6.21) | 7.7 (6.84) | 7.8 (6.50) |
| Mean (SD) CNV Size, mm | 7.9 (6.20) | 7.5 (6.86) | 7.7 (6.51) |
| Baseline FA classification [%] Occult CNV | 22 (41.5%) | 26 (49.1%) | 48 (45.3%) |
| Minimally Classic | 26 (49.1%) | 19 (35.8%) | 45 (42.5%) |
| Predominantly Classic | 4 (7.5%) | 8 (15.1%) | 12 (11.3%) |
| Missing | 1 (1.9%) | 0 | 1 (0.9%) |

*58 ETDRS letters ≈20/60-20/70 Snellen visual acuity

# FIG. 28

**Baseline Blood Pressure and Medical History of Hypertension (All patients)**

| | IAI (n=53) | HD (n=53) |
|---|---|---|
| Systolic Pressure (Mean (SD) mmHg) | 125.4 (10.6) | 124.6 (10.6) |
| Diastolic Pressure (Mean (SD) mmHg) | 70.7 (9.0) | 71.7 (8.5) |

| | IAI (n=53) | HD (n=53) |
|---|---|---|
| Medical History of Vascular Hypertensive Disorders, n (%) | 31 (58.5%) | 26 (49.1%) |

## FIG. 29

**Primary Efficacy Endpoint: Proportion of Pts without Fluid in the
Central (1mm) Subfield at Week 16**

Without fluid in central
1mm subfield =
No IRF *and* no SRF in the
center subfield on SD-OCT

51%

34%

18/53

27/53

Week 16

☒IAI  ☒HD

Treatment Difference (95% CI) = 17.0% (-1.6%,
35.5%); p=0.0770

LOCF

FIG. 30

**Proportion of Pts without Intraretinal Fluid & without Subretinal Fluid in the Center (1mm) Subfield at Week 16**

FIG. 31

MEDIAN Change from BL in CRT through Week 16

-96.0

-125.0

-138.0

-149.0

-149.0

-157.0

-161.0

-161.0

-◇- IAI (n=53)
-○- HD (n=53)

Median change in Central Retinal Thickness, μm

-10
-35
-60
-85
-110
-135
-160
-185
-210

0        4        8        12        16

Week

FAS, LOCF

FIG. 32

EP 4 647 125 A2

MEAN Change from BL in CRT through Week 16

FIG. 33

FAS, LOCF

EP 4 647 125 A2

Mean Change from BL to Week 12 in Choroidal Neovascular Size and Lesion Size - OC

FIG. 34

Mean Change from BL in BCVA

FIG. 35

Changes in Vision at Week 16 - OC

FIG. 36

**Proportion of patients Receiving Additional Treatment at Week 16**

|  | IAI (N=53) | HD (N=53) |
|---|---|---|
| Number of patients who had a Week 16 visit | 48 | 53 |
| Proportion of patients who had a Week 16 visit and who received Additional Treatment | 27% | 19% |

FIG. 37

## Ocular TEAEs in Study Eye Occurring in ≥2% of Patients Through Week 16

| | IAI (N=53) | HD (N=53) |
|---|---|---|
| Number of Patients with at Least One TEAE in Study Eye, n (%) | 12 (22.6%) | 9 (17.0%) |
| CONJUNCTIVAL HAEMORRHAGE | 2 (3.8%) | 3 (5.7%) |
| NEOVASCULAR AGE-RELATED MACULAR DEGENERATION | 2 (3.8%) | 0 |
| RETINAL HAEMORRHAGE | 2 (3.8%) | 0 |
| VISUAL ACUITY REDUCED | 2 (3.8%) | 0 |
| VISUAL IMPAIRMENT | 2 (3.8%) | 0 |
| VITREOUS DETACHMENT | 1 (1.9%) | 2 (3.8%) |

FIG. 38A

Ocular Serious Treatment Emergent Adverse Events in Study Eye Through Week 16

|  | IAI (N=53) | HD (N=53) |
|---|---|---|
| Number of Patients with at Least One Ocular SAE in the Study Eye*, n (%) | 1 (1.9%) | 1 (1.9%) |
| VISUAL ACUITY REDUCED | 1 (1.9%) | 0 |
| RETINAL TEAR | 0 | 1 (1.9%) |

FIG. 38B

Intraocular Inflammation TEAEs in the Study Eye Through Week 16

| | IAI (N=53) | HD (N=53) |
|---|---|---|
| Number of Patients with at Least One Such AE, n | 0 | 0 |

FIG. 38C

EP 4 647 125 A2

Mean Intraocular Pressure, Change from Baseline Through Week 16

FIG. 39

EP 4 647 125 A2

Summary of Intraocular Pressure Events Through Week 16

| | IAI (N=53) | HD (N=53) |
|---|---|---|
| # pts with ≥10 mmHg Increase in pre-injection IOP from BL | 0 | 0 |
| # pts with pre-injection IOP ≥25 mmHg at any visit | 0 | 1 (1.9%) |
| # pts with any visit IOP ≥35 mmHg at Any Time | 0 | 0 |

Safety Analysis Set

Percentages are calculated based on N

FIG. 40

APTC Events or Deaths Through Week 16

| | IAI (N=53) | HD (N=53) |
|---|---|---|
| Number of Patients with at Least One Such AE, n | 0 | 0 |

# FIG. 41

Hypertension Adverse Events Through Week 16

| | IAI (N=53) | HD (N=53) |
|---|---|---|
| Number of Patients with at Least One Such AE, n (%) | 1 (1.9%) | 1 (1.9%) |
| Worsening Hypertension | 1 (1.9%) | 1 (1.9%) |

# FIG. 42

Mean Change from Baseline in Systolic Blood Pressure through Week 16: All Patients

FIG. 43A

EP 4 647 125 A2

**Mean Change from Baseline in Diastolic Blood Pressure through Week 16: All Patients**

EP 4 647 125 A2

FIG. 43B

Mean Change from BL in Systolic BP through Wk 16: Dense PK Substudy Patients

| | Mean at BL (SD) | Mean (SD) TWA Δ D1 - W9 | Upper 95% CI | Treatment difference |
|---|---|---|---|---|
| IAI (n=15) | 126.8 (7.6) | 1.86 (7.46) Median:3.10 | 5.25 | 0.0104 (-5.42, 5.63) P=0.9695 |
| HD (n=15) | 129.0 (6.7) | 1.96 (7.32) Median:1.55 | 5.29 | |

Mean (SD) Baseline Values:
IAI: 126.8 (7.6)
HD: 129.0 (6.7)

FIG. 44A

Mean Change from BL in Diastolic BP through Wk 16: Dense PK Substudy Patients

| | Mean at BL (SD) | Mean (SD) TWA Δ D1 - w9 | Upper 95% CI | Treatment difference |
|---|---|---|---|---|
| IAI (n=53) | 72.3 (7.1) | 0.02 (6.15) Median:0.82 | 2.82 | -0.124 (-4.34, 4.09) p=0.9523 |
| HD (n=53) | 74.4 (7.1) | -0.10 (5.06) Median:0.70 | 2.20 | |

Mean (SD) Baseline Values:
IAI: 72.3 (7.1)
HD: 74.4 (7.1)

FIG. 44B

EP 4 647 125 A2

**Patient Disposition**

| | Aflibercept 2 mg | Aflibercept 8 mg | Total |
|---|---|---|---|
| Randomized patients, n | 53 | 53 | 106 |
| Patients completing Week 16, n (%) | 51 (96) | 53 (100) | 104 (98) |
| Patients completing Week 44, n (%) | 49 (92) | 51 (96) | 100 (94) |

# FIG. 45

**Baseline Demographics**

| | Aflibercept 2 mg (n=53) | Aflibercept 8 mg (n=53) | Total (N=106) |
|---|---|---|---|
| Sex, n(%) | | | |
|   Male | 17 (32.1) | 23 (43.4) | 40 (37.7) |
|   Female | 36 (67.9) | 30 (56.6) | 66 (62.3) |
| Race, n(%) | | | |
|   White | 51 (96.2) | 52 (98.1) | 103 (97.2) |
| Ethnicity, n (%) | | | |
|   Hispanic or Latino | 4 (7.5) | 2 (3.8) | 6 (5.7) |
|   Not Hispanic or Latino | 49 (92.5) | 51 (96.2) | 100 (94.3) |
| Age, mean (SD), years | 77.7 (8.3) | 77.0 (7.7) | 77.4 (8.0) |

SD. standard deviation.

# FIG. 46

**Baseline Characteristics - Study Eye**

| | Aflibercept 2 mg (n=53) | Aflibercept 8 mg (n=53) | Total (N=106) |
|---|---|---|---|
| BCVA, mean (SD), ETDRS letters | 58.2 (10.5) | 57.9 (13.6) | 58.0[a] (11.9) |
| CRT, mean (SD), μm | 488.1 (204.9) | 516.2 (175.6) | 502.1 (190.6) |
| IOP, mean (SD), mm Hg | 14.9 (3.4) | 14.8 (3.4) | 14.9 (3.4) |
| Lesion size, mean (SD), mm$^2$ | 7.9 (6.2) | 7.7 (6.8) | 7.8 (6.5) |
| CNV size, mean (SD), mm$^2$ | 7.9 (6.2) | 7.5 (6.9) | 7.7 (6.5) |
| FA classification, n (%) Occult Minimally classic Predominantly classic Missing | 22 (41.5) 26 (49.1) 4 (7.5) 1 (1.9) | 26 (49.1) 19 (35.8) 8 (15.1) 0 | 48 (45.3) 45 (42.5) 12 (11.3) 1 (0.9) |

*58 ETDRS letters ≈ 20/60-20/70 Snellen visual acuity.
CRT, central retinal thickness; IOP, intraocular pressure.

## FIG. 47

Treatment Exposure and PRN Treatment through Week 44

| Exposure | Aflibercept 2 mg (n=53) | Aflibercept 8 mg (n=53) |
|---|---|---|
| Mean number of aflibercept injections through Week 44 | 5.8 | 5.8 |
|  |  |  |
| Proportion of patients who did NOT receive additional or PRN treatment | 24 (45%) | 28 (53%) |
| Total number of PRN injections given | 38 | 33 |

FIG. 48

**Proportion of Eyes Without Fluid in the Center Subfield**

FAS, LOCF
Without fluid in central subfield defined as no IRF and no SRF in the center subfield on SD-OCT
LOCF: Patients receiving treatment at Week 16 were considered not dry from Week 16 onward
FAS, full analysis set; IRF, intra-retinal fluid; SD-OCT, spectral domain optical coherence tomography; SRF, sub-retinal fluid

FIG. 49

## Proportion of Eyes Without Fluid in the Macula

☒ Aflibercept 2 mg (n=53)     ☷ Aflibercept 8 mg (n=53)

Difference (95% CI): 17.0% (-0.9%, 34.8%);
*P*=0.0667

Difference (95% CI): 17.0% (1.1%, 32.8%);
*P*=0.0395

43%

26%

15%

32%

Proportion of patients, %

Week 16

Week 44

FAS. LOCF
Without fluid in macula defined as no IRF and no SRF in the macula on SD-OCT
LOCF: Patients receiving treatment at Week 16 were considered not dry from Week 16 onward

FIG. 50

## Proportion of Eyes Without Fluid in the Center Subfield

☐ Aflibercept 2 mg (n=53)   ☐ Aflibercept 8 mg (n=53)

✐ = Scheduled dose visit

FAS, LOCF,
Without fluid in central subfield defined as no IRF and no SRF in the center subfield on SD-OCT
LOCF: Patients receiving treatment at Week 16 were considered not dry from Week 16 onward

FIG. 51

Proportion of Eyes without Intraretinal Fluid* in the Center Subfield at Weeks 16 & 44

FAS: 2 mg N=53; 8 mg N=53; LOCF

*Without Intraretinal Fluid = Dry
or with Subretinal Fluid only

FIG. 52

Proportion of Eyes without Subretinal Fluid* in the Center Subfield at Weeks 16 & 44

FAS: 2 mg N=53; 8 mg N=53; LOCF

Week 16 & 44 Treatment Effect = 18.9% (95% CI= 0.6, 37.1); p=0.0471

*Without Subretinal Fluid = Dry or with Intraretinal Fluid only

FIG. 53

Mean Change from Baseline in CRT through Week 44

FIG. 54

**Median Change from Baseline in CRT through Week 44**

= Scheduled dose visit

FAS, LOCF

LOCF: Patients receiving treatment at Week 16 were considered not dry from Week 16 onward

FIG. 55

**Mean Change from Baseline in BCVA through Week 44**

Mean BCVA at Baseline:
*Aflibercept 2 mg: 58.2 ETDRS letters*
*Aflibercept 8 mg: 57.9 ETDRS letters*

= Scheduled dose visit

FAS, LOCF

LOCF: Patients receiving treatment at Week 16 were considered not dry from Week 16 onward

FIG. 56

Proportion of Patients with Vision Loss or Gain at Week 44

FAS, LOCF
[a]Two patients in the aflibercept 2 mg group did not have post-baseline values

FIG. 57

## Ocular TEAEs in Study Eye Occurring in ≥2% of Pts

|  | IAI (N=53) | HD (N=53) |
|---|---|---|
| Number of Patients with at Least One Ocular TEAE in Study Eye, n(%) | 20 (37.7%) | 20 (37.7%) |
| CONJUNCTIVAL HAEMORRHAGE | 2 (3.8%) | 3 (5.7%) |
| DRY EYE | 2 (3.8%) | 2 (3.8%) |
| MACULAR DEGENERATION | 1 (1.9%) | 1 (1.9%) |
| NEOVASCULAR AGE-RELATED MACULAR DEGENERATION | 4 (7.5%) | 2 (3.8%) |
| PUNCTATE KERATITIS | 2 (3.8%) | 1 (1.9%) |
| RETINAL HAEMORRHAGE | 2 (3.8%) | 1 (1.9%) |
| RETINAL TEAR | 0 | 2 (3.8%) |
| SUBRETINAL FLUID | 1 (1.9%) | 1 (1.9%) |
| VISUAL ACUITY REDUCED | 2 (3.8%) | 1 (1.9%) |
| VISUAL IMPAIRMENT | 2 (3.8%) | 1 (1.9%) |
| VITREOUS DETACHMENT | 2 (3.8%) | 4 (7.5%) |

## FIG. 58

**Ocular Serious TEAEs in Study Eye**

| | IAI (N=53) | HD (N=53) |
|---|---|---|
| Number of Patients with at Least One Ocular SAE in the Study Eye*, n (%) | 1 (1.9%) | 2 (3.8) |
| VISUAL ACUITY REDUCED | 1 (1.9%) | 0 |
| RETINAL TEAR | 0 | 1 (1.9%) |
| VISUAL IMPAIRMENT | 0 | 1 (1.9%) |

*No Ocular SAEs occurred in the Fellow Eye*

## FIG. 59

**Intraocular Inflammation TEAEs in Study Eye**

| | IAI (N=53) | HD (N=53) |
|---|---|---|
| Number of Patients with at Least One Such AE, n (%) | 0 | 1 (1.9%) |
| IRITIS | 0 | 1 (1.9%) |

Iritis was characterized as mild cells in the anterior chamber which resolved within 1 month with topical therapy

## FIG. 60

## Summary of Intraocular Pressure Events

|  | IAI (n=53) | HD (n=53) |
|---|---|---|
| # pts with ≥10 mmHg Increase in pre-injection IOP from BL | 0/51 | 2/53 (3.8%) |
| # pts with pre-injection IOP ≥25 mmHg at any visit | 0/53 | 2/53 (3.8%) |
| # pts with any visit IOP ≥35 mmHg at Any Time | 0/53 | 0/53 |

Safety Analysis Set
Percentages are calculated based on N

## FIG. 61

Mean Pre-Dose Intraocular Pressure, Change from Baseline

Safety Analysis Set

## FIG. 62

**Non-Ocular TEAEs Occurring in ≥2% of Patients**

|  | IAI (N=53) | HD (N=53) |
|---|---|---|
| Number of Patients with at Least One Non-Ocular TEAE, n(%) | 24 (45.3%) | 28 (52.8%) |
| DIARRHEA | 1 (1.9%) | 2 (3.8%) |
| GASTRITIS | 1 (1.9%) | 1 (1.9%) |
| GASTROOESOPHAGEAL REFLUX DISEASE | 2 (3.8%) | 0 |
| NAUSEA | 0 | 2 (3.8%) |
| CHEST PAIN | 0 | 2 (3.8%) |
| COVID-19 | 2 (3.8%) | 2 (3.8%) |
| SINUSITIS | 2 (3.8%) | 1 (1.9%) |
| URINARY TRACT INFECTION | 1 (1.9%) | 2 (3.8%) |
| FALL | 2 (3.8%) | 3 (5.7%) |
| ARTHRALGIA | 2 (3.8%) | 0 |
| CARPAL TUNNEL SYNDROME | 1 (1.9%) | 1 (1.9%) |
| DIZZINESS | 0 | 3 (5.7%) |
| HYPERTENSION | 1 (1.9%) | 1 (1.9%) |

FIG. 63

**Non-Ocular Serious TEAEs**

|  | IAI (N=53) | HD (N=53) |
|---|---|---|
| Number of Patients with at Least One Non-Ocular SAE, n(%) | 4 (7.5%) | 5 (9.4%) |
| ACUTE LEFT VENTRICULAR FAILURE | 0 | 1 (1.9%) |
| COLITIS | 1 (1.9%) | 0 |
| SMALL INTESTINAL OBSTRUCTION | 1 (1.9%) | 0 |
| CHEST PAIN | 0 | 1 (1.9%) |
| NON-CARDIAC CHEST PAIN | 0 | 1 (1.9%) |
| COVID-19 | 1 (1.9%) | 0 |
| FEMUR FRACTURE | 1 (1.9%) | 0 |
| LUMBAR SPINAL STENOSIS | 0 | 1 (1.9%) |
| HEPATIC ENCEPHALOPATHY | 0 | 1 (1.9%) |
| TRANSIENT ISCHEMIC ATTACK | 0 | 1 (1.9%) |
| ACUTE RESPIRATORY FAILURE | 0 | 1 (1.9%) |

FIG. 64

**Hypertension TEAEs**

|  | IAI (N=53) | HD (N=53) |
|---|---|---|
| Number of Patients with at Least One Such AE, n (%) | 1 (1.9%) | 1 (1.9%) |
| Worsening Hypertension | 1 (1.9%) | 1 (1.9%) |

## FIG. 65

**Adjudicated APTC Events and Deaths**

|  | IAI (N=53) | HD (N=53) |
|---|---|---|
| Adjudicated APTC Events, n | 0 | 0 |
| Deaths, n | 0 | 1* |

*Death due to glioblastoma

## FIG. 66

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63189541
- US 63235398
- US 63297420
- US 63306315
- US 7396664 B
- US 7354579 B

- WO 2005121176 A
- WO 2007112675 A
- WO 2019217927 A
- US 11103552 B
- WO 2019118588 A
- WO 2020247686 A

**Non-patent literature cited in the description**

- **HANNA et al.** Three patients with injection of intravitreal vascular endothelial growth factor inhibitors and subsequent exacerbation of chronic proteinuria and hypertension. *Clinical Kidney Journal*, 2019, vol. 12 (1), 92-100
- **RAISER et al.** The effect of intravitreal bevacizumab (Avastin) administration on systemic hypertension. *Eye*, 2009, vol. 23, 1714-1718
- **HEIER et al.** Intravitreal Aflibercept (VEGF Trap-Eye) in Wet Age-related Macular Degeneration. *Ophthalmology*, 2012, vol. 119, 2537-2548
- **BLAUG** ; **SEYMOUR**. Remington's Pharmaceutical Sciences. Mack Publishing Company, 1975, vol. 87
- Photocoagulation for diabetic macular edema. Early Treatment Diabetic Retinopathy Study report number 1. *Arch Ophthalmol.*, December 1985, vol. 103 (12), 1796-806

- **CHEN et al.** *Comparison of visual acuity estimates using three different letter charts under two ambient room illuminations*, 2012, vol. 60 (2), 101-104
- **BAILEY** ; **LOVIE**. *New design principles for visual acuity letter charts*, 1976, vol. 53 (11), 740-745
- **SHAMIR et al.** Comparison of Snellen and Early Treatment Diabetic Retinopathy Study charts using a computer simulation. *Int. J. Opthamology*, 2016, vol. 9 (1), 119-123
- **KAISER**. Prospective Evaluation of Visual Acuity Assessment: A Comparison of Snellen Versus ETDRS Charts in Clinical Practice (An AOS Thesis). *Trans Am Ophthalmol Soc*, 2009, vol. 107, 311-324
- **BAILEY** ; **LOVIE**. New design principles for visual acuity letter charts. *Am J Optometry Physiol Opt*, 1976, vol. 53, 740-745